# EUROPEAN PATENT SPECIFICATION

(11) **EP 2 099 761 B1**
(45) Date of publication and mention of the grant of the patent: **27.10.2010**
(21) Application number: 07847817.9
(22) Date of filing: 04.12.2007
(51) Int. Cl.: C07D 215/22, C07D 401/12, C07D 487/08, A61K 31/47, C07D 401/06, C07D 401/14, C07D 417/06, C07D 215/227, A61K 31/551, A61K 31/4995, A61P 31/04

(54) **ANTIBACTERIAL QUINOLINE DERIVATIVES**
ANTIBAKTERIELLE CHINOLINDERIVATE
DÉRIVÉS DE QUINOLINE ANTIBACTÉRIENS

(30) Priority: 06.12.2006 EP 06125545
(43) Date of publication of application: 16.09.2009
(73) Proprietor: Janssen Pharmaceutica NV, 2340 Beerse (BE)
(72) Inventor: GUILLEMONT, Jérôme Emile Georges, 27106 Val de Reuil Cedex (FR); DORANGE, Ismet, 13165 Nacka (SE); LANÇOIS, David Francis Alain, 27106 Val de Reuil Cedex (FR); VILLALGORDO-SOTO, José Manuel, 30320 Fuente Alamo, Murcia (ES); SIMONNET, Yvan René Ferdinand, 27106 Val de Reuil Cedex (FR); MOTTE, Magali Madeleine Simone, 27106 Val de Reuil Cedex (FR); ANDRIES, Koenraad Jozef Lodewijk Marcel, 2340 Beerse (BE); KOUL, Anil, 2340 Beerse (BE)
(74) Representative: Vervoort, Liesbeth
(86) International application number: PCT/EP2007/063319
(87) International publication number: WO 2008/068272

(56) References cited:
- WO-A-2004/011436
- WO-A-2005/117875
- WO-A-2006/067048
- WO-A-2006/131519
- WO-A-2007/000434
- WO-A-2007/000435
- ANDRIES KOEN ET AL: "A diaryiquinotine drug active on the ATP synthase of Mycobacterium tuberculosis" SCIENCE (WASHINGTON D C), vol. 307, no. 5707, 14 January 2005 (2005-01-14), pages 223-227, XP002358962 ISSN: 0036-8075

## Description

The present invention relates to novel substituted quinoline derivatives useful for the treatment of bacterial diseases, including but not limited to diseases caused by pathogenic mycobacteria such as *Mycobacterium tuberculosis, M. bovis, M. leprae, M. avium and M. marinum,* or pathogenic Staphylococci or Streptococci.

### BACKGROUND OF THE INVENTION

*Mycobacterium tuberculosis* is the causative agent of tuberculosis (TB), a serious and potentially fatal infection with a world-wide distribution. Estimates from the World Health Organization indicate that more than 8 million people contract TB each year, and 2 million people die from tuberculosis yearly. In the last decade, TB cases have grown 20% worldwide with the highest burden in the most impoverished communities. If these trends continue, TB incidence will increase by 41% in the next twenty years. Fifty years since the introduction of an effective chemotherapy, TB remains after AIDS, the leading infectious cause of adult mortality in the world. Complicating the TB epidemic is the rising tide of multi-drug- resistant strains, and the deadly symbiosis with HIV. People who are HIV-positive and infected with TB are 30 times more likely to develop active TB than people who are HIV-negative and TB is responsible for the death of one out of every three people with HIV/AIDS worldwide

Existing approaches to treatment of tuberculosis all involve the combination of multiple agents. For example, the regimen recommended by the U.S. Public Health Service is a combination of isoniazid, rifampicin and pyrazinamide for two months, followed by isoniazid and rifampicin alone for a further four months. These drugs are continued for a further seven months in patients infected with HIV. For patients infected with multi-drug resistant strains of *M. tuberculosis,* agents such as ethambutol, streptomycin, kanamycin, amikacin, capreomycin, ethionamide, cycloserine, ciprofoxacin and ofloxacin are added to the combination therapies. There exists no single agent that is effective in the clinical treatment of tuberculosis, nor any combination of agents that offers the possibility of therapy of less than six months' duration.

There is a high medical need for new drugs that improve current treatment by enabling regimens that facilitate patient and provider compliance. Shorter regimens and those that require less supervision are the best way to achieve this. Most of the benefit from treatment comes in the first 2 months, during the intensive, or bactericidal, phase when four drugs are given together; the bacterial burden is greatly reduced, and patients become noninfectious. The 4- to 6-month continuation, or sterilizing, phase is required to eliminate persisting bacilli and to minimize the risk of relapse. A potent sterilizing drug that shortens treatment to 2 months or less would be extremely beneficial. Drugs that facilitate compliance by requiring less intensive supervision also are needed. Obviously, a compound that reduces both the total length of treatment and the frequency of drug administration would provide the greatest benefit.

Complicating the TB epidemic is the increasing incidence of multi-drug- resistant strains or MDR-TB. Up to four percent of all cases worldwide are considered MDR-TB - those resistant to the most effective drugs of the four-drug standard, isoniazid and rifampin. MDR-TB is lethal when untreated and cannot be adequately treated through the standard therapy, so treatment requires up to 2 years of "second-line" drugs. These drugs are often toxic, expensive and marginally effective. In the absence of an effective therapy, infectious MDR-TB patients continue to spread the disease, producing new infections with MDR-TB strains. There is a high medical need for a new drug with a new mechanism of action, which is likely to demonstrate activity against drug resistant, in particular MDR strains.

The term "drug resistant" as used hereinbefore or hereinafter is a term well understood by the person skilled in microbiology. A drug resistant Mycobacterium is a Mycobacterium which is no longer susceptible to at least one previously effective drug; which has developed the ability to withstand antibiotic attack by at least one previously effective drug. A drug resistant strain may relay that ability to withstand to its progeny. Said resistance may be due to random genetic mutations in the bacterial cell that alters its sensitivity to a single drug or to different drugs.
MDR tuberculosis is a specific form of drug resistant tuberculosis due to a bacterium resistant to at least isoniazid and rifampicin (with or without resistance to other drugs), which are at present the two most powerful anti-TB drugs. Thus, whenever used hereinbefore or hereinafter "drug resistant" includes multi drug resistant.

Another factor in the control of the TB epidemic is the problem of latent TB. In spite of decades of tuberculosis (TB) control programs, about 2 billion people are infected by M. tuberculosis, though asymptomatically. About 10% of these individuals are at risk of developing active TB during their lifespan. The global epidemic ofTB is fuelled by infection of HIV patients with TB and rise of multi-drug resistant TB strains (MDR-TB). The reactivation of latent TB is a high risk factor for disease development and accounts for 32% deaths in HIV infected individuals. To control TB epidemic, the need is to discover new drugs that can kill dormant or latent bacilli. The dormant TB can get reactivated to cause disease by several factors like suppression of host immunity by use of immunosuppressive agents like antibodies against tumor necrosis factor α or interferon-γ. In case of HIV positive patients the only prophylactic treatment available for latent TB is two- three months regimens of rifampicin, pyrazinamide. The efficacy of the treatment regime is still not clear and furthermore the length of the treatments is an important constrain in resource-limited environments. Hence there is a drastic need to identify new drugs, which can act as chemoprophylatic agents for individuals harboring latent TB bacilli.
The tubercle bacilli enter healthy individuals by inhalation; they are phagocytosed by the alveolar macrophages of the lungs. This leads to potent immune response and formation of granulomas, which consist of macrophages infected with M. tuberculosis surrounded by T cells. After a period of 6-8 weeks the host immune response cause death of infected cells by necrosis and accumulation of caseous material with certain extracellular bacilli, surrounded by macrophages, epitheloid cells and layers of lymphoid tissue at the periphery. In case of healthy individuals, most of the mycobacteria are killed in these environments but a small proportion of bacilli still survive and are thought to exist in a non-replicating, hypometabolic state and are tolerant to killing by anti-TB drugs like isoniazid. These bacilli can remain in the altered physiological environments even for individual's lifetime without showing any clinical symptoms of disease. However, in 10% of the cases these latent bacilli may reactivate to cause disease. One of the hypothesis about development of these persistent bacteria is patho-physiological environment in human lesions namely, reduced oxygen tension, nutrient limitation, and acidic pH. These factors have been postulated to render these bacteria phenotypically tolerant to major anti-mycobacterial drugs.

In addition to the management of the TB epidemic, there is the emerging problem of resistance to first-line antibiotic agents. Some important examples include penicillin-resistant Streptococcus pneumoniae, vancomycin-resistant enterococci, methicillin-resistant Staphylococcus aureus, multi-resistant salmonellae.

The consequences of resistance to antibiotic agents are severe. Infections caused by resistant microbes fail to respond to treatment, resulting in prolonged illness and greater risk of death. Treatment failures also lead to longer periods of infectivity, which increase the numbers of infected people moving in the community and thus exposing the general population to the risk of contracting a resistant strain infection.
Hospitals are a critical component of the antimicrobial resistance problem worldwide. The combination of highly susceptible patients, intensive and prolonged antimicrobial use, and cross-infection has resulted in infections with highly resistant bacterial pathogens.

Self-medication with antimicrobials is another major factor contributing to resistance. Self-medicated antimicrobials may be unnecessary, are often inadequately dosed, or may not contain adequate amounts of active drug.

Patient compliance with recommended treatment is another major problem. Patients forget to take medication, interrupt their treatment when they begin to feel better, or may be unable to afford a full course, thereby creating an ideal environment for microbes to adapt rather than be killed.

Because of the emerging resistance to multiple antibiotics, physicians are confronted with infections for which there is no effective therapy. The morbidity, mortality, and financial costs of such infections impose an increasing burden for health care systems worldwide.

Therefore, there is a high need for new compounds to treat bacterial infections, especially mycobacterial infections including drug resistant and latent mycobacterial infections, and also other bacterial infections especially those caused by resistant bacterial strains.

W02004/011436, W02005/070924, WO2005/070430 and WO2005/075428 disclose certain substituted quinoline derivatives having activity against *Mycobacteria,* in particular against *Mycobacterium tuberculosis.* WO2005/117875 describes substituted quinoline derivatives having activity against resistant Mycobacterial strains. W02006/067048 describes substituted quinoline derivatives having activity against latent tuberculosis. One particular compound of these substituted quinoline derivatives is described in Science (2005), 307, 223-227 and its mode of action is described in WO2006/035051.

Other substituted quinolines are disclosed in US-5,965,572 (The United States of America) for treating antibiotic resistant infections and in WO00/34265 to inhibit the growth of bacterial microorganisms.

The purpose of the present invention is to provide novel compounds, in particular substituted quinoline derivatives, having the property of inhibiting bacterial growth especially of mycobacteria but also of other bacteria such as Streptococci and Staphylococci and the compounds are therefore useful for the treatment of bacterial diseases, particularly those diseases caused by pathogenic bacteria such as *Streptococcus pneumonia, Staphylococcus aureus* or *Mycobacterium tuberculosis* (including the latent disease and including drug resistant *M. tuberculosis* strains), *M. bovis, M. leprae, M. avium and M. marinum.*

### SUMMARY OF THE INVENTION

The present invention relates to novel substituted quinoline derivatives according to formula (Ia) or (Ib): 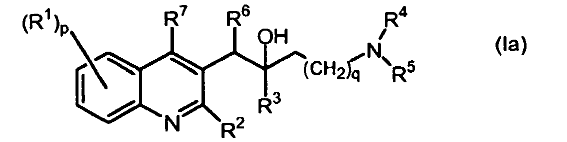 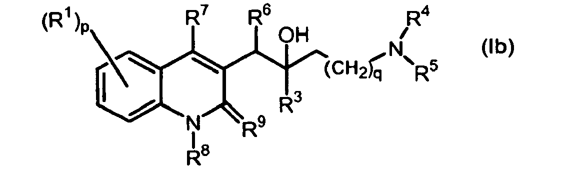 including any stereochemically isomeric form thereof, wherein
- p: is an integer equal to 1, 2, 3 or 4;
- q: is an integer equal to zero, 1, 2, 3 or 4 ;
- R¹: is hydrogen, cyano, formyl, carboxyl, halo, alkyl, C₂₋₆alkenyl, C₂₋₆alkynyl, haloalkyl, hydroxy, alkyloxy, alkylthio, alkylthioalkyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylalkyl, arylcarbonyl, R^{5a}R^{4a}Nalkyl, di(aryl)alkyl, aryl, R^{5a}R^{4a}N-, R^{5a}R^{4a}N-C(=O)-, or Het;
- R²: is hydrogen, alkyloxy, aryl, aryloxy, hydroxy, mercapto, alkyloxyalkyloxy, alkylthio, mono or di(alkyl)amino, pyrrolidino or a radical of formula 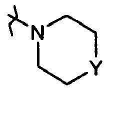 wherein Y is CH₂, O, S, NH or *N*-alkyl ;
- R³: is alkyl, arylalkyl, aryl-O-alkyl, aryl-alkyl-O-alkyl, aryl, aryl-aryl, Het, Het-alkyl, Het-O-alkyl, Het-alkyl-O-alkyl or 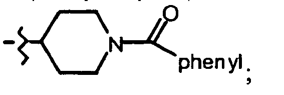
- R⁴: is hydrogen or alkyl;
- R⁵: is -C(=NH)-NH₂; arylalkyl; Het-alkyl; mono- or dialkylaminoalkyl; bicyclo[2.2.1]heptyl; Het; or aryl; or
- R⁴ and R⁵: together with the nitrogen atom to which they are attached form a radical selected from the group consisting of azetidinyl; 2,3- dihydroisoindol-1-yl; thiazolidin-3-yl; 1,2,3,6-tetrahydropyridyl; hexahydro-1*H*-azepinyl; hexahydro-1*H*-1,4-diazepinyl; hexahydro-1,4- oxazepinyl; 1,2,3,4-tetrahydroisoquinolin-2-yl; 2,5- diazabicyclo[2.2.1]heptyl; 1,1-dioxide-thiomorpholinyl; each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, mono- or dialkylaminoalkyl, alkylthio, alkyloxyalkyl, alkylthioalkyl, aryl, piperidinyl optionally substituted with alkyl, pyrrolidinyl optionally substituted with arylalkyl, pyridyl or pyrimidinyl; or
- R⁴ and R⁵: together with the nitrogen atom to which they are attached form a radical selected from the group consisting piperidinyl or piperazinyl, each substituted with aryl, alkylcarbonyl, piperidinyl or pyrrolidinyl optionally substituted with arylalkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H*-azepinyl, hexahydro-1*H*-1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from aryl, haloalkyl, halo, arylalkyl, hydroxy, alkyloxy, amino, mono- or dialkylamino, alkylthio, alkylthioalkyl, aryl, pyridyl or pyrimidinyl;
- R⁶: is aryl¹ or Het;
- R⁷: is hydrogen, halo, alkyl, aryl or Het;
- R⁸: is hydrogen or alkyl;
- R⁹: is oxo; or
- R⁸ and R⁹: together form the radical -CH=CH-N=;
- R¹¹: is hydrogen or alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, C₂₋₆alkenyl optionally substituted with phenyl, haloalkyl, alkyloxy, haloalkyloxy, carboxyl, alkyloxycarbonyl, aminocarbonyl, morpholinyl or mono- or dialkylaminocarbonyl;
- aryl¹: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, alkylthio, haloalkyloxy, carboxyl, alkyloxycarbonyl, aminocarbonyl, morpholinyl, Het or mono- or dialkylaminocarbonyl;
- Het: is a monocyclic heterocycle selected from *N*-phenoxypiperidinyl, piperidinyl, piperazine, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, alkyl or alkyloxy;
provided R⁵ is other than benzyl;
the *N*-oxides thereof, the pharmaceutically acceptable salts thereof or the solvates thereof.

Whenever used herein, the term "compounds of formula (Ia) or (Ib)" or "compounds according to the invention" is meant to also include their pharmaceutically acceptable salts or their *N*-oxide forms or their solvates.

The compounds of formula (Ia) and (Ib) are interrelated in that e.g. a compound according to formula (Ib), with R⁹ equal to oxo and R⁸ equal to hydrogen, is the tautomeric equivalent of a compound according to formula (Ia) with R² equal to hydroxy (keto-enol tautomerism).

In the definition of Het, it is meant to include all the possible isomeric forms of the heterocycles, for instance, pyrrolyl comprises 1*H*-pyrrotyl and 2*H*-pyrrolyl.

The aryl, aryl¹ or Het listed in the definitions of the substituents of the compounds of formula (Ia) or (Ib) (see for instance R³ or R⁶) as mentioned hereinbefore or hereinafter may be attached to the remainder of the molecule of formula (Ia) or (Ib) through any ring carbon or heteroatom as appropriate, if not otherwise specified. Thus, for example, when Het is imidazolyl, it may be 1-imidazolyl, 2-imidazolyl, 4-imidazolyl and the like.

Lines drawn from substituents into ring systems indicate that the bond may be attached to any of the suitable ring atoms.

The pharmaceutically acceptable salts as mentioned hereinbefore or hereinafter are meant to comprise the therapeutically active non-toxic acid addition salt forms which the compounds according to formula (Ia) or formula (Ib) are able to form. Said acid addition salts can be obtained by treating the base form of the compounds according to formula (Ia) or formula (Ib) with appropriate acids, for example inorganic acids, for example hydrohalic acid, in particular hydrochloric acid, hydrobromic acid, sulfuric acid, nitric acid and phosphoric acid; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, methanesulfonic acid, ethanesulfonic acid, benzenesulfonic acid, p-toluenesulfonic acid, cyclamic acid, salicyclic acid, p-aminosalicylic acid and pamoic acid.

The compounds of formula (Ia) or (Ib) containing acidic protons may be converted into their therapeutically active non-toxic metal or amine addition salt forms by treatment with appropriate organic and inorganic bases. The pharmaceutically acceptable salts as mentioned hereinbefore or hereinafter are meant to also comprise the therapeutically active non-toxic metal or amine addition salt forms (base addition salt forms) which the compounds of formula (Ia) or (Ib) are able to form. Appropriate base addition salt forms comprise, for example, the ammonium salts, the alkali and earth alkaline metal salts, e.g. the lithium, sodium, potassium, magnesium, calcium salts and the like, salts with organic bases, e.g. primary, secondary and tertiary aliphatic and aromatic amines such as methylamine, ethylamine, propylamine, isopropylamine, the four butylamine isomers, dimethylamine, diethylamine, diethanolamine, dipropylamine, diisopropylamine, di-n-butylamine, pyrrolidine, piperidine, morpholine, trimethylamine, triethylamine, tripropylamine, quinuclidine, pyridine, quinoline and isoquinoline, the benzathine, *N*-methyl-D-glucamine, 2-amino-2-(hydroxymethyl)-1,3-propanediol, hydrabamine salts, and salts with amino acids such as, for example, arginine, lysine and the like.

Conversely, said acid or base addition salt forms can be converted into the free forms by treatment with an appropriate base or acid.

The term pharmaceutically acceptable salt also comprises the quaternary ammonium salts (quaternary amines) which the compounds of formula (Ia) or (Ib) are able to form by reaction between a basic nitrogen of a compound of formula (Ia) or (Ib) and an appropriate quaternizing agent, such as, for example, an optionally substituted C₁₋₆alkythalide, arylC₁₋₆alkythalide, C₁₋₆alkylcarbonylhatide, arylcarbonylhalide, HetC₁₋₆alkylhalide or Hetcarbonylhalide, e.g. methyliodide or benzyliodide. Preferably, Het represents a monocyclic heterocycle selected from furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halo, alkyl and aryl. Preferably, the quaternizing agent is C₁₋₆alkylhalide. Other reactants with good leaving groups may also be used, such as C₁₋₆alkyl trifluoromethanesulfonates, C₁₋₆alkyl methanesulfonates, and C₁₋₆alkyl p-toluenesulfonates. A quaternary amine has a positively charged nitrogen. Pharmaceutically acceptable counterions include chloro, bromo, iodo, trifluoroacetate, acetate, triflate, sulfate, sulfonate. Preferably, the counterion is iodo. The counterion of choice can be introduced using ion exchange resins.

The term solvate comprises the hydrates and solvent addition forms which the compounds of formula (Ia) or (Ib) are able to form, as well as the salts thereof. Examples of such forms are e.g. hydrates, alcoholates and the like.

In the framework of this application, a compound according to the invention is inherently intended to comprise all stereochemically isomeric forms thereof. The term "stereochemically isomeric forms" as used hereinbefore or hereinafter defines all the possible stereoisomeric forms which the compounds of formula (Ia) and (Ib), and their *N*-oxides, pharmaceutically acceptable salts, solvates or physiologically functional derivatives may possess. Unless otherwise mentioned or indicated, the chemical designation of compounds denotes the mixture of all possible stereochemically isomeric forms.
In particular, stereogenic centers may have the R- or S-configuration; substituents on bivalent cyclic (partially) saturated radicals may have either the *cis*- or *trans-*configuration. Compounds encompassing double bonds can have an E (entgegen) or Z (zusammen) -stereochemistry at said double bond. The terms cis, trans, R, S, E and Z are well known to a person skilled in the art.
Stereochemically isomeric forms of the compounds of formula (Ia) and (Ib) are obviously intended to be embraced within the scope of this invention.
Of special interest are those compounds of formula (Ia) or (Ib) which are stereochemically pure.

Following CAS-nomenclature conventions, when two stereogenic centers of known absolute configuration are present in a molecule, an *R* or *S* descriptor is assigned (based on Cahn-Ingo ld-Prelog sequence rule) to the lowest-numbered chiral center, the reference center. The configuration of the second stereogenic center is indicated using relative descriptors [*R**,*R** ] or [*R**,*S**], where *R** is always specified as the reference center and [*R**,*R**] indicates centers with the same chirality and [*R**,*S**] indicates centers of unlike chirality. For example, if the lowest-numbered chiral center in the molecule has an S configuration and the second center is *R*, the stereo descriptor would be specified as *S*-[*R**,*S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system relative to the position of the highest priority substituent on the reference atom is denominated "α", if it is on the same side of the mean plane determined by the ring system, or "β", if it is on the other side of the mean plane determined by the ring system.

When a specific stereoisomeric form is indicated, this means that said form is substantially free, *i.e.* associated with less than 50 %, preferably less than 20 %, more preferably less than 10 %, even more preferably less than 5 %, further preferably less than 2 % and most preferably less than 1 % of the other isomer(s). Thus, when a compound of formula (Ia) or (Ib) is for instance specified as (R,S), this means that the compound is substantially free of the (S,R) isomer.

Compounds of either formula (Ia) and (Ib) and some of the intermediate compounds invariably have at least two stereogenic centers in their structure which may lead to at least 4 stereochemically different structures.

The compounds of either formula (Ia) and (Ib) may be synthesized in the form of mixtures, in particular racemic mixtures, of enantiomers which can be separated from one another following art-known resolution procedures. The racemic compounds of either formula (Ia) and (Ib) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of either formula (Ia) and (Ib) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The tautomeric forms of the compounds of formula (Ia) or (Ib) are meant to comprise those compounds of formula (Ia) or (Ib) wherein e.g. an enol group is converted into a keto group (keto-enol tautomerism). Tautomeric forms of the compounds of formula (Ia) and (Ib) or of intermediates of the present invention are intended to be embraced by the ambit of this invention.

The *N*-oxide forms of the present compounds are meant to comprise the compounds of formula (Ia) or (Ib) wherein one or several tertiary nitrogen atoms are oxidized to the so-called *N*-oxide.

The compounds of formula (Ia) and (Ib) may be converted to the corresponding *N*-oxide forms following art-known procedures for converting a trivalent nitrogen into its *N*-oxide form. Said *N*-oxidation reaction may generally be carried out by reacting the starting material of formula (Ia) or (Ib) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. t.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

In the framework of this application, a compound according to the invention is inherently intended to comprise all isotopic combinations of its chemical elements. In the framework of this application, a chemical element, in particular when mentioned in relation to a compound according to formula (Ia) or (Ib), comprises all isotopes and isotopic mixtures of this element, either naturally occuring or synthetically produced, either with natural abundance or in an isotopically enriched form. In particular, when hydrogen is mentioned, it is understood to refer to ¹H, ²H, ³H and mixtures thereof ; when carbon is mentioned, it is understood to refer to ¹¹C, ¹²C, ¹³C, ¹⁴C and mixtures thereof ; when nitrogen is mentioned, it is understood to refer to ¹³N, ¹⁴N, ¹⁵N and mixtures thereof ; when oxygen is mentioned, it is understood to refer to ¹⁴O, ¹⁵O, ¹⁶O, ¹⁷O, ¹⁸O and mixtures thereof; and when fluor is mentioned, it is understood to refer to ¹⁸F, ¹⁹F and mixtures thereof.

A compound according to the invention therefore inherently comprises a compound with one or more isotopes of one or more element, and mixtures thereof, including a radioactive compound, also called radiolabelled compound, wherein one or more nonradioactive atoms has been replaced by one of its radioactive isotopes. By the term "radiolabelled compound" is meant any compound according to formula (Ia) or (Ib), a pharmaceutically acceptable salt thereof or an *N*-oxide form thereof or a solvate thereof, which contains at least one radioactive atom. For example, a compound can be labelled with positron or with gamma emitting radioactive isotopes. For radioligand-binding techniques (membrane receptor assay), the ³H-atom or the ¹²⁵I-atom is the atom of choice to be replaced. For imaging, the most commonly used positron emitting (PET) radioactive isotopes are ¹¹C, ¹⁸F, ¹⁵O and ¹³N, all of which are accelerator produced and have half-lives of 20, 100, 2 and 10 minutes respectively. Since the half-lives of these radioactive isotopes are so short, it is only feasible to use them at institutions which have an accelerator on site for their production, thus limiting their use. The most widely used of these are ¹⁸F, ^{99m}Tc, ²⁰¹Tl and ¹²³I. The handling of these radioactive isotopes, their production, isolation and incorporation in a molecule are known to the skilled person.

In particular, the radioactive atom is selected from the group of hydrogen, carbon, nitrogen, sulfur, oxygen and halogen. Preferably, the radioactive atom is selected from the group of hydrogen, carbon and halogen.

In particular, the radioactive isotope is selected from the group of ¹H, ¹¹C, ¹⁸F, ¹²²I, ¹²³I, ¹²⁵I, ¹³¹I, ⁷⁵Br, ⁷⁶Br, ⁷⁷Sr and ⁸²Br. Preferably, the radioactive isotope is selected from the group of ³H, ¹¹C and ¹⁸F.

In the framework of this application, alkyl is a straight or branched saturated hydrocarbon radical having from 1 to 6 carbon atoms ; or is a cyclic saturated hydrocarbon radical having from 3 to 6 carbon atoms ; or is a cyclic saturated hydrocarbon radical having from 3 to 6 carbon atoms attached to a straight or branched saturated hydrocarbon radical having from 1 to 6 carbon atoms ; wherein each carbon atom can be optionally substituted with cyano, hydroxy, C₁₋₆alkyloxy or oxo. Preferably alkyl is a straight or branched saturated hydrocarbon radical having from 1 to 6 carbon atoms ; or is a cyclic saturated hydrocarbon radical having from 3 to 6 carbon atoms ; wherein each carbon atom can be optionally substituted with hydroxyl or C₁₋₆alkyloxy.
Preferably, alkyl is methyl, ethyl or cyclohexylmethyl, more preferably methyl or ethyl. An interesting embodiment of alkyl in all definitions used hereinbefore or hereinafter is C₁₋₆alkyl which represents a straight or branched saturated hydrocarbon radical having from 1 to 6 carbon atoms such as for example methyl, ethyl, propyl, 2-methyl-ethyl, pentyl, hexyl and the like. A preferred subgroup of C₁₋₆alky is C₁₋₄alkyl which represents a straight or branched saturated hydrocarbon radical having from 1 to 4 carbon atoms such as for example methyl, ethyl, propyl, 2-methyl-ethyl and the like.

In the framework of this application C₂₋₆alkenyl is a straight or branched hydrocarbon radical having from 2 to 6 carbon atoms containing a double bond such as ethenyl, propenyl, butenyl, pentenyl, hexenyl and the like; C₂₋₆alkynyl is a straight or branched hydrocarbon radical having from 2 to 6 carbon atoms containing a triple bond such as ethynyl, propynyl, butynyl, pentynyl, hexynyl and the like; C₃₋₆cycloalkyl is a cyclic saturated hydrocarbon radical having from 3 to 6 carbon atoms and is generic to cyclopropyl, cyclobutyl, cyclopentyl, cyclohexyl

In the framework of this application, halo is a substituent selected from the group of fluoro, chloro, bromo and iodo and haloalkyl is a straight or branched saturated hydrocarbon radical having from 1 to 6 carbon atoms or a cyclic saturated hydrocarbon radical having from 3 to 6 carbon atoms or a cyclic saturated hydrocarbon radical having from 3 to 6 carbon atoms attached to a straight or branched saturated hydrocarbon radical having from I to 6 carbon atoms; wherein one or more carbon atoms are substituted with one or more halo atoms. Preferably, halo is bromo, fluoro or chloro; in particular chloro or bromo. Preferably, haloalkyl is polyhaloC₁₋₆alkyl which is defined as mono- or polyhalosubstituted C₁₋₆alkyl, for example, methyl with one or more fluoro atoms, for example, difluoromethyl or trifluoromethyl, 1,1-diftuoro-ethyt and the like. In case more than one halo atom is attached to an alkyl or C₁₋₆alkyl group within the definition of haloalkyl or polyhaloC₁₋₆alkyl, they may be the same or different.

A first interesting embodiment relates to a compound of formula (Ia) or (Ib) wherein
- p: is an integer equal to 1, 2, 3 or 4;
- q: is an integer equal to zero, 1, 2, 3 or 4 ;
- R¹: is hydrogen, cyano, formyl, carboxyl, halo, alkyl, C₂₋₆alkenyl, C₂₋₆alkynyl, haloalkyl, hydroxy, alkyloxy, alkylthio, alkylthioalkyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylalkyl, arylcarbonyl, R^{5a}R^{4a}Nalkyl, di(aryl)alkyl, aryl, R^{5a}R^{4a}N-, R^{5a}R^{4a}N-C(=O)-, or Het;
- R²: is hydrogen, alkyloxy, aryl, aryloxy, hydroxy, mercapto, alkyloxyalkyloxy, alkylthio, mono or di(alkyl)amino, pyrrolidino or a radical of formula 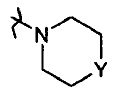 wherein Y is CH₂, O, S, NH or *N*-alkyl ;
- R³: is alkyl, arylalkyl, aryl-O-alkyl, aryl-alkyl-O-alkyl, aryl, Het, Het- alkyl, Het-O-alkyl, Het-alkyl-O-alkyl or 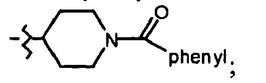
- R⁴: is hydrogen or alkyl;
- R⁵: is -C(=NH)-NH₂; arylalkyl; Het-alkyl; mono- or dialkylaminoalkyl; Het; or aryl; or
- R⁴ and R⁵: together with the nitrogen atom to which they are attached form a radical selected from the group consisting of 2,3-dihydroisoindol-1- yl; thiazolidin-3-yl; 1,2,3,6-tetrahydropyridyl; hexahydro-1*H*- azepinyl; hexahydro-1*H*-1,4-diazepinyl; hexahydro-1,4-oxazepinyl; 1,2,3,4-tetrahydroisoquinolin-2-yl or 2,5-diazabicyclo[2.2.]heptyt; each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, alkylthio, alkyloxyalkyl, alkylthioalkyl, aryl, pyridyl or pyrimidinyl; or
- R⁴ and R⁵: together with the nitrogen atom to which they are attached form a radical selected from the group consisting piperidinyl or piperazinyl, each substituted with aryl, alkylcarbonyl, piperidinyl or pyrrolidinyl optionally substituted with arylalkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H*-azepinyl, hexahydro-1*H*-1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, halo, arylalkyl, hydroxy, alkyloxy, amino, mono- or dialkylamino, alkylthio, alkylthioalkyl, aryl, pyridyl or pyrimidinyl;
- R⁶: is aryl¹ or Het;
- R⁷: is hydrogen, halo, alkyl, aryl or Het;
- R⁸: is hydrogen or alkyl;
- R⁹: is oxo; or
- R⁸ and R⁹: together form the radical -CH=CH-N=;
- R¹¹: is hydrogen or alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl or mono- or dialkylaminocarbonyl;
- aryl¹: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, alkylthio, haloalkyloxy, carboxyl, alkyloxycarbonyl, aminocarbonyl, morpholinyl, Het or mono- or dialkylaminocarbonyl;
- Het: is a monocyclic heterocycle selected from *N*-phenoxypiperidinyl, piperidinyl, piperazine, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, alkyl or alkyloxy;
provided R⁵ is other than benzyl.

A second interesting embodiment relates to a compound of formula (Ia) or (Ib) wherein
- p: is an integer equal to 1, 2, 3 or 4;
- q: is an integer equal to zero, 1, 2, 3 or 4 ;
- R¹: is hydrogen, cyano, formyl, carboxyl, halo, C₁₋₆alkyl, C₂₋₆alkenyl, C₂₋₆alkynyl, haloC₁₋₆alkyl, hydroxy, C₁₋₆alkyloxy, C₁₋₆alkylthio, C₁₋₆alkylthioC₁₋₆alkyl, -C=N-OR¹¹, amino, mono or di(C₁₋₆alkyl)amino, aminoC₁₋₆alkyl, mono or di(C₁₋₆alkyl)aminoC₁₋₆alkyl, C₁₋₆alkylcarbonylaminoC₁₋₆alkyl, aminocarbonyl, mono or di(C₁₋₆alkyl)aminocarbonyl, arylC₁₋₆alkyl, arylcarbonyl, R^{5a}R^{4a}NC₁₋₆alkyl, di(aryl)C₁₋₆alkyl, aryl, R^{5a}R^{4a}N-, R^{5a}R^{4a}N-C(=O)-, or Het;
- R²: is hydrogen, C₁₋₆alkyloxy, aryl, aryloxy, hydroxy, mercapto, C₁₋₆alkyloxyC₁₋₆alkyloxy, C₁₋₆alkylthio, mono or di(C₁₋₆alkyl)amino, pyrrolidino or a radical of formula 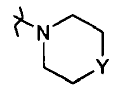 wherein Y is CH₂, O, S, NH or *N*-C₁₋₆alkyl ;
- R³: is C₁₋₆alkyl, arylC₁₋₆alkyl, aryl-O-C₁₋₆alkyl, aryl-alkyl-O-C₁₋₆alkyl, aryl, aryl-aryl, Het, Het-C₁₋₆alkyl, Het-O-C₁₋₆alkyl, Het-C₁₋₆alkyl-O-C₁₋₆alkyl or 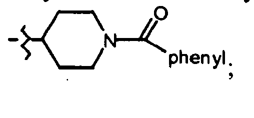
- R⁴: is hydrogen or C₁₋₆alkyl;
- R⁵: is -C(=NH)-NH₂; arylC₁₋₆alkyl; Het-C₁₋₆alkyl; mono- or diC₁₋₆alkylaminoC₁₋₆alkyl; bicyclo[2.2.1]heptyl; Het; or aryl; or
- R⁴ and R⁵: together with the nitrogen atom to which they are attached form a radical selected from the group consisting of azetidinyl; 2,3-dihydroisoindol-1-yl; thiazolidin-3-yl; 1,2,3,6-tetrahydropyridyl; hexahydro-1*H*-azepinyl; hexahydro-1*H*-1,4-diazepinyl; hexahydro-1,4- oxazepinyl; 1,2,3,4-tetrahydroisoquinolin-2-yl; 2,5-diazabicyclo[2.2.1]heptyl; or 1,1-dioxide-thiomorpholinyl; each radical optionally substituted with 1, 2, 3 or 4 substituents, each substituent independently selected from C₁₋₆alkyl, haloC₁₋₆alkyl, C₁₋₆alkylcarbonyl, halo, arylC₁₋₆alkyl, hydroxy, C₁₋₆alkyloxy, amino, mono- or diC₁₋₆alkylamino, mono- or diC₁₋₆alkylaminoC₁₋₆alkyl, C₁₋₆alkylthio, C₁₋₆alkyloxyC₁₋₆alkyl, C₁₋₆alkylthioC₁₋₆alkyl, aryl, piperidinyl optionally substituted with C₁₋₆alkyl, pyrrolidinyl optionally substituted with arylC₁₋₆alkyl, pyridyl or pyrimidinyl; or
- R⁴ and R⁵: together with the nitrogen atom to which they are attached form a radical selected from the group consisting piperidinyl or piperazinyl, each substituted with aryl, C₁₋₆alkylcarbonyl, piperidinyl or pyrrolidinyl optionally substituted with arylC₁₋₆alkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H*-azepinyl, hexahydro-1*H*-1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from C₁₋₆alkyl, haloC₁₋₆alkyl, halo, arylC₁₋₆alkyl, hydroxy, C₁₋₆alkyloxy, amino, mono- or diC₁₋₆alkylamino, C₁₋₆alkylthio, C₁₋₆alkylthioC₁₋₆alkyl, aryl, pyridyl or pyrimidinyl;
- R⁶: is aryl¹ or Het;
- R⁷: is hydrogen, halo, C₁₋₆alkyl, aryl or Het;
- R⁸: is hydrogen or C₁₋₆alkyl;
- R⁹: is oxo; or
- R⁸ and R⁹: together form the radical -CH=CH-N=;
- R¹¹: is hydrogen or C₁₋₆alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or diC₁₋₆alkylamino, C₁₋₆alkyl, C₂₋₆alkenyl optionally substituted with phenyl, haloC₁₋₆alkyl, C₁₋₆alkyloxy, haloC₁₋₆alkyloxy, carboxyl, C₁₋₆alkyloxycarbonyl, aminocarbonyl, morpholinyl or mono- or diC₁₋₆alkylaminocarbonyl;
- aryl¹: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or diC₁₋₆alkylamino, C₁₋₆alkyl, haloC₁₋₆alkyl, C₁₋₆alkyloxy, C₁₋₆alkylthio, haloC₁₋₆alkyloxy, carboxyl, C₁₋₆alkyloxycarbonyl, aminocarbonyl, morpholinyl, Het or mono- or diC₁₋₆alkylaminocarbonyl;
- Het: is a monocyclic heterocycle selected from *N*-phenoxypiperidinyl, piperidinyl, piperazine, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, C₁₋₆alkyl or C₁₋₆alkyloxy;
provided R⁵ is other than benzyl.

A third interesting embodiment relates to a compound of formula (Ia) or (Ib) wherein
- p: is an integer equal to 1, 2, 3 or 4;
- q: is an integer equal to zero, 1, 2, 3 or 4 ;
- R¹: is hydrogen, cyano, formyl, carboxyl, halo, C₁₋₆alkyl, C₂₋₆alkenyl, C₂₋₆alkynyl, polyhaloC₁₋₆alkyl, hydroxy, C₁₋₆alkyloxy, C₁₋₆alkylthio, C₁₋₆alkyloxyC₁₋₆alkyl, C₁₋₆alkylthioC₁₋₆alkyl, hydroxyC₁₋₆alkyl, -C=N-OR¹¹, amino, mono or di(C₁₋₆alkyl)amino, aminoC₁₋₆alkyl, mono or di(C₁₋₆alkyl)aminoC₁₋₆alkyl, C₁₋₆alkylcarbonylaminoC₁₋₆alkyl, aminocarbonyl, mono or di(C₁₋₆alkyl)aminocarbonyl, arylC₁₋₆alkyl, arylcarbonyl, R^{5a}R^{4a}NC₁₋₆alkyl, di(aryl)C₁₋₆alkyl, aryl, R^{5a}R^{4a}N-, R^{5a}R^{4a}N-C(=O)-, or Het;
- R²: is hydrogen, C₁₋₆alkyloxy, aryl, aryloxy, hydroxy, mercapto, C₁₋₆alkyloxyC₁₋₆alkyloxy, C₁₋₆alkylthio, mono or di(C₁₋₆alkyl)amino, pyrrolidino or a radical of formula 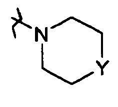 wherein Y is CH₂, O, S, NH or *N*-C₁₋₆alkyl ;
- R³: is C₁₋₆alkyl, C₁₋₆cycloalkyl, arylC₁₋₆alkyl, aryl-O-C₁₋₆alkyl, arylC₁₋₆alkyl-O-C₁₋₆alkyl, aryl, Het, Het-C₁₋₆alkyl, Het-O-C₁₋₆alkyl or HetC₁₋₆alkyl-O-C₁₋₆alkyl, or 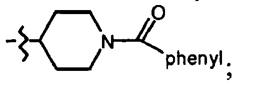
- R^{3a}: is hydrogen, cyano, C₁₋₆alkyl, C₁₋₆cycloalkyl, arylC₁₋₆alkyl, aryl-O-C₁₋₆alkyl, arylC₁₋₆alkyl-O-C₁₋₆alkyl, aryl, Het, Het-C₁₋₆alkyl, Het-O-C₁₋₆alkyl or HetC₁₋₆alkyl-O-C₁₋₆alkyl;
- R⁴: is hydrogen or C₁₋₆alkyl;
- R⁵: is -C(=NH)-NH₂; arylC₁₋₆alkyl; Het-C₁₋₆alkyl; mono- or diC₁₋₆alkylaminoC₁₋₆alkyl; Het; or aryl; or
- R⁴ and R⁵: together with the nitrogen atom to which they are attached form a radical selected from the group consisting of 2,3-dihydroisoindol-1-yl; thiazolidin-3-yl; 1,2,3,6-tetrahydropyridyl; hexahydro-1*H*-azepinyl; hexahydro-1*H*-1,4-diazepinyl; hexahydro-1,4-oxazepinyl; 1,2,3,4- tetrahydroisoquinolin-2-yl or 2,5-diazabicyclo[2.2.1]heptyl; each radical optionally substituted with 1, 2, 3 or 4 substituents, each substituent independently selected from C₁₋₆alkyl, haloC₁₋₆alkyl, C₁₋₆alkylcarbonyl, halo, arylC₁₋₆alkyl, hydroxy, C₁₋₆alkyloxy, amino, mono- or diC₁₋₆alkylamino, C₁₋₆alkylthio, C₁₋₆alkyloxyC₁₋₆alkyl, C₁₋₆alkylthioC₁₋₆alkyl, aryl, pyridyl or pyrimidinyl; or
- R⁴ and R⁵: together with the nitrogen atom to which they are attached form a radical selected from the group consisting piperidinyl or piperazinyl, each substituted with aryl, C₁₋₆alkylcarbonyl, piperidinyl or pyrrolidinyl optionally substituted with arylC₁₋₆alkyl;
- R^{4a} and R^{5a}: together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H*-azepinyl, hexahydro-1*H*-1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from C₁₋₆alkyl, polyhaloC₁₋₆alkyl, halo, arylC₁₋₆alkyl, hydroxy, C₁₋₆alkyloxy, C₁₋₆alkyloxyC₁₋₆alkyl, amino, mono- or di(C₁₋₆alkyl)amino, C₁₋₆alkylthio, C₁₋₆alkyloxyC₁₋₆alkyl, C₁₋₆alkylthioC₁₋₆alkyl, aryl, pyridyl or pyrimidinyl;
- R⁶: is aryl¹ or Het;
- R⁷: is hydrogen, halo, C₁₋₆alkyl, aryl or Het;
- R⁸: is hydrogen or C₁₋₆alkyl;
- R⁹: is oxo; or
- R⁸ and R⁹: together form the radical -CH=CH-N=;
- R¹¹: is hydrogen or C₁₋₆alkyl;
- aryl: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or di(C₁₋₆alkyl)amino, C₁₋₆alkyl, polyhaloC₁₋₆alkyl, C₁₋₆alkyloxy, haloC₁₋₆alkyloxy, carboxyl, C₁₋₆alkyloxycarbonyl, aminocarbonyl, morpholinyl or mono- or di(C₁₋₆alkyl)aminocarbonyl;
- aryl¹: is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or di(C₁₋₆alkyl)amino, C₁₋₆alkyl, polyhaloC₁₋₆alkyl, C₁₋₆alkyloxy, C₁₋₆alkylthio, haloC₁₋₆alkyloxy, carboxyl, C₁₋₆alkyloxycarbonyl, aminocarbonyl, morpholinyl, Het or mono- or di(C₁₋₆alkyl)aminocarbonyl;
- Het: is a monocyclic heterocycle selected from *N*-phenoxypipcridinyl, piperidinyl, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, C₁₋₆alkyl or C₁₋₆alkyloxy;
provided R⁵ is other than benzyl.

A fourth interesting embodiment relates to a compound of formula (Ia) or (Ib) or any subgroup thereof as mentioned hereinbefore as interesting embodiment wherein R¹ is hydrogen, cyano, halo, alkyl, haloalkyl, hydroxy, alkyloxy, alkylthio, alkyloxyalkyl, alkylthioalkyl, arylalkyl, di(aryl)alkyl, aryl, or Het; in particular R¹ is hydrogen, halo, aryl, Het, alkyl or alkyloxy; more in particular R¹ is hydrogen or halo. Most preferably, R¹ is halo, in particular bromo. Or R¹ represents formyl, carboxyl, C₂₋₆alkenyl, C₂₋₆alkynyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylcarbonyl, R^{5a}R^{4a}Nalkyl, R^{5a}R^{4a}N-, R^{5a}R^{4a}N-C(=O)-.

A fifth interesting embodiment relates to a compound of formula (Ia) or (Ib) or any subgroup thereof as mentioned hereinbefore as interesting embodiment wherein p is equal to 1.

A sixth interesting embodiment relates to a compound of formula (Ia) or (Ib) or any subgroup thereof as mentioned hereinbefore as interesting embodiment wherein R² is hydrogen, alkyloxy or alkylthio, in particular hydrogen, C₁₋₆alkyloxy or C₁₋₆alkylthio. More in particular, R² is C₁₋₆alkyloxy, preferably methyloxy.

A seventh interesting embodiment relates to a compound of formula (Ia) or (Ib) or any subgroup thereof as mentioned hereinbefore as interesting embodiment wherein R³ is alkyl, arylalkyl, aryl, or Het; in particular C₁₋₆alkyl, arylC₁₋₆alkyl, aryl, or Het; more in particular C₁₋₆alkyl, optionally substituted phenyl, optionally substituted naphthyl, arylC₁₋₆alkyl wherein aryl represents optionally substituted phenyl or optionally substituted naphthyl, or Het; even more in particular phenyl, naphthyl, arylC₁₋₆alkyl wherein aryl represents phenyl or naphthyl.

An eighth interesting embodiment relates to a compound of formula (Ia) or (Ib) or any subgroup thereof as mentioned hereinbefore as interesting embodiment wherein q is equal to 3.

A ninth interesting embodiment relates to a compound of formula (Ia) or (Ib) or any subgroup thereof as mentioned hereinbefore as interesting embodiment wherein R⁴ is hydrogen or alkyl; in particular hydrogen or C₁₋₆alkyl; more in particular hydrogen or methyl; even more in particular methyl.

A tenth interesting embodiment relates to a compound of formula (Ia) or (Ib) or any subgroup thereof as mentioned hereinbefore as interesting embodiment wherein R⁵ is -C(=NH)-NH₂; Het-alkyl; mono- or dialkylaminoalkyl; Het; bicyclo[2.2.1]heptyl or aryl; in particular R⁵ is -C(=NH)-NH₂; Het-alkyl; Het; mono- or dialkylaminoalkyl; or bicyclo[2.2.1]heptyl; more in particular R⁵ is -C(=NH)-NH₂; Het-alkyl; Het; or bicyclo[2.2.1]heptyl.

An eleventh interesting embodiment relates to a compound of formula (Ia) or (Ib) or any subgroup thereof as mentioned hereinbefore as interesting embodiment wherein R⁴ and R⁵ together with the nitrogen atom to which they are attached form a radical selected from the group consisting of azetidinyl; 2,3-dihydroisoindol-1-yl; thiazolidin-3-yl; 1,2,3,6-tetrahydropyridyl; hexahydro-1*H*-azepinyl; hexahydro-1*H*-1,4-diazepinyl; hexahydro-1,4-oxazepinyl; 1,2,3,4-tetrahydroisoquinolin-2-yl; 2,5-diazabicyclo[2.2.1]heptyl; 1,1-dioxide-thiomorpholinyl; each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, mono- or dialkylaminoalkyl, alkylthio, alkyloxyalkyl, alkylthioalkyl, aryl, piperidinyl optionally substituted with alkyl, pyrrolidinyl optionally substituted with arylalkyl, pyridyl or pyrimidinyl; or R⁴ and R⁵ together with the nitrogen atom to which they are attached form a radical selected from the group consisting piperidinyl or piperazinyl, each substituted with aryl, alkylcarbonyl, piperidinyl or pyrrolidinyl optionally substituted with arylalkyl; in particular R⁴ and R⁵ together with the nitrogen atom to which they are attached form a radical selected from the group consisting of azetidinyl, hexahydro-1*H*-1,4-diazepinyl, 2,5-diazabicyclo[2.2.1]heptyl or hexahydro-1*H*-azepinyl; each radical optionally substituted with 1, 2, 3 or 4 substituents, each substituent independently selected from alkyl or arylalkyl; or R⁴ and R⁵ together with the nitrogen atom to which they are attached form a radical selected from the group consisting piperidinyl or piperazinyl, each substituted with aryl, alkylcarbonyl, piperidinyl or pyrrolidinyl optionally substituted with arylalkyl; more in particular R⁴ and R⁵ together with the nitrogen atom to which they are attached form a radical selected from the group consisting of azetidinyl, hexahydro-1*H*-1,4-diazepinyl, 2,5-diazabicyclo[2.2.1]heptyl or hexahydro-1*H*-azepinyl; each radical optionally substituted with 1, 2, 3 or 4 substituents, each substituent independently selected from C₁₋₆alkyl or arylC₁₋₆alkyl; or R⁴ and R⁵ together with the nitrogen atom to which they are attached form a radical selected from the group consisting of piperidinyl or piperazinyl, each substituted with aryl, C₁₋₆alkylcarbonyl, piperidinyl or pyrrolidinyl optionally substituted with arylC₁₋₆alkyl.

A twelfth interesting embodiment relates to a compound of formula (Ia) or (Ib) or any subgroup thereof as mentioned hereinbefore as interesting embodiment wherein R⁴ and R⁵ together with the nitrogen atom to which they are attached form a radical selected from the group consisting of azetidinyl; 2,3-dihydroisoindol-1-yl; thiazolidin-3-yl; 1,2,3,6-tetrahydropyridyl; hexahydro-1*H*-azepinyl; hexahydro-1*H*-1,4-diazepinyl; hexahydro-1,4-oxazepinyl; 1,2,3,4-tetrahydroisoquinolin-2-yl; 2,5-diazabicyclo[2.2.1]heptyl; 1,1-dioxide-thiomorpholinyl; each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, mono- or dialkylaminoalkyl, alkylthio, alkyloxyalkyl, alkylthioalkyl, aryl, piperidinyl optionally substituted with alkyl, pyrrolidinyl optionally substituted with arylalkyl, pyridyl or pyrimidinyl; in particular R⁴ and R⁵ together with the nitrogen atom to which they are attached form a radical selected from the group consisting of azetidinyl; 2,3-dihydroisoindol-1-yl; thiazolidin-3-yl; 1,2,3,6-tetrahydropyridyl; hexahydro-1*H*-azepinyl; hexahydro-1*H*-1,4-diazepinyl; hexahydro-1,4-oxazepinyl; 1,2,3,4-tetrahydroisoquinolin-2-yl; 2,5-diazabicyclo[2.2.1]heptyl; 1,1-dioxide-thiomorpholinyl; each radical optionally substituted with 1, 2, 3 or 4 substituents, each substituent independently selected from C₁₋₆alkyl, haloC₁₋₆alkyl, C₁₋₆alkylcarbonyl, halo, arylC₁₋₆alkyl, hydroxy, C₁₋₆alkyloxy, amino, mono- or diC₁₋₆alkylamino, mono- or diC₁₋₆alkylaminoC₁₋₆alkyl, C₁₋₆alkylthio, C₁₋₆alkyloxyC₁₋₆alkyl, C₁₋₆alkylthioC₁₋₆alkyl, aryl, piperidinyl optionally substituted with C₁₋₆alkyl, pyrrolidinyl optionally substituted with arylC₁₋₆alkyl, pyridyl or pyrimidinyl; more in particular R⁴ and R⁵ together with the nitrogen atom to which they are attached form a radical selected from the group consisting of azetidinyl, hexahydro-1*H*-1,4-diazepinyl, 2,5-diazabicyclo[2.2.1]heptyl or hexahydro-1*H*-azepinyl; each radical optionally substituted with 1, 2, 3 or 4 substituents, each substituent independently selected from alkyl or arylalkyl; even more in particular R⁴ and R⁵ together with the nitrogen atom to which they are attached form a radical selected from the group consisting of azetidinyl, hexahydro-1*H*-1,4-diazepinyl, 2,5-diazabicyclo[2.2.1]heptyl or hexahydro-1*H*-azepinyl; each radical optionally substituted with 1, 2, 3 or 4 substituents, each substituent independently selected from C₁₋₆alkyl or arylC₁₋₆alkyl.

A thirteenth interesting embodiment relates to a compound of formula (Ia) or (Ib) or any subgroup thereof as mentioned hereinbefore as interesting embodiment wherein R⁶ is aryl¹; in particular phenyl optionally substituted with halo, cyano or C₁₋₆alkyloxy; more in particular phenyl optionally substituted with halo; even more in particular phenyl.

A fourteenth interesting embodiment relates to a compound of formula (Ia) or (Ib) or any subgroup thereof as mentioned hereinbefore as interesting embodiment wherein R⁷ is hydrogen.

A fifteenth interesting embodiment relates to a compound of formula (Ia) or (Ib) or any subgroup thereof as mentioned hereinbefore as interesting embodiment wherein the compound is a compound of formula (Ib) and wherein R⁸ is hydrogen and R⁹ is oxo.

A sixteenth interesting embodiment relates to a compound of formula (Ia) or (Ib) or any subgroup thereof as mentioned hereinbefore as interesting embodiment wherein the compound is a compound of formula (Ia).

A seventeenth interesting embodiment relates to a compound of formula (Ia) or (Ib) or any subgroup thereof as mentioned hereinbefore as interesting embodiment wherein the compound is a compound of formula (Ib), in particular wherein R⁸ is alkyl, more preferable C₁₋₆alkyl, e.g. methyl.

An eighteenth interesting embodiment is a compound of formula (Ia) or (Ib) or any subgroup thereof as mentioned hereinbefore as interesting embodiment wherein aryl is naphthyl or phenyl, more preferably phenyl, each optionally substituted with one or two substituents selected from halo, for example chloro; cyano; alkyl for example methyl; or alkyloxy, for example methyloxy.

A nineteenth interesting embodiment relates to a compound of formula (Ia) or (Ib) or any subgroup thereof as mentioned hereinbefore as interesting embodiment wherein R¹ is placed in position 6 of the quinoline ring.
In the framework of this application, the quinoline ring of the compounds of formula (Ia) or (Ib) is numbered as follows :

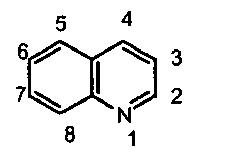

A twentieth interesting embodiment is the use of a compound of formula (Ia) or (Ib) or any subgroup thereof as mentioned hereinbefore as interesting embodiment for the manufacture of a medicament for the treatment of a bacterial infection with a gram-positive and/or a gram-negative bacterium, preferably a bacterial infection with a gram-positive bacterium.

A twenty first interesting embodiment is the use of a compound of formula (Ia) or (Ib) or any subgroup thereof as mentioned hereinbefore as interesting embodiment for the manufacture of a medicament for the treatment of a bacterial infection wherein the compound of formula (Ia) or (Ib) has a IC₉₀ < 15 µl/ml against at least one bacterium, in particular a gram-positive bacterium; preferably a IC₉₀ < 10 µl/ml; more preferably a IC₉₀ < 5 µl/ml; the IC₉₀ value being determined as described hereinafter.

A twenty second interesting embodiment relates to a compound of formula (Ia) or any subgroup thereof as mentioned hereinbefore as interesting embodiment wherein one or more, preferably all, of the following definitions apply :
R¹ is hydrogen or halo; in particular hydrogen or bromo;
R² is alkyloxy, in particular C₁₋₆alkyloxy; preferably methyloxy;
R³ is arylalkyl or aryl; in particular arylC₁₋₆alkyl or aryl; more in particular phenyl, naphthyl or phenylC₁₋₆alkyl;
R⁴ is hydrogen or alkyl; in particular hydrogen or C₁₋₆alkyl; more in particular hydrogen or methyl;
R⁵ is -C(=NH)-NH₂; Het-C₁₋₆alkyl; mono- or dialkylaminoalkyl;
bicyclo[2.2.1]heptyl;or Het; in particular -C(=NH)-NH₂; Het-C₁₋₆alkyl; mono- or di(C₁₋₆alkyl)aminoC₁₋₆alkyl; bicyclo[2.2.1]heptyl;or Het; or
R⁴ and R⁵ together with the nitrogen atom to which they are attached form a radical selected from the group consisting of azetidinyl; hexahydro-1*H*-azepinyl; hexahydro-1*H*-1,4-diazepinyl; 2,5-diazabicyclo[2.2.1]heptyl; or 1,1-dioxide-thiomorpholinyl; each radical optionally substituted with 1, 2, 3 or 4 substituents, each substituent independently selected from alkyl, arylalkyl, piperidinyl optionally substituted with alkyl; or R⁴ and R⁵ together with the nitrogen atom to which they are attached form a radical selected from the group consisting of piperidinyl or piperazinyl, each substituted with aryl, alkylcarbonyl, piperidinyl or pyrrolidinyl optionally substituted with arylalkyl; in particular R⁴ and R⁵ together with the nitrogen atom to which they are attached form a radical selected from the group consisting of azetidinyl; hexahydro-1*H-*azepinyl; hexahydro-1*H*-1,4-diazepinyl; 2,5-diazabicyclo[2.2.1]heptyl; or 1,1-dioxide-thiomorpholinyl; each radical optionally substituted with 1, 2, 3 or 4 substituents, each substituent independently selected from C₁₋₆alkyl, arylC₁₋₆alkyl, piperidinyl optionally substituted with C₁₋₆alky; or R⁴ and R⁵ together with the nitrogen atom to which they are attached form a radical selected from the group consisting of piperidinyl or piperazinyl, each substituted with aryl, C₁₋₆alkylcarbonyl, piperidinyl or pyrrolidinyl optionally substituted with arylC₁₋₆alkyl;
R⁶ is phenyl optionally substituted with halo, e.g. chloro;
R⁷ is hydrogen;
q is 3 or 4;
p is 1.

Preferably, in the compounds of formula (Ia) and (Ib) or any subgroup thereof as mentioned hereinbefore as interesting embodiment, the term "alkyl" represents C₁₋₆alkyl, more preferably C₁₋₄alkyl, and the term haloalkyl represents polyhaloC₁₋₆alkyl.

Preferably, the compound of formula (Ia) or (Ib) is a particular mixture of enantiomers (hereinafter indicated as a particular A or B diastereoisomer) and hence is substantially free of the other diastereoisomer(s)). In case the compound of formula (Ia) or (Ib) has two chiral centers, this means that the compound is a mixture, in particular a racemic mixture, of the (R,S) and (S,R) enantiomers or a mixture, in particular a racemic mixture, of the (R,R) and (S,S) enantiomer. Hereinafter, the mixtures, in particular the racemic mixtures, of 2 enantiomers are indicated as diastereoisomer A or B. Whether the racemic mixture is indicated as A or B depends on whether it is first isolated in the synthesis protocol (i.e. A) or second (i.e. B). More preferably, the compound of formula (Ia) or (Ib) is a particular enantiomer (substantially free of the other enantiomers). In case the compound of formula (Ia) or (Ib) has two chiral centers this means that the compound is the (R,S), (S,R), (R,R) or (S,S) enantiomer. Hereinafter, said particular enantiomers are indicated as A1, A2, B1 or B2. Whether the enantiomer is indicated as A1, A2, B1 or B2 depends on whether it is isolated first or second (1 or 2) in the synthesis protocol and whether it is separated from the A (A1, A2) or B (B1, B2) diastereoisomer.

Preferred compounds according to the present invention are selected from 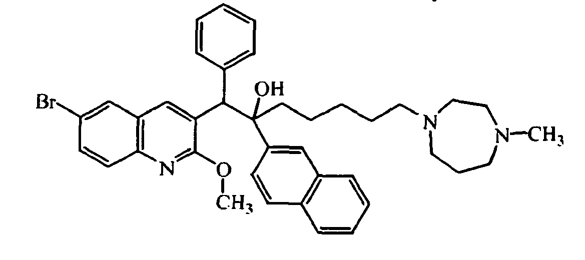 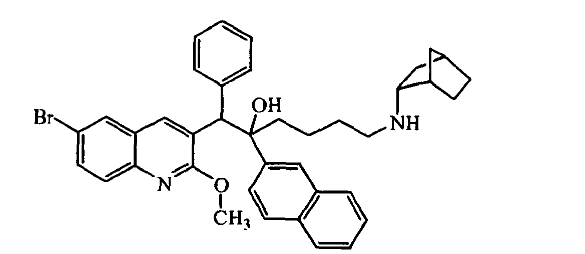 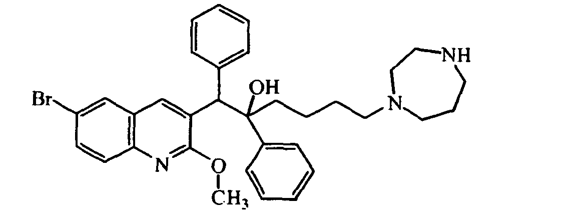 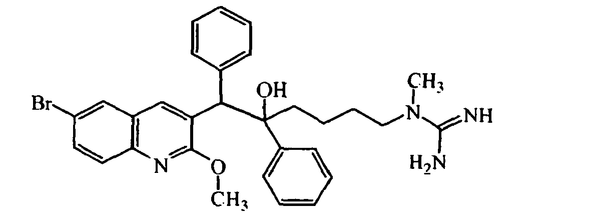 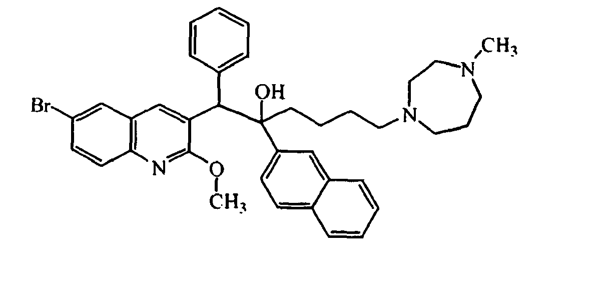 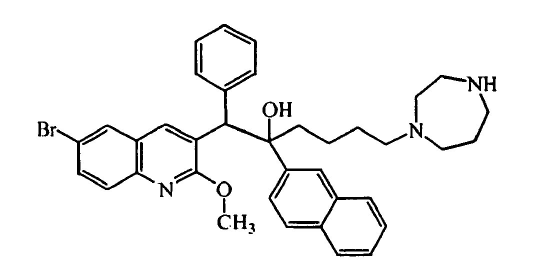 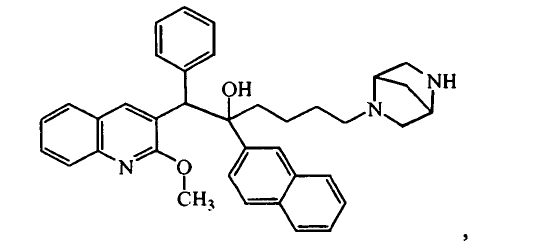 including any stereochemically isomeric form thereof;
a *N*-oxide thereof, a pharmaceutically acceptable salt thereof or a solvate thereof.

In particular, preferred compounds according to the present invention are compounds 53, 40, 47, 2, 33, 18, and 34d (see Tables hereinafter); a *N*-oxide thereof, a pharmaceutically acceptable salt thereof or a solvate thereof.

### PHARMACOLOGY

The compounds according to the invention have surprisingly been shown to be suitable for the treatment of a bacterial infection including a mycobacterial infection, particularly those diseases caused by pathogenic mycobacteria such as *Mycobacterium tuberculosis* (including the latent and drug resistant form thereof), *M. bovis, M. avium, M. leprae* and *M. marinum.* The present invention thus also relates to compounds of formula (Ia) or (Ib) as defined hereinabove, the pharmaceutically acceptable salts thereof or the *N*-oxide forms thereof or the solvates thereof, for use as a medicine, in particular for use as a medicine for the treatment of a bacterial infection including a mycobacterial infection.

Further, the present invention also relates to the use of a compound of formula (Ia) or (Ib), the pharmaceutically acceptable salts thereof or the *N*-oxide forms thereof or the solvates thereof, as well as any of the pharmaceutical compositions thereof as described hereinafter for the manufacture of a medicament for the treatment of a bacterial infection including a mycobacterial infection.

Accordingly, in another aspect, the invention provides for compounds as specified in the claims for use in a method of treating a patent suffering from, or at risk of, a bacterial infection, including a mycobacterial infection, which comprises administering to the patient a therapeutically effective amount of a compound or pharmaceutical composition according to the invention.

In addition to their activity against mycobacteria, the compounds according to the invention are also active against other bacteria. In general, bacterial pathogens may be classified as either gram-positive or gram-negative pathogens. Antibiotic compounds with activity against both gram-positive and gram-negative pathogens are generally regarded as having a broad spectrum of activity. The compounds of the present invention are regarded as active against gram-positive and/or gram-negative bacterial pathogens, in particular against gram-positive bacterial pathogens. In particular, the present compounds are active against at least one gram-positive bacterium, preferably against several gram-positive bacteria, more preferably against one or more gram-positive bacteria and/or one or more gram-negative bacteria.

The present compounds have bactericidal or bacteriostatic activity.

Examples of gram-positive and gram-negative aerobic and anaerobic bacteria, include Staphylococci, for example *S. aureus;* Enterococci, for example *E. faecalis;* Streptococci, for example *S. pneumoniae, S. mutans, S. pyogens;* Bacilli, for example *Bacillus subtilis;* Listeria, for example *Listeria monocytogenes;* Haemophilus, for example *H. influenza;* Moraxella, for example *M. catarrhalis;* Pseudomonas, for example *Pseudomonas aeruginosa;* and Escherichia, for example *E. coli.* Gram-positive pathogens, for example Staphylococci, Enterococci and Streptococci are particularly important because of the development of resistant strains which are both difficult to treat and difficult to eradicate from for example a hospital environment once established. Examples of such strains are methicillin resistant *Staphylococcus aureus* (MRSA), methicillin resistant coagulase negative staphylococci (MRCNS), penicillin resistant *Streptococcus pneumoniae* and multiple resistant *Enterococcus faecium.*

The compounds of the present invention also show activity against resistant bacterial strains.

The compounds of the present invention are especially active against *Streptococcus pneumoniae* and *Staphylococcus aureus*, including resistant *Staphylococcus aureus* such as for example methicillin resistant *Staphylococcus aureus* (MRSA).

Therefore, the present invention also relates to the use of a compound of formula (Ia) or (Ib), the pharmaceutically acceptable salts thereof or the *N*-oxide forms thereof or the solvates thereof, as well as any of the pharmaceutical compositions thereof as described hereinafter for the manufacture of a medicament for the treatment of a bacterial infection including an infection caused by Staphylococci and/or Streptococci.

Accordingly, in another aspect, the invention provides for compounds for use in a method of treating a patient suffering from, or at risk of, a bacterial infection, including an infection caused by Staphylococci and/or Streptococci, which comprises administering to the patient a therapeutically effective amount of a compound or pharmaceutical composition according to the invention.

Without being bound to any theory, it is taught that the activity of the present compounds lies in inhibition of the F1F0 ATP synthase, in particular the inhibition of the F0 complex of the F1F0 ATP synthase, more in particular the inhibition of subunit c of the F0 complex of the F1F0 ATP synthase, leading to killing of the bacteria by depletion of the cellular ATP levels of the bacteria. Therefore, in particular, the compounds of the present invention are active on those bacteria of which the viability depends on proper functioning of F1F0 ATP synthase.

Bacterial infections which may be treated by the present compounds include, for example, central nervous system infections, external ear infections, infections of the middle ear, such as acute otitis media, infections of the cranial sinuses, eye infections, infections of the oral cavity, such as infections of the teeth, gums and mucosa, upper respiratory tract infections, lower respiratory tract infections, genitourinary infections, gastrointestinal infections, gynaecological infections, septicemia, bone and joint infections, skin and skin structure infections, bacterial endocarditis, bums, antibacterial prophylaxis of surgery, and antibacterial prophylaxis in immunosuppressed patients, such as patients receiving cancer chemotherapy, or organ transplant patients.

Whenever used hereinbefore or hereinafter, that the compounds can treat a bacterial infection it is meant that the compounds can treat an infection with one or more bacterial strains.

The invention also relates to a composition comprising a pharmaceutically acceptable carrier and, as active ingredient, a therapeutically effective amount of a compound according to the invention. The compounds according to the invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration orally or by parenteral injection. For example, in preparing the compositions in oral dosage form, any of the usual pharmaceutical media may be employed such as, for example, water, glycols, oils, alcohols and the like in the case of oral liquid preparations such as suspensions, syrups, elixirs, emulsions and solutions; or solid carriers such as starches, sugars, kaolin, diluents, lubricants, binders, disintegrating agents and the like in the case of powders, pills, capsules and tablets. Because of their ease in administration, tablets and capsules represent the most advantageous oral dosage unit forms in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight, even more preferably from 0.1 to 50 % by weight of the active ingredient(s), and, from 1 to 99.95 % by weight, more preferably from 30 to 99.9 % by weight, even more preferably from 50 to 99.9 % by weight of a pharmaceutically acceptable carrier, all percentages being based on the total weight of the composition.

The pharmaceutical composition may additionally contain various other ingredients known in the art, for example, a lubricant, stabilising agent, buffering agent, emulsifying agent, viscosity-regulating agent, surfactant, preservative, flavouring or colorant.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, powder packets, wafers, suppositories, injectable solutions or suspensions and the like, and segregated multiples thereof.

The daily dosage of the compound according to the invention will, of course, vary with the compound employed, the mode of administration, the treatment desired and the mycobacterial disease indicated. However, in general, satisfactory results will be obtained when the compound according to the invention is administered at a daily dosage not exceeding 1 gram, e.g. in the range from 10 to 50 mg/kg body weight.

Given the fact that the compounds of formula (Ia) or Formula (Ib) are active against bacterial infections, the present compounds may be combined with other antibacterial agents in order to effectively combat bacterial infections.

Therefore, the present invention also relates to a combination of (a) a compound according to the invention, and (b) one or more other antibacterial agents.

The present invention also relates to a combination of (a) a compound according to the invention, and (b) one or more other antibacterial agents, for use as a medicine.

The present invention also relates to a combination or pharmaceutical composition as defined directly above for use in the treatment of a bacterial infection.

A pharmaceutical composition comprising a pharmaceutically acceptable carrier and, as active ingredient, a therapeutically effective amount of (a) a compound according to the invention, and (b) one or more other antibacterial agents, is also comprised by the present invention.

The weight ratio of (a) the compound according to the invention and (b) the other antibacterial agent(s) when given as a combination may be determined by the person skilled in the art. Said ratio and the exact dosage and frequency of administration depends on the particular compound according to the invention and the other antibacterial agent(s) used, the particular condition being treated, the severity of the condition being treated, the age, weight, gender, diet, time of administration and general physical condition of the particular patient, the mode of administration as well as other medication the individual may be taking, as is well known to those skilled in the art. Furthermore, it is evident that the effective daily amount may be lowered or increased depending on the response of the treated subject and/or depending on the evaluation of the physician prescribing the compounds of the instant invention. A particular weight ratio for the present compound of formula (Ia) or (Ib) and another antibacterial agent may range from 1/10 to 10/1, more in particular from 1/5 to 5/1, even more in particular from 1/3 to 3/1.

The compounds according to the invention and the one or more other antibacterial agents may be combined in a single preparation or they may be formulated in separate preparations so that they can be administered simultaneously, separately or sequentially. Thus, the present invention also relates to a product containing (a) a compound according to the invention, and (b) one or more other antibacterial agents, as a combined preparation for simultaneous, separate or sequential use in the treatment of a bacterial infection.

The other antibacterial agents which may be combined with the compounds of formula (Ia) or (Ib) are for example antibacterial agents known in the art. The other antibacterial agents comprise antibiotics of the β-lactam group such as natural penicillins, semisynthetic penicillins, natural cephalosporins, semisynthetic cephalosporins, cephamycins, 1-oxacephems, clavulanic acids, penems, carbapenems, nocardicins, monobactams; tetracyclines, anhydrotetracyclines, anthracyclines; aminoglycosides; nucleosides such as *N*-nucleosides, C-nucleosides, carbocyclic nucleosides, blasticidin S; macrolides such as 12-membered ring macrolides, 14-membered ring macrolides, 16-membered ring macrolides; ansamycins; peptides such as bleomycins, gramicidins, polymyxins, bacitracins, large ring peptide antibiotics containing lactone linkages, actinomycins, amphomycin, capreomycin, distamycin, enduracidins, mikamycin, neocarzinostatin, stendomycin, viomycin, virginiamycin; cycloheximide; cycloserine; variotin; sarkomycin A; novobiocin; griseofulvin; chloramphenicol; mitomycins; fumagillin; monensins; pyrrolnitrin; fosfomycin; fusidic acid; D-(*p*-hydroxyphenyl)glycine; D-phenylglycine; enediynes.

Specific antibiotics which may be combined with the present compounds of formula (Ia) or (Ib) are for example benzylpenicillin (potassium, procaine, benzathine), phenoxymethylpenicillin (potassium), phenethicillin potassium, propicillin, carbenicillin (disodium, phenyl sodium, indanyl sodium), sulbenicillin, ticarcillin disodium, methicillin sodium, oxacillin sodium, cloxacillin sodium, dicloxacillin, flucloxacillin, ampicillin, mezlocillin, piperacillin sodium, amoxicillin, ciclacillin, hectacillin, sulbactam sodium, talampicillin hydrochloride, bacampicillin hydrochloride, pivmecillinam, cephalexin, cefaclor, cephaloglycin, cefadroxil, cephradine, cefroxadine, cephapirin sodium, cephalothin sodium, cephacetrile sodium, cefsulodin sodium, cephaloridine, cefatrizine, cefoperazone sodium, cefamandole, vefotiam hydrochloride, cefazolin sodium, ceftizoxime sodium, cefotaxime sodium, cefmenoxime hydrochloride, cefuroxime, ceftriaxone sodium, ceftazidime, cefoxitin, cefmetazole, cefotetan, latamoxef, clavulanic acid, imipenem, aztreonam, tetracycline, chlortetracycline hydrochloride, demethylchlortetracyc line, oxytetracycline, methacycline, doxycycline, rolitetracycline, minocycline, daunorubicin hydrochloride, doxorubicin, aclarubicin, kanamycin sulfate, bekanamycin, tobramycin, gentamycin sulfate, dibekacin, amikacin, micronomicin, ribostamycin, neomycin sulfate, paromomycin sulfate, streptomycin sulfate, dihydrostreptomycin, destomycin A, hygromycin B, apramycin, sisomicin, netilmicin sulfate, spectinomycin hydrochloride, astromicin sulfate, validamycin, kasugamycin, polyoxin, blasticidin S, erythromycin, erythromycin estolate, oleandomycin phosphate, tracetyloleandomycin, kitasamycin, josamycin, spiramycin, tylosin, ivermectin, midecamycin, bleomycin sulfate, peplomycin sulfate, gramicidin S, polymyxin B, bacitracin, colistin sulfate, colistinmethanesulfonate sodium, enramycin, mikamycin, virginiamycin, capreomycin sulfate, viomycin, enviomycin, vancomycin, actinomycin D, neocarzinostatin, bestatin, pepstatin, monensin, lasalocid, salinomycin, amphotericin B, nystatin, natamycin, trichomycin, mithramycin, lincomycin, clindamycin, clindamycin palmitate hydrochloride, flavophospholipol, cycloserine, pecilocin, griseofulvin, chloramphenicol, chloramphenicol palmitate, mitomycin C, pyrrolnitrin, fosfomycin, fusidic acid, bicozamycin, tiamulin, siccanin.

Other Mycobacterial agents which may be combined with the compounds of formula (Ia) or (Ib) are for example rifampicin (=rifampin); isoniazid; pyrazinamide; amikacin; ethionamide; ethambutol; streptomycin; para-aminosalicylic acid; cycloserine; capreomycin; kanamycin; thioacetazone; PA-824; quinolones/fluoroquinolones such as for example moxifloxacin, gatifloxacin, ofloxacin, ciprofloxacin, sparfloxacin; macrolides such as for example clarithromycin, clofazimine, amoxycillin with clavulanic acid; rifamycins; rifabutin; rifapentine; the compounds disclosed in WO2004/011436.

### GENERAL PREPARATION

The compounds according to the invention can generally be prepared by a succession of steps, each of which is known to the skilled person.

Compounds of formula (Ia) or (Ib) wherein R⁵ represents -C(=NH)-NH₂, said compounds being represented by formula (Ia-1) or (Ib-1), can be prepared by reacting an intermediate of formula (II-a) or (II-b) with 1H-pyrazole-1-carboximidamide in the presence of a suitable base, such as for example *N*-ethyl-*N*-(1-methylethyl)-2-propanamine, and a suitable solvent, such as for example *N,N*-dimethylformamide.

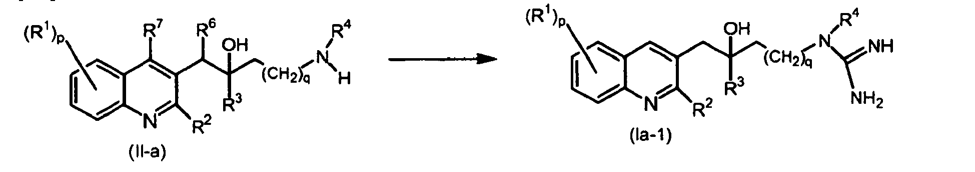

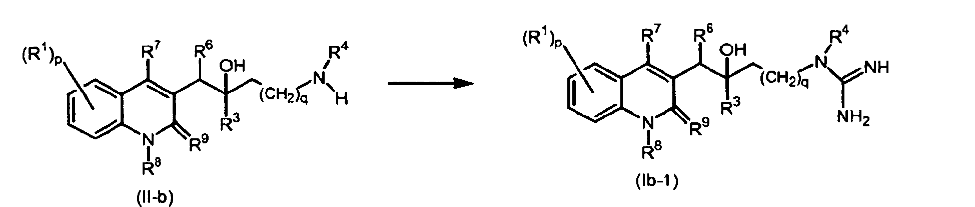

Compounds of formula (I-a) or (Ib) can also be prepared by reacting an intermediate of formula (III-a) or (III-b) with an intermediate of formula (IV) according to the following reaction scheme : 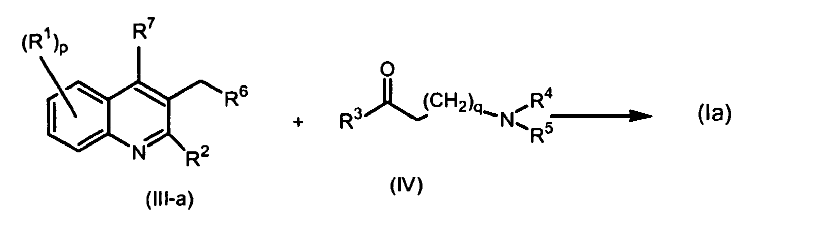 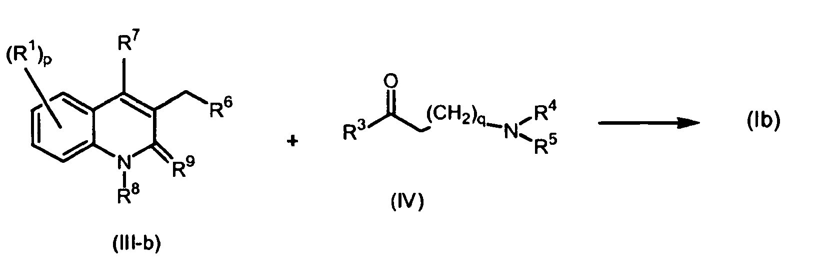 using nBuLi in a mixture of a suitable base, such as for example diisopropyl amine, and a suitable solvent, such as for example tetrahydrofuran, wherein all variables are defined as in formula (Ia) or (Ib). Stirring may enhance the rate of the reaction. The reaction may conveniently be carried out at a temperature ranging between -20 and -70 °C.

Compounds of formula (Ia) or (b) wherein q is equal to 2, 3 or 4, said compounds being represented by formula (Ia-2) or (Ib-2), can also be prepared by reacting an intermediate of formula (V-a) or (V-b) wherein q' is 0, 1 or 2, with a primary or secondary amine HNR⁴R⁵ in the presence of a suitable catalyst, such as for example Rh(cod)₂BF₄, optionally in the presence of a second catalyst (for the reduction), such as for example Ir(cod)₂BF₄, in the presence of a suitable ligand, such as for example Xantphos, in a suitable solvent, such as for example tetrahydrofuran and an alcohol, e.g. methanol, in the presence of CO and H₂ (under pressure) at elevated temperature. This reaction is preferably done for intermediates of formula (V) wherein q' is 1.

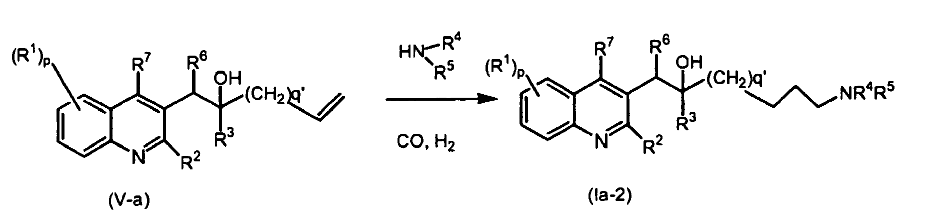

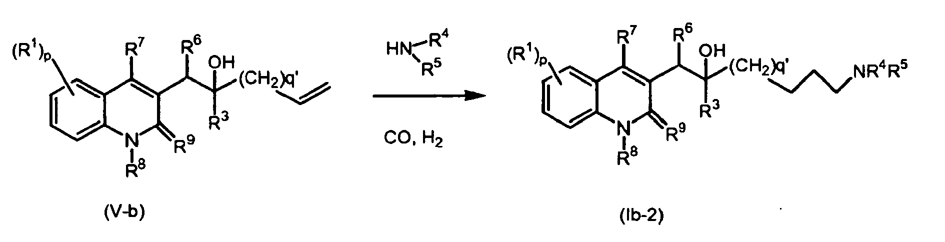

Compounds of formula (Ia) or (Ib) can also be prepared by reacting an intermediate of formula (VI-a) or (VI-b) wherein W₂ represents a suitable leaving group, such as for example halo, e.g. chloro or bromo, with a suitable primary or secondary amine HNR⁴R⁵, optionally in the presence of a suitable solvent, such as for example acetonirile.

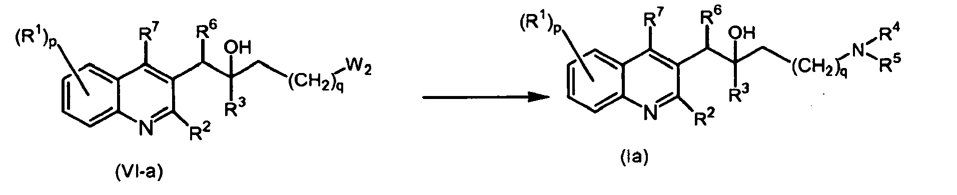

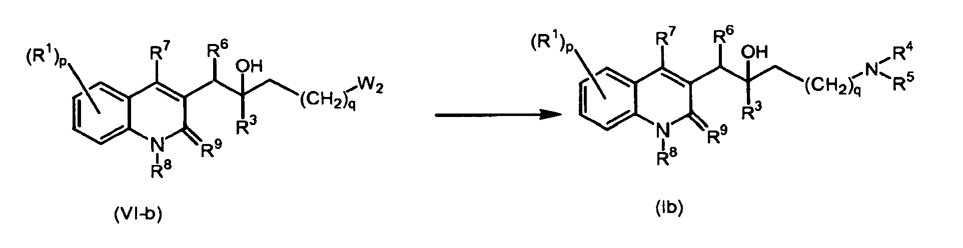

It is considered within the knowledge of the skilled man to explore the appropriate temperatures, dilutions, and reaction times in order to optimize the above reactions in order to obtain a desired compound.

The compounds of formula (Ia) or (Ib) may further be prepared by converting compounds of formula (Ia) or (Ib) into each other according to art-known group transformation reactions.

The compounds of formula (Ia) or (Ib) may be converted to the corresponding *N*-oxide forms following art-known procedures for converting a trivalent nitrogen into its *N*-oxide form. Said *N*-oxidation reaction may generally be carried out by reacting the starting material of formula (Ia) or (Ib) with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (Ia) or (Ib) wherein R¹ represents halo, e.g. bromo, can be converted into a compound of formula (Ia) or (Ib) wherein R¹ represents Het, by reaction with Het-B(OH)₂ in the presence of a suitable catalyst, such as for example Pd(OAc)₂ or Pd(PPh₃)₄, in the presence of a suitable base, such as for example K₃PO₄ or Na₂CO₃, and a suitable solvent, such as for example toluene or 1,2-dimethoxyethane (DME).

Similarly, compounds of formula (Ia) or (Ib) in which R¹ is halo, for example bromo, may be converted into compounds of formula (Ia) or (Ib) in which R¹ is alkyl, for example methyl, by treatment with an appropriate alkylating agent such as CH₃B(OH)₂ or (CH₃)₄Sn in the presence of a suitable catalyst, such as for example Pd(PPh₃)₄, in a suitable solvent such as for example toluene or 1,2-dimethoxyethane (DME).

Compounds of formula (Ia) or (Ib) wherein R¹ is halo, in particular bromo, or arylalkyl, can be converted into a compound of formula (Ia) or (Ib) wherein R¹ is hydrogen, by reaction with HCOONH₄ in the presence of a suitable catalyst such as for example palladium on charcoal, and in the presence of a suitable solvent, such as for example an alcohol, e.g. methanol.

Compounds of formula (Ia) or (Ib) wherein R¹ is halo, in particular bromo, can also be converted into a compound wherein R¹ is formyl, by reaction with *N,N*-dimethylformamide in the presence of nBuLi and a suitable solvent, such as for example tetrahydrofuran. These compounds can then further be converted into a compound of formula (Ia) or (Ib) wherein R¹ is -CH₂-OH by reaction with a suitable reducing agent, such as for example NaBH₄, and in the presence of a suitable solvent, such as for example an alcohol, e.g. methanol, and tetrahydrofuran.

Compounds of formula (Ia) or (Ib) wherein R¹ represents C₂₋₆alkenyl, can be prepared by reacting a compound of formula (Ia) or (Ib) wherein R¹ is halo, e.g. bromo and the like, with tributyl(C₂₋₆alkenyl)tin, such as for example tributyl(vinyl)tin, in the presence of a suitable catalyst, such as for example Pd(PPh₃)₄, in the presence of a suitable solvent, such as for example *N,N*-dimethylformamide. This reaction is preferably performed at elevated temperature.

Compounds of formula (Ia) or (Ib) wherein R¹ represents R^{5a}R^{4a}N-, can be prepared from a compound of formula (Ia) or (Ib) wherein R¹ is halo, e.g. bromo and the like, by reaction with R^{5a}R^{4a}NH in the presence of a suitable catalyst, such as for example tris(dibenzylideneacetone)palladium, a suitable ligand, such as for example 2-(di-t-butylphosphino)biphenyl, a suitable base, such as for example sodium t-butoxide, and a suitable solvent, such as for example toluene.

Compounds of formula (Ia) or (Ib) wherein R¹ represents -C=N-OR¹¹, can be prepared from a compound of formula (Ia) or (Ib) wherein R¹ is formyl, by reaction with hydroxylamine hydrochloride or C₁₋₆alkoxylamine hydrochloride in the presence of a suitable solvent, such as for example pyridine.

Compounds of formula (Ia) or (Ib) wherein R¹ represents -CH₂-NH₂, can be prepared from a compound of formula (Ia) or (Ib) wherein R¹ is formyl, by reduction in the presence of H₂, a suitable catalyst, such as for example palladium on charcoal, and a suitable solvent, such as for example NH₃/alcohol, e.g. NH₃/methanol. Compounds of formula (Ia) or (Ib) wherein R¹ represents -CH₂-NH₂ can be converted into a compound of formula (Ia) or (Ib) wherein R¹ represents -CH₂-N(C₁₋₆alkyl)₂ by reaction with a suitable aldehyde or ketone reagent, such as for example paraformaldehyde or formaldehyde, in the presence of sodium cyanoborohydride, acetic acid and a suitable solvent, such as for example acetonitrile.

Compounds of formula (Ia) or (Ib) wherein R¹ represents R^{5a}R^{4a}N-CH₂-, can be prepared by reacting a compound of formula (Ia) or (Ib) wherein R¹ is formyl, with a suitable reagent of formula R^{5a}R^{4a}N-H in the presence of a suitable reducing agent, such as for example BH₃CN, a suitable solvent, such as for example acetonitrile and tetrahydrofuran, and a suitable acid, such as for example acetic acid.

Compounds of formula (Ia) or (Ib) wherein R¹ represents amino, can be prepared by reacting a compound of formula (Ia) or (Ib) wherein R¹ is carboxyl, with a suitable azide, such as for example diphenylphosphorylazide (DPPA), and a suitable base, such as for example triethylamine, in a suitable solvent, such as for example toluene. The obtained product undergoes a Curtius reaction, and by adding trimethylsilylethanol a carbamate intermediate is formed. In a next step, this intermediate is reacted with tetrabutylammonium bromide (TBAB) in a suitable solvent, such as for example tetrahydrofuran to obtain the amino derivative.

Compounds of formula (Ia) or (Ib) wherein R¹ represents aminocarbonyl, mono or di(alkyl)aminocarbonyl or R^{5a}R^{4a}N-C(=O)-, can be prepared by reacting a compound of formula (Ia) or (Ib) wherein R¹ is carboxyl, with a suitable amine, a suitable coupling reagent such as for example hydroxybenzotriazole, a suitable activating reagent such as for example 1,1'-carbonyldiimidazole or *N,N'*-dicyclohexylcarbodiimide or 1-(3-dimethylaminopropyl)-3-ethylcarbodiimide, a suitable base, such as for example triethylamine, and a suitable solvent, such as for example tetrahydrofuran and methylenechloride.

Compounds of formula (Ia) or (Ib) wherein R¹ represents arylcarbonyl, can be prepared by reacting in a first step (a) a compound of formula (Ia) or (Ib) wherein R¹ is halo, e.g. bromo and the like, with a suitable arylaldehyde in the presence of nBuLi and a suitable solvent, such as for example tetrahydrofuran. This reaction is preferably performed at low temperature such as for example -70°C. In a next step (b), the product obtained in step (a) is oxidized with a suitable oxidans, such as for example manganese oxide, in the presence of a suitable solvent, such as for example methylene chloride.

Compounds of formula (Ia) or (Ib) wherein R⁴ and R⁵ represent a ring moiety substituted with alkylcarbonyl, can be prepared from the corresponding compound wherein the ring moiety is unsubstituted by reaction with an appropriate acyl chloride, e.g. acetyl chloride, in the presence of a suitable base, such as for example triethylamine, and a suitable solvent, such as for example methylene chloride.

Compounds of formula (Ia) or (Ib) wherein R⁴ and R⁵ represent an unsubstituted ring moiety, can be prepared from the corresponding compound wherein the ring moiety is substituted with arylalkyl, by reaction with ammonium formate in the presence of a suitable catalyst, such as for example palladium on charcoal, and a suitable solvent, such as for example an alcohol, e.g. methanol.

Compounds of formula (Ia) or (Ib) wherein R⁶ represents phenyl substituted with halo, can be converted into a compound of formula (Ia) or (Ib) wherein R⁶ represents phenyl substituted with Het, by reaction with Het-B(OH)₂ in the presence of a suitable catalyst, such as for example Pd(PPh₃)₄, in the presence of a suitable base, such as for example Na₂CO₃, and a suitable solvent, such as for example toluene or 1,2-dimethoxyethane (DME) and an alcohol, for example methanol.

A compound of formula (Ia) wherein R² represents methoxy, can be converted into the corresponding compound of fomula (Ib) wherein R⁸ is hydrogen and R⁹ is oxo, by hydrolysis in the presence of a suitable acid, such as for example hydrochloric acid, and a suitable solvent, such as for example dioxane.

Compounds of formula (Ia) or (Ib) wherein R⁴ and R⁵ are taken together with the nitrogen to which they are attached to form 1,1-dioxide-thiomorpholinyl, can be prepared from the corresponding thiomorpholine derivative by reaction with an appropriate organic or inorganic peroxide. Appropriate inorganic peroxides comprise, for example, hydrogen peroxide, alkali metal or earth alkaline metal peroxides, e.g. sodium peroxide, potassium peroxide; appropriate organic peroxides may comprise peroxy acids such as, for example, benzenecarboperoxoic acid or halo substituted benzenecarboperoxoic acid, e.g. 3-chlorobenzenecarboperoxoic acid, peroxoalkanoic acids, e.g. peroxoacetic acid, alkylhydroperoxides, e.g. tert.butyl hydro-peroxide. Suitable solvents are, for example, water, lower alcohols, e.g. ethanol and the like, hydrocarbons, e.g. toluene, ketones, e.g. 2-butanone, halogenated hydrocarbons, e.g. dichloromethane, and mixtures of such solvents.

Compounds of formula (Ia) or (Ib) can also be converted into a quaternary amine by reaction with a suitable quaternizing agent, such as, for example, an optionally substituted C₁₋₆alkylhalide, arylC₁₋₆alkylhalide, C₁₋₆alkylcarbonylhalide, arylcarbonylhalide, Het¹C₁₋₆alkylhalide or Het¹carbonylhalide, e.g. methyliodide or benzyliodide, in the presence of a suitable solvent, such as for example acetone wherein Het¹ represents furanyl or thienyl; or a bicyclic heterocycle selected from benzofuranyl or benzothienyl; each monocyclic and bicyclic heterocycle may optionally be substituted with 1, 2 or 3 substituents, each substituent independently selected from the group of halo, C₁₋₆alkyl and aryl. Said quaternary amines are represented by the below formula wherein R¹⁰ represents C₁₋₆alkyl, C₁₋₆alkylcarbonyl, arylC₁₋₆alkyl, arylcarbonyl, Het¹C₁₋₆alkyl or Het¹carbonyl and wherein A⁻ represents a pharmaceutically acceptable counter ion, such as for example iodide.

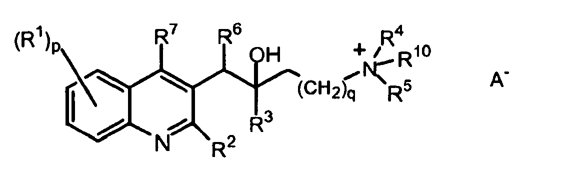

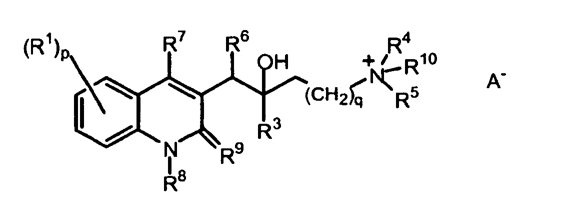

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC, chiral chromatography. Individual diastereoisomers or individual enantiomers can also be obtained by Supercritical Fluid Chromatography (SCF).

The starting materials and the intermediates are compounds that are either commercially available or may be prepared according to conventional reaction procedures generally known in the art. For example, the intermediates of formula (II-a) or (II b) or (III-a) or (III-b) can be prepared according to the methods described in WO 2004/011436, WO2005/070924, W02005/070430 or WO2005/075428.

In particular, the intermediates of formula (II-a) and (II-b) can be prepared by reacting an intermediate of formula (III-a) or (III-b) with an intermediate of formula (VIII) according to the following reaction scheme (1) : 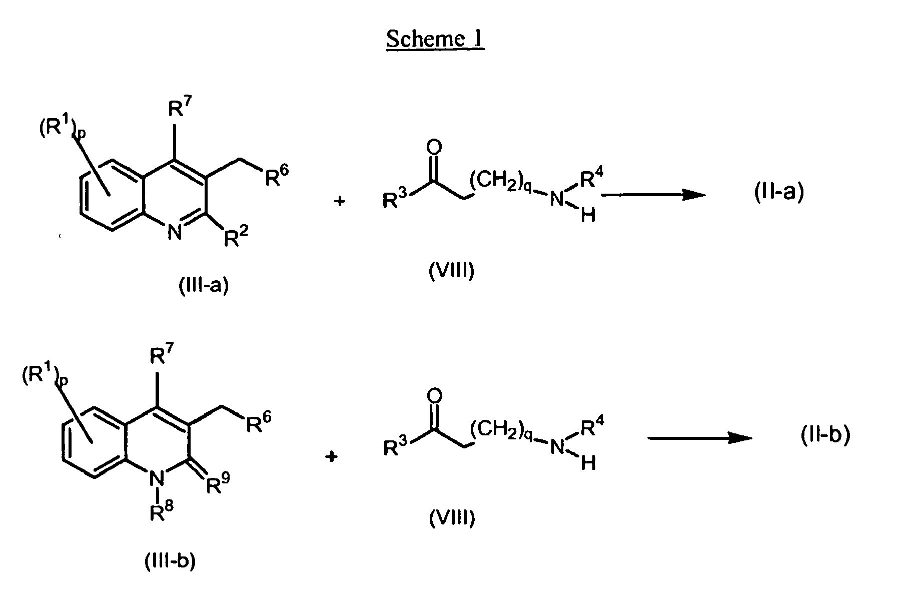 using nBuLi in a mixture of diisopropyl amine and tetrahydrofuran, wherein all variables are defined as in formula (Ia) or (Ib). Stirring may enhance the rate of the reaction. The reaction may conveniently be carried out at a temperature ranging between -20 and -70 °C.

Intermediates of formula (II-a) or (II-b) can also be prepared from the corresponding intermediates wherein R⁵ is benzyl by reaction with carbonochloridic acid, 1-chloroethyl ester in the presence of a suitable solvent, such as for example dichloro ethane.

Intermediates of formula (III-a) may be prepared according to the following reaction scheme (2): 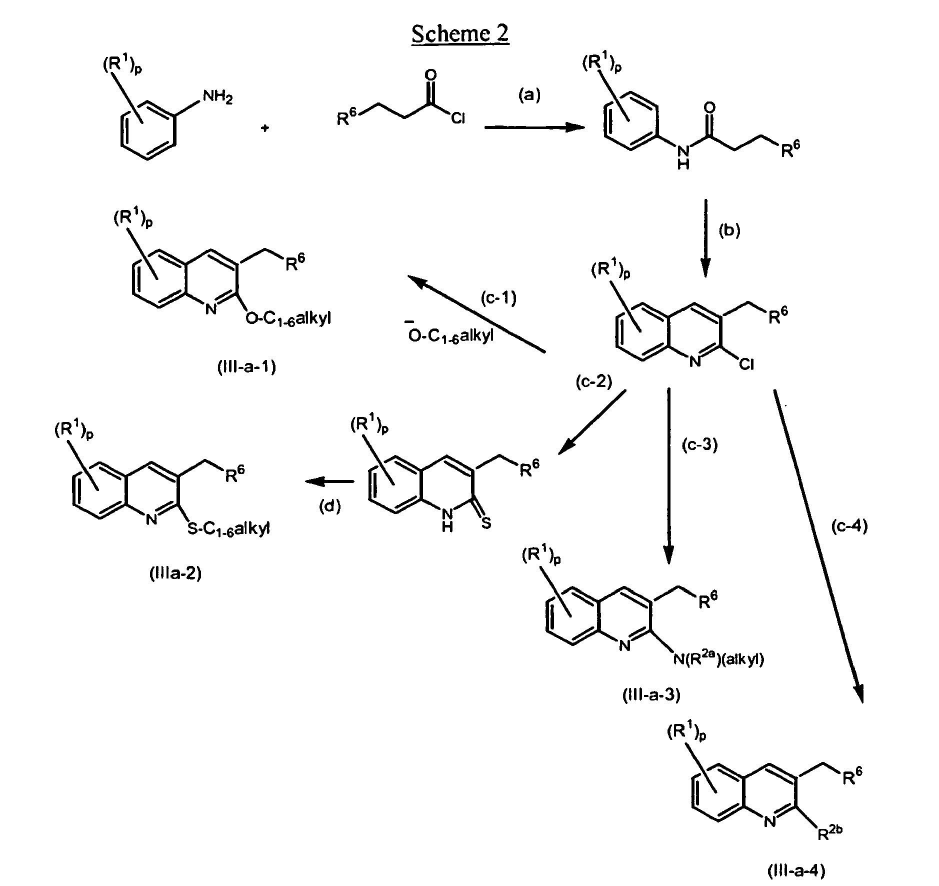 wherein all variables are defined as in formula (Ia). Reaction scheme (2) comprises step (a) in which an appropriately substituted aniline is reacted with an appropriate acylchloride such as for example 3-phenylpropionyl chloride, 3-fluorobenzenepropionyl chloride or *p*-chlorobenzenepropionyl chloride, in the presence of a suitable base, such as triethylamine, and a suitable reaction-inert solvent, such as methylene chloride or ethylene dichloride. The reaction may conveniently be carried out at a temperature ranging between room temperature and reflux temperature. In a next step (b) the adduct obtained in step (a) is reacted with phosphoryl chloride (POCl₃) in the presence of *N,N*-dimethylformamide (Vilsmeier-Haack formylation followed by cyclization). The reaction may conveniently be carried out at a temperature ranging between room temperature and reflux temperature. In a next step (c-1), a specific R²-group, wherein R² is for example a C₁₋₆alkyloxy radical is introduced by reacting the intermediate compound obtained in step (b) with -O-C₁₋₆alkyl in the presence of a suitable solvent, such as for example HO-C₁₋₆alkyl. The intermediate obtained in step (b) can also be converted into an intermediate wherein R² is for example a C₁₋₆alkylthio radical by reaction with S=C(NH₂)₂ in the presence of a suitable solvent, such as for example an alcohol, e.g. ethanol, or an alcohol/water mixture, optionally in the presence of a suitable base, such as for example KOH, (see step (c-2)) followed by reaction with C₁₋₆alkyl-I in the presence of a suitable base, such as for example K₂CO₃, and a suitable solvent, such as for example 2-propanone (see step (d)). The intermediate obtained in step (b) can also be converted into an intermediate wherein R² is -N(R^{2a})(alkyl) wherein R^{2a} is hydrogen or alkyl, by reaction with a suitable salt of NH(R^{2a})(alkyl) in the presence of a suitable base, such as for example potassium carbonate, and a suitable solvent, such as for example acetonitrile (step (c-3)). The intermediate obtained in step (b) can also be converted into an intermediate wherein R² is C₁₋₆alkyloxyC₁₋₆alkyloxy optionally substituted with C₁₋₆alkyloxy, said R² being represented by R^{2b}, by reaction with C₁₋₆alkyloxyC₁₋₆yalkylOH optionally substituted with C₁₋₆alkyloxy, in the presence of NaH and a suitable solvent, such as for example tetrahydrofuran (step (c-4)).

Intermediates of formula (III-a) wherein R² and R⁷ represent hydrogen , said intermediates being represented by formula (III-a-5), may be prepared according to the following reaction scheme (3), wherein in a first step (a) a substituted indole-2,3-dione is reacted with an optionally substituted 3-phenylpropionaldehyde in the presence of a suitable base such as sodium hydroxide (Pfitzinger reaction), after which the carboxylic acid compound is decarboxylated in a next step (b) at high temperature in the presence of a suitable reaction-inert solvent such as diphenylether.

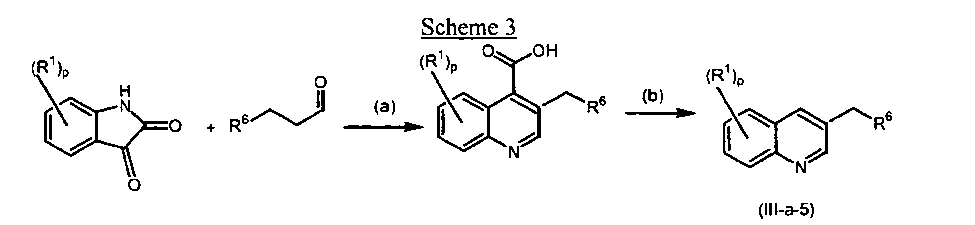

Intermediates of formula (III-a) wherein R⁶ represents Het, said intermediates being represented by formula (III-a-6), can be prepared according to the following reaction scheme 3a.

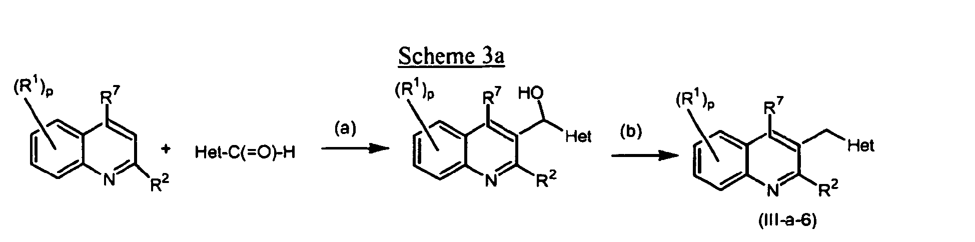

Reaction scheme (3a) comprises step (a) in which an appropriate quinoline moiety is reacted with Het-C(=O)-H using nBuLi in a mixture of a suitable base, such as for example 2,2,6,6-tetramethylpiperidine, and a suitable solvent, such as for example tetrahydrofuran. Stirring may enhance the rate of the reaction. The reaction may conveniently be carried out at a temperature ranging between -20 and -70 °C. In a next step (b), the product obtained in step (a) is converted in aan intermediate of formula (III-a-6) by reaction with a suitable acid, such as for example trifluoroacetic acid, and triisopropylsilane, in the presence of a suitable solvent, such as for example methylene chloride.

Intermediates of formula (III-b), in particular (III-b-1) or (III-b-2), can be prepared according to the following reaction scheme (4).

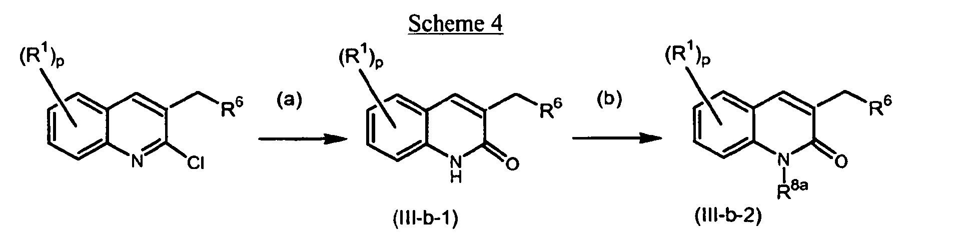

Reaction scheme (4) comprises step (a) in which the quinoline moiety is converted in the quinolinone moiety by reaction with a suitable acid, such as for example hydrochloric acid. In a next step (b), a R^{8a} substituent representing alkyl, is introduced by reacting the intermediate obtained in step (a) with a suitable alkylating agent, such as for example alkyliodide, e.g. methyliodide, in the presence of a suitable base, such as for example NaOH or benzyltriethylammonium chloride, a suitable solvent, such as for example tetrahydrofuran.

Intermediates of formula (III-b) wherein the R⁸ and R⁹ are taken together to form the radical -CH=CH-N=, said intermediates being represented by formula (III-b-3), can be prepared according to the following reaction scheme (5).

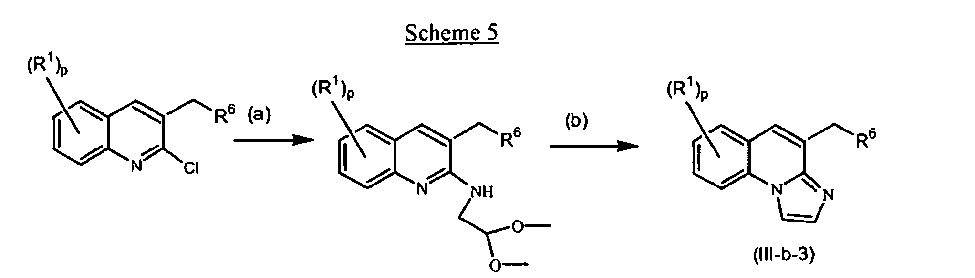

Reaction scheme (5) comprises step (a) in which the intermediate is reacted with NH₂-CH₂-CH(OCH₃)₂. In a next step (b), the fused imidazolyl moiety is formed by reaction with acetic acid in the presence of a suitable solvent, such as for example xylene.

The intermediates of formula (IV) are compounds that are either commercially available or may be prepared according to conventional reaction procedures generally known in the art. For example, intermediates of formula (IV) may be prepared according to the following reaction scheme (6):

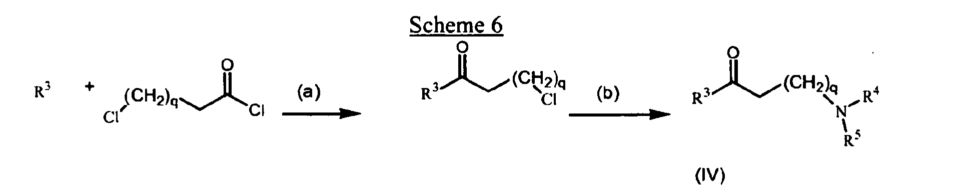

Reaction scheme (6) comprises step (a) in which R³, in particular an appropriately substituted aryl, more in particular an appropriately substituted phenyl, is reacted by Friedel-Craft reaction with an appropriate acylchloride such as 3-chloropropionyl chloride or 4-chlorobutyryl chloride, in the presence of a suitable Lewis acid, such as for example AlCl₃, FeCl₃, SnCl₄, TiCl₄ or ZnCl₂ and a suitable reaction-inert solvent, such as methylene chloride or ethylene dichloride. The reaction may conveniently be carried out at a temperature ranging between room temperature and reflux temperature. In a next step (b), an amino group (-NR⁴R⁵) is introduced by reacting the intermediate obtained in step (a) with a primary or secondary amine (HNR⁴R⁵) in the presence of a suitable solvent, such as for example acetonitrile, and a suitable base, such as for example K₂CO₃. Depending on the amine which is used in step (b), it may be appropriate to first react the intermediate obtained in step (a) with a protected form of the amine, such as for example 2,5-diazabicyclo[2.2.1]heptane-2-carboxylic acid, 1,1-dimethylethyl ester(1S,4S), followed by deprotecting the resulting product in the presence of a suitable acid, such as for example trifluoroacetic acid, and a suitable solvent, such as for example CH₂Cl₂. It is considered to be within the knowledge of the skilled person to recognize when the amine needs to be protected and to recognize the most appropriate protective group for a particular amine.

The intermediates of formula (IV) may also be prepared according to the following reaction Scheme (6a) :

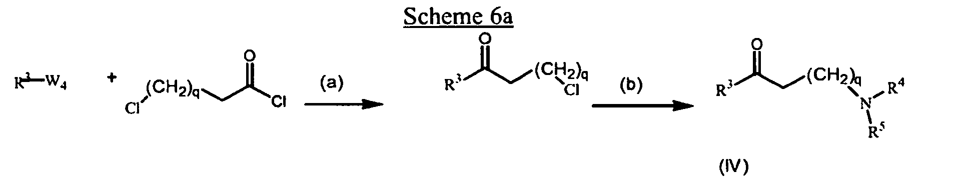

Reaction scheme (6a) comprises step (a) in which R³-W₄, wherein W₄ represents a suitable leaving group, such as for example halo, e.g. chloro or bromo, in particular an appropriately substituted aryl, more in particular an appropriately substituted naphthyl, e.g. 2-bromo-naphthalene, is reacted with an appropriate acylchloride such as 3-chloropropionyl chloride or 4-chlorobutyryl chloride or 5-bromo-pentanoyl chloride, in the presence of Mg, I₂ and a suitable solvent, such as for example tetrahydrofuran. The reaction may conveniently be carried out at a temperature ranging between room temperature and reflux temperature. In a next step (b), an amino group (-NR⁴R⁵) is introduced by reacting the intermediate obtained in step (a) with a primary or secondary amine (HNR⁴R⁵) in the presence of a suitable solvent, such as for example acetonitrile, and a suitable base, such as for example K₂CO₃. Depending on the amine which is used in step (b), it may be appropriate to first react the intermediate obtained in step (a) with a protected form of the amine, such as for example 2,5-diazabicyclo[2.2.1]heptane-2-carboxylic acid, 1,1-dimethylethyl ester(1S,4S), followed by deprotecting the resulting product in the presence of a suitable acid, such as for example trifluoroacetic acid, and a suitable solvent, such as for example CH₂Cl₂. It is considered to be within the knowledge of the skilled person to recognize when the amine needs to be protected and to recognize the most appropriate protective group for a particular amine.

The intermediates of formula (IV) may also be prepared according to the following reaction Scheme (7) :

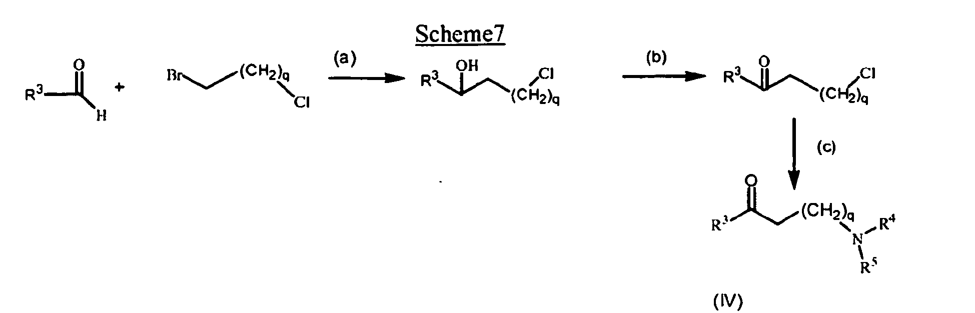

Reaction scheme (7) comprises step (a) in which R³-C(=O)-H, for instance an appropriately substituted arylcarboxaldehyde, more in particular an appropriately substituted phenyl or naphthylcarboxaldehyde, is reacted with an appropriate intermediate compound such as for example 1-bromo-4-chlorobutane, in the presence of Grignard reagent and a suitable solvent, such as for example diethyl ether, tetrahydrofuran. The reaction may conveniently be carried out at a low temperature for instance 5°C. In a next step (b), an oxidation is performed in the presence of Jones'reagent in a suitable solvent, such as for example acetone. In a next step (c), an amino group (-NR₄R₅) is introduced by reacting the intermediate compound obtained in step (b) with a primary or secondary amine HNR₄R₅ in the presence of a suitable solvent, such as for example acetonitrile, and a suitable base, such as for example K₂CO₃.

Alternatively, intermediates of formula (IV) may be prepared according to the following reaction scheme (8):

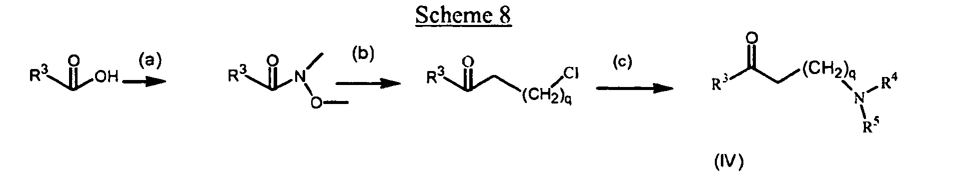

Reaction scheme (8) comprises step (a) in which for instance a suitable acid is reacted with NH(CH₃)(OCH₃) in the presence of 1,1'-carbonyldiimidazole and a suitable solvent, such as for example CH₂Cl₂. In a next step (b), the product obtained in step (a) is reacted with a suitable Grignard reagens, e.g. 4-chlorobutyl magnesium bromide, in the presence of a suitable solvent, such as for example tetrahydrofuran. In a next step (c), an amino group (-NR₄R₅) is introduced by reacting the intermediate obtained in step (b) with a primary or secondary amine HNR₄R₅ in the presence of a suitable solvent, such as for example acetonitrile, and a suitable base, such as for example K₂CO₃.

Alternatively, intermediates of formula (IV) wherein q is 1, said intermediates being represented by formula (IV-a), may be prepared according to the following reaction scheme (9):

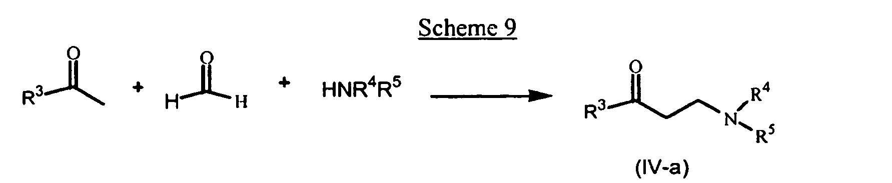

Reaction scheme (9) comprises the step in which a suitable acetyl derivative of R³ such as for example acetylcyclohexane, is reacted with paraformaldehyde and a suitable primary or secondary amine HNR⁴R⁵, preferably in its salt form, in the presence of a suitable acid, such as for example hydrochloric acid and the like, and a suitable solvent, such as for example an alcohol, e.g. ethanol.

Intermediates of formula (IV) wherein R³ represents R^{3a'}-CH₂-CH₂- (which is possible for those intermediates of formula (VI) wherein R³ represents alkyl, arylalkyl, aryl-O-alkyl, aryl-alkyl-O-alkyl, Het-alkyl, Het-O-alkyl or Het-alkyl-O-alkyl and R^{3a'} is the same as R³ but with 2 carbon atoms less in the alkyl chain attached to the remainder of the molecule and wherein q represents 1, said intermediates being represented by formula (IV-b), can be prepared according to the following reaction scheme (10):

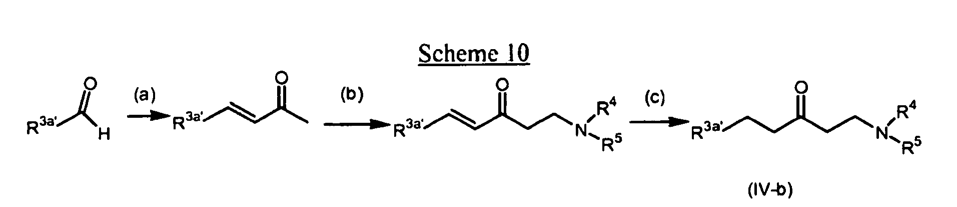

Reaction scheme (10) comprises step (a) wherein a suitable aldehyde is reacted with acetone in the presence of a suitable base, such as for example sodium hydroxide. In a next step (b), the product obtained in step (a) is reacted with a primary or secondary amine HNR⁴R⁵ in the presence of CH₂(=O), a suitable acid, such as for example hydrochloric acid and the like, and a suitable solvent, such as for example an alcohol, e.g. ethanol. In a next step (c), the product obtained in step (b) is hydrogenated (H₂) in the presence of a suitable catalyst, such as for example palladium on charcoal, and a suitable solvent, such as for example water and an alcohol, e.g. ethanol.

Intermediates of formula (IV) wherein R³ represents a halo substituted phenyl, may be converted into an intermediate of formula (IV) wherein R³ represents phenyl substituted with aryl, by reaction with arylboronic acid in the presence of a suitable base, such as for example potassium phosphate, a suitable catalyst, such as for example palladium acetate, and a suitable ligand, such as for example 2-dicyclohexylphosphino-2',6'-dimethoxybiphenyl, in an appropriate solvent, such as for example toluene.

Intermediates of formula (IV) wherein R³ represents a halo substituted phenyl, may also be converted into an intermediate of formula (IV) wherein R³ represents phenyl substituted with C₂₋₆alkenyl optionally substituted with phenyl, by reaction with an appropriate C₂₋₆alkene, such as for example styrene, in the presence of a suitable base, such as for example triethylamine, a suitable catalyst, such as for example palladium acetate, and a suitable ligand, such as for example tri-o-tolylphosphine, in an appropriate solvent, such as for example DMF.

In case in the above reaction schemes, the suitable amine HNR⁴R⁵ represents substituted 2,5-diazabicyclo[2.2.1]heptyl, said amine can be prepared according to the following reaction scheme (11):

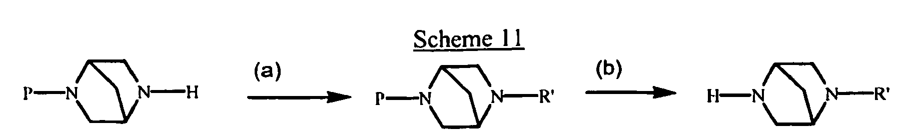

Reaction scheme (11) comprises the step of reacting an appropriately protected 2,5-diazabicyclo[2.2.1]heptyl wherein P represents for instance tert-butyloxycarbonyl, with an appropriate reagens of formula W-R' wherein W represents a suitable leaving group, such as for example halo, e.g. bromo and the like, and wherein R' represents the substituent to be introduced, in the presence of a suitable base, such as for example K₂CO₃, NaHCO₃ or triethylamine, a suitable phase transfer reagent, such as for example tetra-n-butylammonium chloride, a suitable solvent, such as for example acetonitrile, and optionally KI to increase the speed of the reaction. In a next step (b), the protective group is removed by reaction with a suitable acid, such as for example trifluoroacetic acid, in the presence of a suitable solvent, such as for example methylene chloride.

Intermediates of formula (V-a) may be prepared according to the following reaction scheme (12):

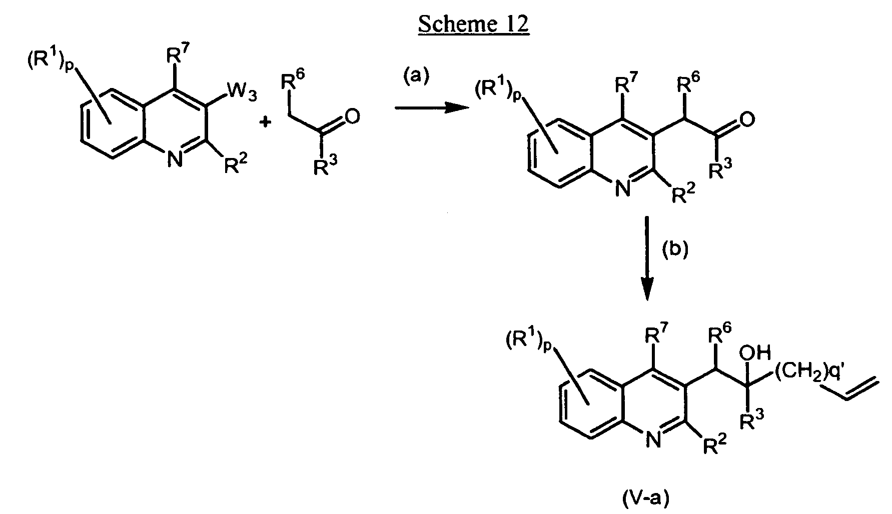

Reaction scheme (12) comprises the step of reacting an appropriately substituted quinoline wherein W₃ represents a suitable leaving group, such as for example halo, e.g. bromo, with an appropriately substituted deoxybenzoin in the presence of a suitable catalyst, such as for example palladium diacetate, a suitable ligand, such as for example X-PHOS, a suitable base, such as for example cesium carbonate, a suitable solvent, such as for example xylene, under N₂ flow. In a next step (b), the product obtained in step (a) is reacted with a suitable Grignard reagens (e.g. CH₂=CH-(CH₂)_{q}-Mg-Br, such as for example allylmagnesium bromide, in a suitable solvent, such as for example tetrahydrofuran.
Intermediates of formula (V-b) can be prepared accordingly.

Intermediates of formula (VI-a) can be prepared according to the following reaction scheme (13):

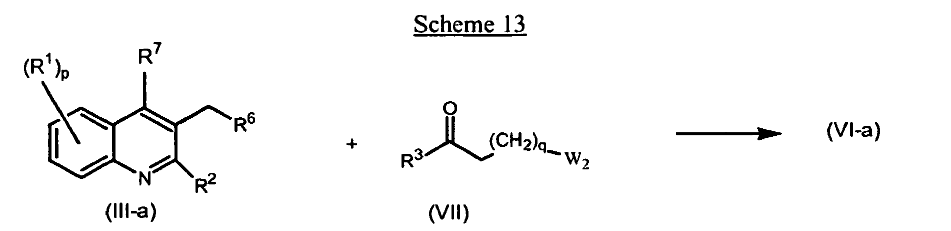

In reaction scheme (13), an intermediate of formula (III-a) is reacted with an intermediate of formula (VII), for its synthesis reference is made to schemes 6, 7 and 8, in the presence of n-BuLi in a suitable solvent, such as for example tetrahydrofuran, and a suitable base, such as for example diisopropyl amine. Stirring may enhance the rate of the reaction. The reaction may conveniently be carried out at a temperature ranging between -20 and -70°C.
Intermediates of formula (VI-b) can be prepared accordingly.

The following examples illustrate the present invention without being limited thereto.

### EXPERIMENTAL PART

Of some compounds or intermediates the absolute stereochemical configuration of the stereogenic carbon atom(s) therein or the configuration at the double bond was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, NMR. It is considered to be within the knowledge of the skilled person to recognize the most appropriate method to determine the actual stereochemical configuration.

In case "A" and "B" are stereoisomeric mixtures, in particular mixtures of enantiomers, they can be further separated whereby the respective first fractions isolated are designated "A1" respectively "B1" and the second as "A2" respectively "B2", without further reference to the actual stereochemical configuration. However, said "A1", "A2" and "B1", "B2" isomeric forms, in particular said "A1", "A2" and "B1", "B2"enantiomeric forms, can be unambiguously characterized by a person skilled in the art, using art-known methods such as, for example, X-ray diffraction.

If a mixture of 4 enantiomers is directly separated into 4 separate enantiomers (without an intermediate step of separating first into two diastereoisomers) the first obtained enantiomer is indicated as "A", the seond enantiomer as "B", the third enantiomer as "C", and the fourth enantiomer as "D".

In some cases, when a final compound or an intermediate, indicated as a particular diastereoisomer or enantiomer, is converted into another final compound/intermediate, the latter may inherit the indication for diastereoisomer (A, B) or enantiomer (A1, A2, B1, B2) from the former.

Hereinafter "THF" means tetrahydrofuran, "DCE" means dichloroethane, "DIPEA" means *N*-ethyl-*N*-(1-methylethyl)-2-propanamine, "DIPE" means diisopropyl ether, "DCM" means dichloromethane, "DMF" means *N,N*-dimethylformamide, and "SFC" means Supercritical Fluid Chromatography.

### A. Preparation of the intermediate compounds

### Example A1

### a-1. Preparation of intermediate 1

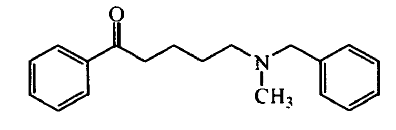

5-Chloro-1-phenyl-1-pentanone (1.50 g, 0.00762 mol), *N*-methylbenzenemethanamine (1.96 ml, 0.015 mol; [103-67-3]) and K₂CO₃ (3.16 g, 0.023 mol) were mixed in a flask. CH₃CN (22.86 ml) was added and the reaction mixture was heated at 80 °C for 48 hours. Then K₂CO₃ was removed by filtration. The product was purified by flash chromatography (eluent: n-hexane/EtOAc 5/1). The product fractions were collected and the solvent was evaporated. Yield: 1.79 g of **intermediate 1** (83 %; yellow oil).

### a-2. Preparation of intermediate 9

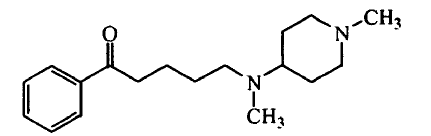

5-Chloro-1-phenyl-1-pentanone (1.02 g, 0.0052 mol, [942-93-8]), 1-methyl-4-(*N-*methylamino)piperidine (1.33 g, 0.01 mol, [73579-08-5]) and K₂CO₃ (2.15 g, 0.015 mol) were mixed in CH₃CN (15 ml) and heated to 80 °C for 48 hours. Then K₂CO₃ was removed by filtration and the crude product was purified by flash chromatography (eluent: CH₂Cl₂/MeOH 10:1). The desired fractions were collected and the solvent was evaporated. Yield: 0.48 g of **intermediate 9** (32 %).

### a-3. Preparation of intermediate 10

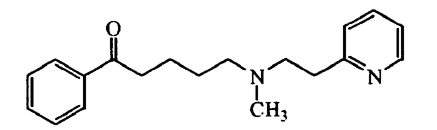

5-Chloro-1-phenyl-1-pentanone (1.5 g, 0.00762 mol), *N*-methyl-2-pyridineethanamine, dihydrochloride (3.19 g, 0.015 mol) and K₂CO₃ (3.16 g, 0.023 mol) were mixed in CH₃CN (22.88 ml) and the reaction mixture was refluxed over the weekend at 80 °C. Then K₂CO₃ was removed by filtration and the product was purified by flash chromatography (eluent: n-hexane/EtOAc; started 5/1, product at 1/1). Yield: 1.77 g of **intermediate 10** (78 %).

### b. Preparation of intermediate 2 and 3

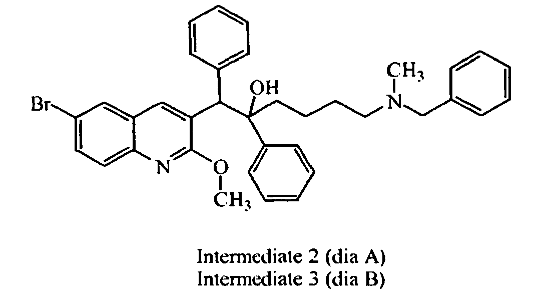

Lithium diisopropylamine ([4111-54-0]) (3.81 ml of a 2.0 M solution in THF/heptanes; 0.00763 mol) was dissolved in THF (25.44 ml; dry) and cooled on an ice-bath at -70 °C. 6-Bromo-2-methoxy-3-(phenylmethyl)-quinoline (intermediate compound 3 (Ex. A3) of W02004/011436) (2.09 g, 0.00636 mol) was added dropwise as a solution in THF (19.08 ml; dry) and the mixture was stirred for 2 hours at -70 °C. Then **intermediate 1** (1.79 g, 0.00636 mol) was added dropwise as a solution in THF (19.08 ml; dry) and the reaction mixture was stirred for 3 hours at -70 °C. Then H₂O was added (quenching) at -70 °C, followed by EtOAc. The layers were separated and the organic layer was washed with brine, dried (MgSO₄), filtered and the solvent was evaporated to give a yellow oil. The residue was purified by flash chromatography (eluent: n-hexane/EtOAc). The desired fractions were collected and the solvent was evaporated. Yield: 0.381 g of **intermediate 2** (dia A) and 0.166 g **intermediate 3** (dia B).

### c. Preparation of intermediate 4

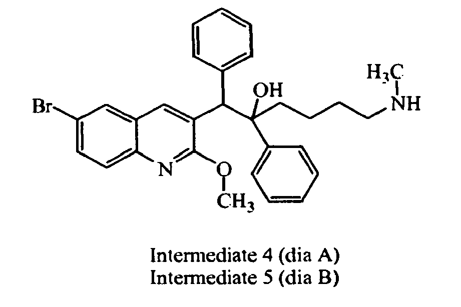

Carbonochloridic acid, 1-chloroethyl ester (0.001 mol) was added to a solution of **intermediate 2** (0.0009 mol) in DCE (10 ml). The mixture was stirred at 80 °C for 1 hour and then the solvent was evaporated till dryness. The residue was taken up in CH₃OH (10 ml). The mixture was stirred at 80 °C for 1 hour and then the solvent was evaporated. The residue (0.7 g) was purified by column chromatography over Kromasil (eluent: CH₂Cl₂/CH₃OH/NH₄OH 95/5/0.5 to 85/15/1.5; 5 µm). The pure fractions were collected and the solvent was evaporated. Yield: 0.21 g of **intermediate 4** (45 %) (m.p.: 121 °C).

**Intermediate 5** was prepared according to the same protocol as intermediate 4, but starting from **intermediate 3**. Yield: 0.31 g **intermediate 5** (52 %) (m.p.: 203 °C).

### Example A2

### a. Preparation of intermediate 6

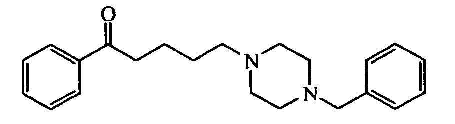

A mixture of 5-chloro-1-phenyl-1-pentanone (0.0102 mol), 1-(phenylmethyl)piperazine (0.0122 mol) and K₂CO₃ (0.0122 mol) in CH₃CN (40 ml) was stirred at 80 °C for 18 hours. Then the mixture was poured out into H₂O, extracted with HCl 1.5 N, basified at 5 °C with NaOH 3 N and extracted with diethyl ether. The organic layer was washed with saturated aqueous NaCl solution, dried (MgSO₄), filtered and the solvent was evaporated. Yield: 2.6 g of **intermediate 6** (78 %).

### b. Preparation of intermediate 7

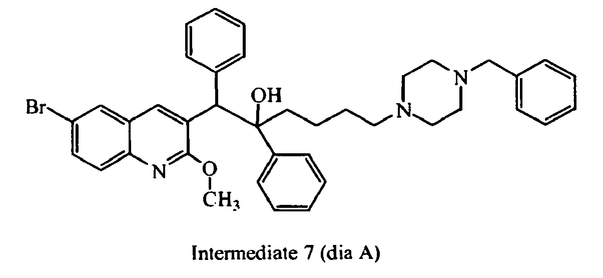

nBuLi (0.0036 mol; 2.3 ml of a 1.6 M solution in hexane) was added dropwise at -20 °C to a solution of diisopropylamine, hydrochloride (0.0036 mol; [819-79-4]) in THF (8 ml) under N₂ flow. The mixture was stirred at -20 °C for 20 minutes and was then cooled to -70 °C. A solution of 6-bromo-2-methoxy-3-(phenylmethyl)-quinoline (intermediate compound 3 (Ex. A3) of WO2004/011436) (0.003 mol) in THF (10 ml) was added. The mixture was stirred at -70 °C for I hour. A solution of **intermediate 6** (0.003 mol) in THF (10 ml) was added. The mixture was stirred at -70 °C for 2 hours. H₂O was added. The mixture was extracted with EtOAc. The organic layer was washed with saturated aqueous NaCl solution, dried (MgSO₄), filtered and the solvent was evaporated. The residue (5.5 g) was purified by column chromatography over silica gel (eluent: CH₂Ch/CH₃OH/NH₄OH 98/2/0.1 then cyclohexane/EtOAc/NH₄OH 65/35/0.2; 15-40 µm). Four fractions were collected. The solvent of the desired product fraction was evaporated. Yield: 0.18 g of intermediate 7 (dia A). (Also the dia B form was isolated in this procedure but was not used further in this context)

### c. Preparation of intermediate 8

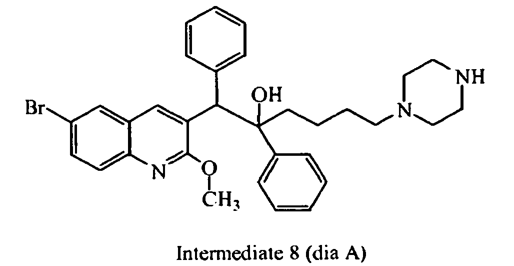

Carbonochloridic acid, 1-chloroethyl ester (0.0004 mol) was added dropwise to a solution of **intermediate 7** (0.0004 mol) in DCE (3 ml). The mixture was stirred at 80 °C for 1 hour and then the solvent was evaporated till dryness. The residue was taken up in CH₃OH. The mixture was stirred at 50 °C for 30 minutes. The solvent was evaporated again and the residue (0.29 g) was purified by column chromatography over Kromasil (eluent: CH₂Cl₂/CH₃OH/NH₄OH 96/4/0.4 to 88/12/1.2 ; 3.5 µm). The pure fractions were collected and the solvent was evaporated. Yield: 0.096 g of **intermediate 8** (36 %; dia A).

### Example A3

### a. Preparation of intermediate 16

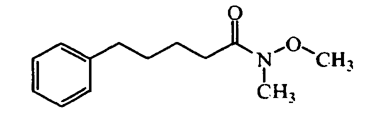

*N,N'*-Carbonyldiimidazole (0.102 mol) was added portionwise at 5 °C to a solution of benzenepentanoic acid (0.068 mol) in DCM (10 ml). The mixture was stirred at 5 °C for 1 hour. *N*-methoxymethanamine hydrochloride (0.102 mol) was added portionwise. The mixture was brought to room temperature, stirred over the weekend, poured out into HCl 1 N and extracted with CH₂Cl₂. The residue was purified by column chromatography over silica gel (eluent: CH₂Cl₂; 15-40 µm). The pure fractions were collected and the solvent was evaporated. Yield: 9.66 g of **intermediate 16** (65 %).

### b-1. Preparation of intermediate 11

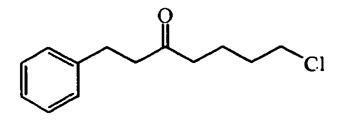

A few drops of 1-bromo-4-chlorobutane were added to a solution of Mg (0.071 mol) in diethyl ether (10 ml) under N₂. The mixture was stirred and refluxed. A solution of 1-bromo-4-chlorobutane (0.071 mol) in diethyl ether (20 ml) was added dropwise. The mixture was stirred for 15 minutes, then cooled to 5 °C. A solution of *N*-methoxy-*N-*methylbenzenepropanamide (0.0595 mol) in THF (35 ml) was added. The mixture was stirred at 5 °C for 2 hours and was then stirred at room temperature for 2 hours. The mixture was poured out into NH₄Cl and the product was extracted with EtOAc. The organic layer was separated, dried (MgSO₄), filtered and the solvent was evaporated. Yield: 13.2 g of **intermediate 11.**

### b-2. Preparation of intermediate 14

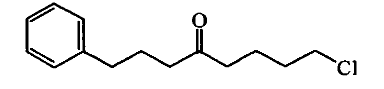

A few drops of 1-bromo-4-chlorobutane were added at room temperature to a solution of Mg (0.0697 mol) in diethyl ether (12 ml). The mixture was stirred for 30 minutes. A solution of 1-bromo-4-chlorobutane (0.0697 mol) in diethyl ether (35 ml) was added. The mixture was stirred and refluxed for 30 minutes, then cooled to 0 °C. A solution of *N*-methoxy-*N*-methylbenzenebutanamide (0.0465 mol; [177756-65-9]) in THF (35 ml) was added dropwise. The mixture was stirred for 15 minutes, then stirred at 50 °C for 4 hours and poured out into NH₄Cl and EtOAc. The organic layer was washed with saturated aqueous NaCl solution, dried (MgSO₄), filtered and the solvent was evaporated. Yield: 11.2 g of **intermediate 14** (100 %).

### b-3. Preparation of intermediate 17

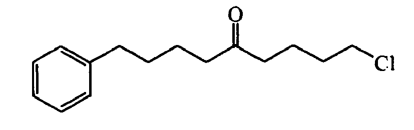

A few drops of 1-bromo-4-chlorobutane were added to a solution of Mg (0.065 mol) in THF (10 ml) under N₂ flow. The mixture was stirred and refluxed. A solution of 1-bromo-4-chlorobutane (0.065 mol) in diethyl ether (15 ml) and THF (15 ml) was added. The mixture was stirred for 30 minutes, then cooled to 5 °C. A solution of **intermediate 16** (0.0437 mol) in THF (30 ml) was added. The mixture was stirred for 30 minutes, then stirred at 55 °C for 3 hours, brought to room temperature, poured out into NH₄Cl and extracted with EtOAc three times. The organic layer was washed with saturated aqueous NaCl solution, dried (MgSO₄), filtered and the solvent was evaporated. Yield: 11 g of **intermediate 17** (100 %).

### c-1. Preparation of intermediate 12

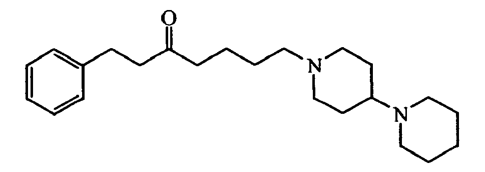

A mixture of **intermediate 11** (0.082 mol), 1,4'-bipiperidine (0.082 mol) and K₂CO₃ (0.09 mol) in CH₃CN (180 ml) was stirred overnight at 80 °C. Then the mixture was brought to room temperature and poured out into H₂O. Diethyl ether was added. The mixture was acidified with HCl 1 N. The aqueous layer was basified with NaOH 3 N and extracted with diethyl ether. The organic layer was separated, dried (MgSO₄), filtered and the solvent was evaporated. Yield: 8 g of **intermediate 12** (28 %).

### c-2. Preparation of intermediate 13

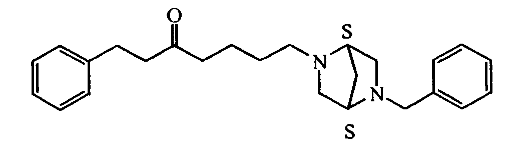

A mixture of **intermediate 11** (0.0089 mol), 2-(phenylmethyl)-2,5-diazabicyclo[2.2.1]heptane, dihydrobromide, (1S,4S) (0.0089 mol) and K₂CO₃ (0.0267 mol) in CH₃CN (23 ml) was stirred at 80 °C for 48 hours, then brought to room temperature and poured out into H₂O. The organic layer was acidified with HCl 1 N. The aqueous layer was basified with NaOH 3 N and extracted with diethyl ether. The organic layer was separated, dried (MgSO₄), filtered and the solvent was evaporated. Yield: 1.23 g of **intermediate 13.**

### c-3. Preparation of intermediate 15

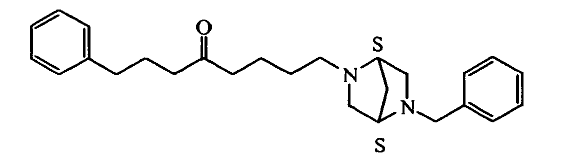

**Intermediate 15** was prepared according to the procedure described for intermediate 13 (A3.c-2), but starting from **intermediate 14**. Yield: **Intermediate 15.**

### c-4. Preparation of intermediate 18

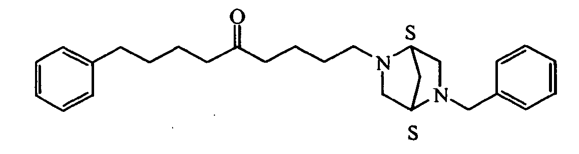

A mixture of **intermediate 17** (0.0059 mol), 2-(phenylmethyl)-2,5-diazabicyclo[2.2.1]heptane, dihydrobromide, (1S,4S) (0.0059 mol) and K₂CO₃ (0.0179 mol) in CH₃CN (15 ml) was stirred at 80 °C for 48 hours, then brought to room temperature, poured out into H₂O, extracted with diethyl ether and acidified with HCl 3 N. The aqueous layer was basified with concentrated NaOH and extracted with diethyl ether. The organic layer was washed with saturated aqueous NaCl solution, dried (MgSO₄), filtered and the solvent was evaporated. Yield: 1.967 g of **intermediate 18** (81 %).

### Example A4

### a. Reparation of intermediate 19

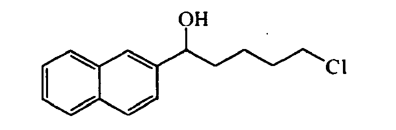

A solution of 1-bromo-4-chlorobutane (22.25 ml, 0.19 mol) in diethyl ether (100 ml) was added dropwise (under N₂ atmosphere) to a suspension of activated Mg turnings (4.67 g, 0.19 mol) in diethyl ether (100 ml). Some crystals of iodine were also added. The temperature in the flask increased, and the orange colour turned to white. Once the addition of 1-bromo-4-chlorobutane was completed, the reaction was cooled in an ice-bath and 2-naphthalenecarboxaldehyde (20.00 g, 0.13 mol) was added dropwise as a solution in THF (200 ml, dry). The reaction mixture was stirred in the ice-bath for 4 hours. Then the mixture was quenched with NH₄Cl 1 N. Both phases were separated. The organic layer was washed with brine, dried (MgSO₄), filtered and the solvent was evaporated. The residue was purified by flash chromatography (eluent: n-hexane/EtOAc 20:1). The desired fractions were collected and the solvent was evaporated, yielding **intermediate 19.**

### b. Preparation of intermediate 20

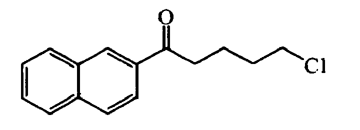

**Intermediate 19** (9.97 g, 0.04 mol) was dissolved in CH₂Cl₂ (120 ml) and the flask was cooled in an ice-bath. MnO₂ (34.85 g, 0.40 mol) was added and the reaction mixture was stirred in the ice-bath for 1 hour and then overnight at room temperature. The next morning, an additional amount of MnO₂ (10 equivalent) was added, and in the afternoon again an additional amount of MnO₂ (10 equivalent) was added. The mixture was stirred overnight at room temperature. Then MnO₂ was removed by filtration over Celite. The product was purified by flash chromatography (eluent: n-hexane/EtOAc 40:1). Yield: 6.91 g of **intermediate 20** (70 %).

### c-1. Preparation of intermediate 22

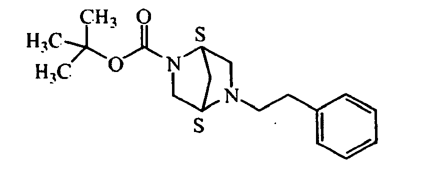

A mixture of (1S,4S)-2,5-diazabicyclo[2.2.1]heptane-2-carboxylic acid, 1,1-dimethylethyl ester (0.2 g, 0.001 mol), (2-bromoethyl)benzene (0.224 g, 0.0012 mol), K₂CO₃ (0.279 g, 0.002 mol), KI (0.167 g, 0.001 mol) and *N,N,N*-tributyl-1-butanaminium chloride (0.02 g) in CH₃CN (5 ml) was heated overnight at 80 °C. Then the mixture was cooled to room temperature and the precipitate was filtered off and washed with EtOAc. The organic phases were washed with brine, dried (MgSO₄), filtered and the solvent was evaporated. The product was purified by flash column chromatography (eluent: CH₂Cl₂/CH₃OH from 50/1 till 40/l). The product fractions were collected and the solvent was evaporated. The residue was dried (vacuum, room temperature). Yield: 0.23 g of intermediate 22 (pale yellow oil; 74 %).

**Intermediate 55** 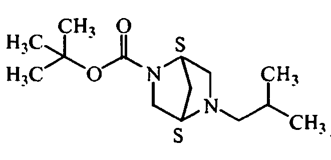 was prepared according to an analogous protocol as **intermediate 22**, but starting from 1-bromo-2-methylpropane instead of (2-bromoethyl)benzene. Yield: 65 % (colourless oil).

### c-2. Reparation of intermediate 23

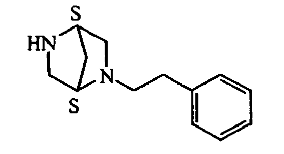

**Intermediate 22** (1 g, 0.0033 mol) was dissolved in CH₂Cl₂ (10 ml) and the solution was cooled to 0 °C. Then trifluoroacetic acid (7.54 g, 0.0066 mol) was added dropwise at 0 °C. After the addition was completed, the reaction mixture was warmed to room temperature and stirred for 2 hours. The solvent was evaporated and the residue was dried. The product was obtained as a colourless oil and it was used in the next step without further purification. Yield: **Intermediate 23.**

**Intermediate 54** 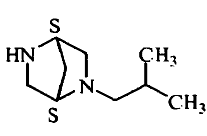 was prepared according to an analogous protocol as **intermediate 23**, but starting from **intermediate 55. Intermediate 54** was obtained as a brown oil and was used as such in the next reaction step.

### d-1. Preparation of intermediate 21

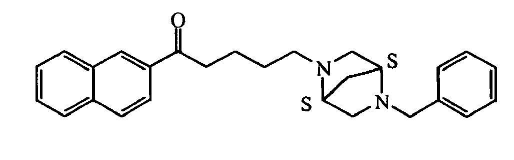

A mixture of **intermediate 20** (0.00571 mol), 2-(phenylmethyl)-2,5-diazabicyclo[2.2.1]heptane, dihydrobromide, (1S,4S) (0.00571 mol) and K₂CO₃ (0.0171 mol) in CH₃CN (150 ml) was stirred under reflux overnight. Then the mixture was cooled to room temperature, poured out into water and extracted with diethyl ether. The organic layer was extracted with HCl 1 N. The resulting aqueous layer was basified with NaOH 3 N and extracted with diethyl ether. The organic layer was separated, washed with brine, dried (MgSO₄), filtered and the solvent was evaporated. Yielding: 1.7 g of **intermediate 21** (75 %).

### d-2. Preparation of intermediate 24

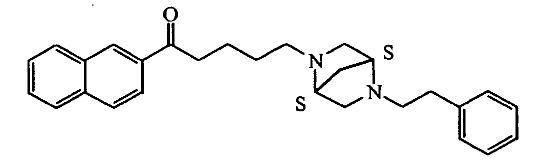

**Intermediate 23** (0.669 g, 0.0033 mol) was dissolved in CH₃CN (10 ml). **Intermediate 20** (0.98 g, 0.004 mol), K₂CO₃ (1.14 g, 0.0083 mol), KI (0.55 g, 0.0033 mol) and *N,N,N*-tributyl-1-butanaminium chloride (0.067 g, 10 % w/w) were added to the solution and the mixture was stirred at 80 °C for 15 hours. Then K₂CO₃ was filtered off and washed with EtOAc. The organic phases were combined and were washed with brine, dried (MgSO₄), filtered and the solvent was evaporated. The residue was purified by flash column chromatography (eluent: CH₂Cl₂/CH₃OH; from 50/1 to 40/1 to 30/1 to 20/1 to 10/1). The product fractions were collected and the solvent was evaporated. The residue was dried (vacuum, room temperature), yielding 0.8 g of **intermediate 24** (pale green; yield: 59 % over two steps).

### d-3. Preparation of intermediate 25

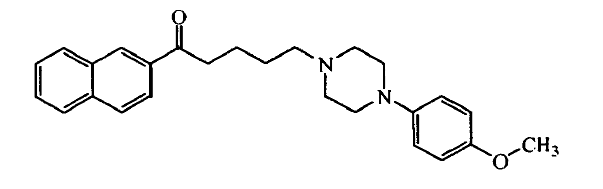

A mixture of **intermediate 20** (0.5 g, 0.00202 mol), 1-(4-methoxyphenyl)piperazine (0.00405 mol) and K₂CO₃ (0.84 g, 0.00608 mol) in CH₃CN (6.06 ml) was refluxed at 80 °C for 2 days. Then the inorganic salts were removed by filtration and purification was performed by flash chromatography (eluent: n-hexane/EtOAc; initial conditions: 5/1, product at 1/1). The product fractions were collected and the solvent was evaporated. Yield: 0.5 g of **intermediate 25** (61 %).

### d-4. Preparation of intermediate 26

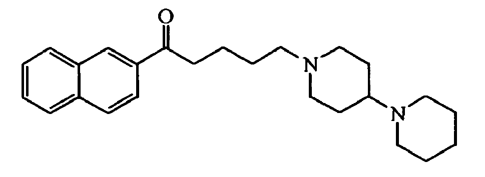

**Intermediate 26** was prepared according to the procedure for intermediate 21 (A4.d-1), starting from 1,4'-bipiperidine and **intermediate 20**. Yield: 0.92g of **Intermediate 26 (30%).**

### d-5. Preparation of intermediate 33

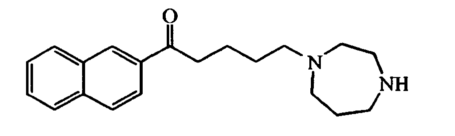

A mixture of **intermediate 20** (5 g, 0.02mol), homopiperazine (0.06 mol) and K₂CO₃ (0.06 mol) in CH₃CN (60 ml) was stirred under reflux for 18 hours then cooled down to room temperature, and poured out into water. The organic layer was extracted with EtOAc and dried (MgSO₄), filtered and the solvent was evaporated. The residue was purified by column chromatography over silica gel (eluent: CH₂Cl₂/CH₃OH/NH₄OH 97/3/0.1, 15-40 µm, 90g). The pure fractions were collected and the solvent was evaporated. Yield: 2.3 g of **intermediate 33** (37 %).

**Intermediate 53** 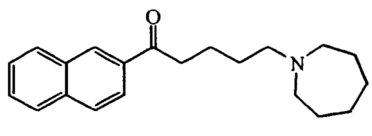 was prepared according to the same protocol as **intermediate 33**, but starting from hexahydro-1H-azepine instead of homopiperazine. The crude compound was purified by column chromatography over silica gel (eluent: CH₂Cl₂/CH₃OH/NH₄OH 93/7/0.6). Yield: 81 %.

### d-6. Preparation of intermediate 34

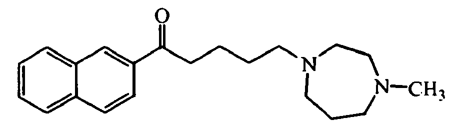

A mixture of **intermediate 20** (1.00 g, 0.00405 mol), 1-methylhomopiperazine (1.01 ml, 0.0081 mol) and K₂CO₃ (1.68 g, 0.0081 mol) in CH₃CN (12.16 ml) was refluxed at 80 °C over the weekend. Inorganic salts were removed by filtration and the crudes were purified by flash chromatography (eluent: n-hexane/EtOAc). The desired fractions were collected and the solvent was evaporated. Yield: 0.26 g of **intermediate 34** (20 %).

### d-7. Preparation of intermediate 44

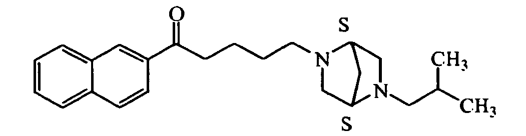

A mixture of **intermediate 54** ((1S,4S)-2-(2-methylpropyl)-2,5-diazabicyclo[2.2.1]heptane) (0.49 g, 0.0032 mol), intermediate 20 (0.95 g, 0.0038 mol), K₂CO₃ (1.1 g, 0.008 mol), KI (0.53 g, 0.0032 mol) and *N,N,N*-tributyl-1-butanaminium chloride (0.049 g) in CH₃CN (5 ml) was heated for 15 hours at 80 °C. Then the mixture was cooled to room temperature and the precipitate was filtered off and washed with EtOAc. The organic phases were washed with brine, dried (MgSO₄), filtered and the solvent was evaporated. The product was purified by flash column chromatography (eluent: CH₂Cl₂/CH₃OH from 50/1 to 40/1 to 30/1 to 20/1 to 10/1). The desired fractions were collected and the solvent was evaporated. The residue was dried (vacuum, room temperature). Yield: 1.04 g of **intermediate 44** (colourless oil; 87 %).

### d-8. Preparation of intermediate 45

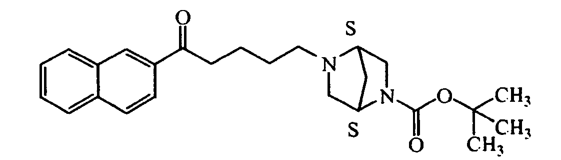

A mixture of 2,5-diazabicyclo[2.2.1]heptane-2-carboxylic acid, 1,1-dimethylethyl ester(1S,4S), (1 g, 0.0050 mol), **intermediate 20** (1.49 g, 0.0060 mol), K₂CO₃ (1.38 g, 0.01 mol), KI (0.83 g, 0.005 mol) and *N,N,N*-tributyl-1-butanaminium chloride (0.1 g) in CH₃CN (15ml) was heated for 15 hours at 80 °C. Then the mixture was cooled to room temperature and the precipitate was filtered off and washed with EtOAc. The organic phases were washed with brine, dried (MgSO₄), filtered and the solvent was evaporated. The product was purified by flash column chromatography (eluent: CH₂Cl₂/CH₃OH from 50/1 till 20/1). The desired fractions were collected and the solvent was evaporated. The residue was dried (vacuum, room temperature). Yield: 1.62 g of **intermediate 45** (colourless oil; 79 %).

### d-9. Preparation of intermediate 49

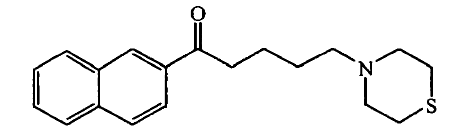

**Intermediate 20** (5 g, 20.26 mmol) and thiomorpholine (10.5 g, 101.2 mmol) were stirred at 100 °C for 2 hours. The mixture was diluted with H₂O and extracted with CH₂Cl₂. The organic layer was washed with brine, dried over MgSO₄, filtered and the solvent was evaporated. The crude product was purified by chromatography over silica gel (15-40 µm / 90 g / eluent: CH₂Cl₂/MeOH/NH₄OH from 97/3/0.1 to 95/5/0.5). The desired fractions were collected and the solvent was evaporated to give 6.1 g of a residue that was crystallized from DIPE. Yield: 5.8 g **of intermediate 49** (91 %; mp = 68 °C).

### e. Preparation of intermediate 46

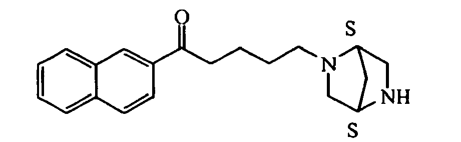

**Intermediate 45** (0.83 g, 0.002 mol) was dissolved in CH₂Cl₂ (10 ml) and the mixture was cooled to 0 °C. Then trifluoroacetic acid (3.1 ml;0.041 mol) was added dropwise at 0 °C. After the addition was completed, the reaction mixture was allowed to warm up to room temperature and stirred for 2 hours. The solvent was evaporated and the residue was dried under vacuum at room temperature. The residue was dissolved in THF and K₂CO₃ was added. The mixture was stirred at room temperature for 20 minutes and then K₂CO₃ was filtered off and washed with THF. The solvent was evaporated and the residue was dried under vacuum at room temperature. Yield: 0.627 g of **intermediate 46** (100 %). The product was used in the next step without further purification.

### f. Preparation of intermediates 51 and 52

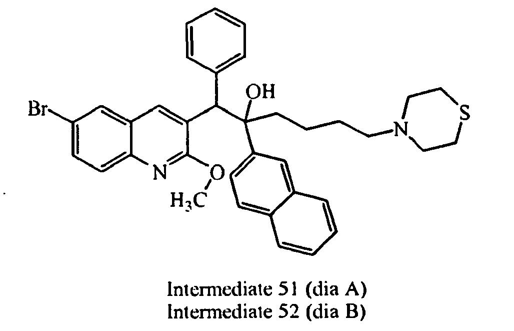

*n*BuLi (6.9 ml of a 1.6 M solution in hexanes; 0.011 mol) was added at -20 °C to a solution of diisopropylamine (1.5 ml, 0.011 mol) in THF (10 ml) under N₂ flow. The mixture was stirred at -20 °C for 30 minutes. A solution of 6-bromo-2-methoxy-3-(phenylmethyl)-quinoline [654655-69-3] (intermediate compound 3 of WO 2004/011436) (1.5 g, 0.0044 mol) in THF (10 ml) was added dropwise at -70 °C. The mixture was stirred at -70 °C for 1 hour. A solution of **intermediate 49** (1.8 g, 0.0054 mol) in THF (10 ml) was added at -70 °C. The mixture was stirred at -70 °C for 1 hour, poured out into H₂O and extracted with EtOAc. The organic layer was washed with brine, dried (MgSO₄), filtered and the solvent was evaporated. The residue was purified twice by column chromatography over silica gel (eluent: Cyclohexane/EtOAc 90/10; 400 g, 15-40 µm). Two different fractions were collected and the solvent was evaporated. The residue of the first fraction (0.8 g) was crystallized in DIPE/CH₃CN, filtered off and dried to give 141 mg of **intermediate 51** (dia A, m.p.: 164 °C). The residue of the second fraction (0.40 g) was crystallized from DIPE/CH₃CN, filtered off and dried to give 141 mg of **intermediate 52** (dia B, m.p.: 207 °C).

### Example A5

### a. Preparation of intermediate 27 and 28

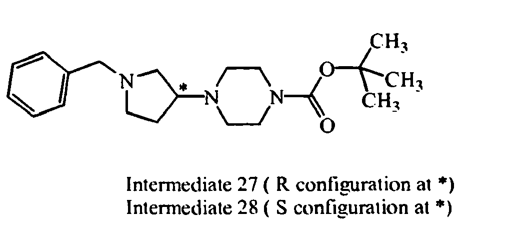

A mixture of 1-piperazinecarboxylic acid, 1,1-dimethylethyl ester (0.25 mol) and 1-(phenylmethyl)-3-pyrrolidinone (0.25 mol) in CH₃OH (400 ml) was hydrogenated at 50 °C for 18 hours with Pd/C 10 % (5 g) as a catalyst in the presence of thiophene solution (3 ml). After uptake of H₂ (1 equivalent), the catalyst was filtered off and the filtrate was evaporated. The residue was crystallised from hexane, the resulting precipitate was filtered off and dried. This fraction was separated into its enantiomers by Chiral separation (Chiralpak AD; eluent: CH₃OH). Two product fractions were collected and the solvents were evaporated. Yield Fraction 1: 30 g of **intermediate 27** (R). Yield Fraction 2: 26 g of **intermediate 28** (S).

### b. Preparation of intermediate 29 and 30

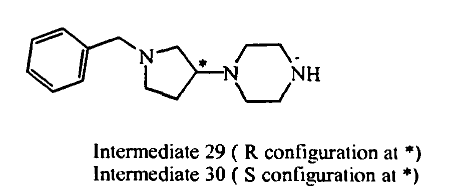

A mixture of **intermediate 27** (0.0868 mol), HCl/2-propanol (85 ml) and CH₃OH (350 ml) was stirred and refluxed for 1 hour, then the reaction mixture was cooled. The precipitate was filtered off and was taken up in H₂O. The mixture was alkalized with a 50 % NaOH solution and extracted with CH₂Cl₂. The organic layer was separated, dried (MgSO₄), filtered off and the solvent was evaporated. Yield: 16.5 g of intermediate 29 (78 %, R).

Intermediate 30 was prepared according to the procedure for intermediate 29 (A5.b), but starting from **intermediate 28.** Yield: **Intermediate 30** (78 %, S).

### c. Preparation of intermediate 31 and 32

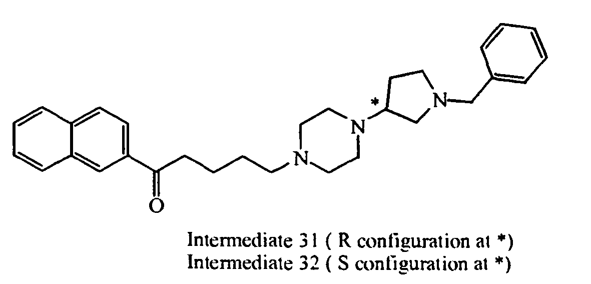

A mixture of **intermediate 20** (0.004 mol), **intermediate 29** (0.0044 mol) and K₂CO₃ (0.01 mol) in CH₃CN (20 ml) was stirred at 80 °C for 18 hours, then cooled to room temperature, poured out into H₂O and extracted with EtOAc. The organic layer was extracted with HCl 1 N, basified with NaOH 3 N at 0 °C and extracted with diethyl ether. The organic layer was washed with H₂O, then with saturated aqueous NaCl solution, dried (MgSO₄), filtered and the solvent was evaporated. Yield: 0.7 g of **intermediate 31** (38 %; R).

### Intermediate 32 was prepared according to the procedure for intermediate 31 (A5.c), but starting from intermediate 30. Yield: 44 % (S)

### Example A6

### a. Preparation of intermediate 35

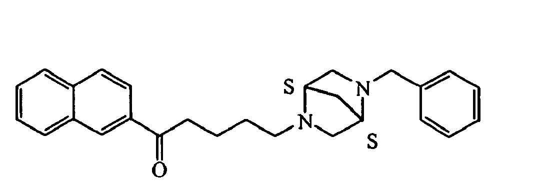

(1S,4S)-2-(Phenylmethyl)-2,5-diazabicyclo[2.2.1]heptane, hydrobromide (1:2) (CAS [116258-17-4]) (4.00 g, 0.011 mol) and K₂CO₃ (4.56 g, 0.033 mol) were mixed in CH₃CN (19 ml) and the mixture was stirred for 5 minutes at room temperature. Subsequently, **intermediate 20** (1.56 g, 0.00634 mol) was added and the reaction mixture was refluxed for 48 hours at 80 °C. Then K₂CO₃ was removed by filtration and the product was purified by flash chromatography (eluent: CH₂Cl₂/MeOH 30/1). The desired fractions were collected and the solvent was evaporated. Yield: 2.17 g of **intermediate 35** (86 %; S,S).

### Example A7

### a. Preparation of intermediate 36

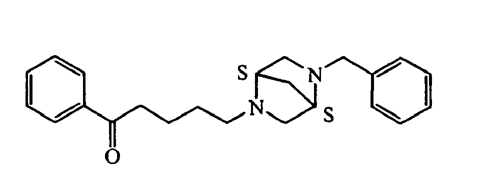

(1S,4S)-2-(Phenylmethyl)-2,5-diazabicyclo[2.2.1]heptane, hydrobromide (1:2) (0.45 g, 0.00128 mol) and K₂CO₃ (0.266 g, 0.00193 mol) were mixed in CH₃CN (2.00 ml) and the mixture was stirred for 5 minutes. 5-Chloro-1-phenyl-1-pentanone (0.14 g, 0.00071 mol) was added and the reaction mixture was refluxed at 80 °C for 48 hours. Then the K₂CO₃ was removed by filtration and the product was purified by flash chromatography (eluent: CH₂Cl₂/MeOH started at 20/1 and product at 10/1). The desired fractions were collected and the solvent was evaporated. Yield: 0.24 g of **intermediate 36** (quantitative yield).

### Example A8

### a. Preparation of intermediate 37

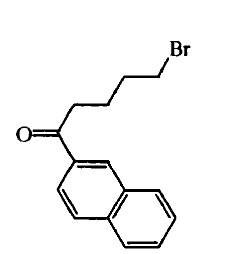

A solution of 2-bromo-naphtalene (4.141 g; 0.02 mol) in THF (20 ml) was slowly added to Mg (0.583 g; 0.024 mol) activated with I₂ and the reaction mixture was refluxed for 2 hours. This solution was slowly added at room temperature to a solution of 5-bromo-pentanoyl chloride (4.38 g; 0.022 mol) in THF (25 ml). The reaction mixture was stirred for 2 hours, then poured on ice and water and neutralized with Na₂CO₃. The aqueous layer was extracted twice with CH₂Cl₂. The combined organic layers were dried over Na₂SO₄, and the solvent was evaporated till dryness. The crude product was crystallized in Et₂O, and the precipitate was filtered off and dried. Yield: 3 g of **intermediate 37** (52 %).

### b-1. Preparation of intermediate 38

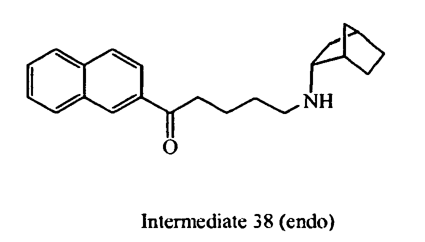

A mixture of **intermediate 37** (1 g; 0.003 mol), 2-norbornanamine, hydrochloride (0.9 g; 0.006 g) and potassium carbonate (1.24 g; 0.009 mol) in acetonitrile (15 ml) was refluxed for 2.5 hours. The reaction mixture was cooled to room temperature and the solid phase was filtered off and washed with CH₂Cl₂. The organic solution was evaporated till dryness. The crude product was purified by chromatography over silica gel (eluent: hexane/Et₂O/Et₃N from 1.5/3.5/0 to 1/4/0.04). The desired fractions were collected and the solvent was evaporated. Yield: 0.7 g of **intermediate 38** (72 %; endo). (It is considered that endo or exo isomerism for bridged ring systems is within the knowledge of the skilled person.)

### b-2. Preparation of intermediate 48

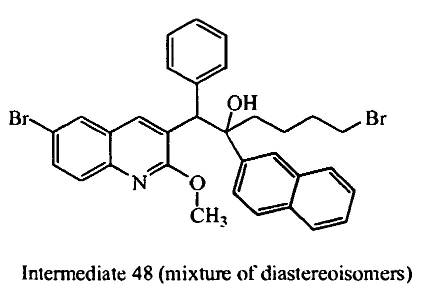

Lithium diisopropylamine (0.5 ml of a 2.0 M solution in THF/heptanes; 0.00103 mol) was slowly added to a solution of 6-bromo-2-methoxy-3-(phenylmethyl)-quinoline (intermediate compound 3 (Ex. A3) of WO2004/011436) (0.33 g, 0.00103 mol) in THF (3 ml; dry) at -78°C under Ar atmosphere. The mixture was stirred for 30 minutes at - 78 °C. Then **intermediate 37** (0.20 g, 0.00068 mol) was added dropwise as a solution in THF (3 ml; dry) and the reaction mixture was stirred for 1 hour at -70 °C. Then H₂O was added (quenching) at -70 °C, followed by EtOAc. The layers were separated and the organic layer was washed with brine and dried (MgSO₄), filtered and the solvent was evaporated. The residue was purified by flash chromatography over silica gel (petroleum ether/Et₃N 5:0.1). The desired fractions were collected and the solvent was evaporated. Yield: 0.2 g of **intermediate 48** (mixture of diastereoisomers; 47%).

### Example A9

### a. Preparation of intermediate 39

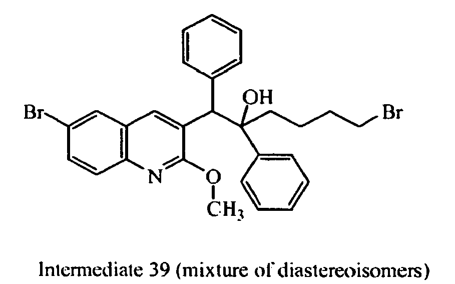

THF (2 ml) and S-bromo-1-phenyl-1-pentanone (0.100 g, 0.00042 mol) were added to the anhydrous cerium chloride at room temperature. The white suspension was stirred until a gel-like mixture was formed. 6-bromo-2-methoxy-3-(phenylmethyl)-quinoline (intermediate compound 3 (Ex. A3) of W02004/011436) (0.20 g, 0.00063 mol) was dissolved in 5 ml of THF and the reaction mixture was cooled to -78 °C. Subsequently lithium diisopropylamine (0.3 ml of a 2.0 M solution in THF/heptanes; 0.00063 mol) was added. After stirring for half an hour at -78 °C, the first solution of ketone with cerium chloride was slowly added and the reaction mixture was stirred at -78°C for 15 minutes. Then the mixture was hydrolyzed with water, filtered through a Celite pad and extracted with CH₂Cl₂. The organic layer was dried (Na₂SO₄), filtered and the solvent was evaporated till dryness, yielding crude **intermediate 39** (mixture of diastereoisomers) that was used as such in the next reaction step.

### Example A 10

### a. Preparation of intermediate 40

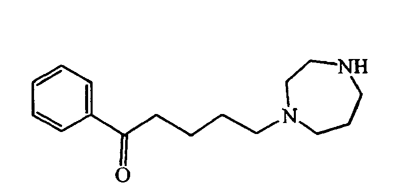

In a sealed vessel, a mixture of 5-chloro-1-phenyl-1-pentanone (1.2g, 0.006 mol), hexahydro-1,4-diazepine (2.4 g, 0.024 mol) and potassium carbonate (4.1 g) in CH₃CN (15 ml) was stirred overnight at 90 °C and was then poured out into H₂O and extracted with CH₂Cl₂. The organic layer was washed with saturated aqueous NaCl solution, dried (MgSO₄), filtered and the solvent was evaporated, yielding **intermediate 40** (71 %).

### Example A11

### a. Preparation of intermediate 41

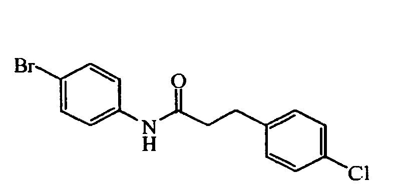

4-Chlorobenzenepropanoyl chloride (0.466 mol) was added slowly at 5°C to a solution of 4-bromobenzenamine (0.388 mol) in Et₃N (70 ml) and CH₂Cl₂ (700 ml). The mixture was stirred at room temperature for 1 hour. H₂O was added. The precipitate was filtered off, washed with H₂O and dried. The residue was recrystallized from diethyl ether. The precipitate was filtered off and dried. Yield: 110 g of **intermediate 41** (83 %) (m.p. 194°C).

### b. Preparation of intermediate 42

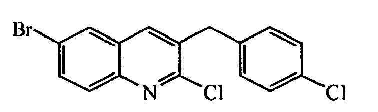

POCl₃ (192.6 ml) was added slowly at 5°C to DMF (35.4 ml). **Intermediate 41** (prepared according to A11.a) (0.296 mol) was added. The mixture was stirred at 80°C for 12 hours, poured out slowly on ice and extracted with CH₂Cl₂. The organic layer was separated, dried (MgSO₄), filtered and the solvent was evaporated. The product was used without further purification. Yield: 150 g of **intermediate 42**.

### c. Preparation of intermediate 43

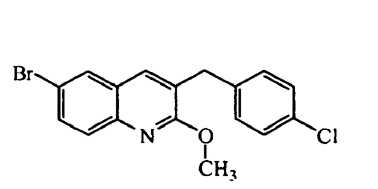

A mixture of **intermediate 42** (prepared according to A11.b) (0.409 mol) in CH₃ONa solution 30% in CH₃OH (300 ml) and CH₃OH (300 ml) was stirred and refluxed for 15 hours. The mixture was poured out on ice and extracted with CH₂Cl₂. The organic layer was separated, dried (MgSO₄), filtered and the solvent was evaporated. The residue (150 g) was purified by column chromatography over silica gel (eluent: cyclohexane/CH₂Cl₂ 90/10; 35-70 µm). The pure fractions were collected and the solvent was evaporated. The residue was crystallized from diethyl ether. The precipitate was filtered off and dried. Yield: 27 g of **intermediate 43** (18 %) (m.p. 100°C).

### Example A12

### a. Preparation of intermediate 47

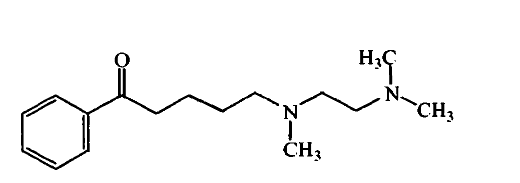

A mixture of 5-chloro-1-phenyl-1-pentanone (1.12 g, 0.0057 mol), *N1,N1,N2-*trimethyl-1,2-ethanediamine (2.0 g, 0.011 mol) and K₂CO₃ (2.36 g, 0.017 mol) in DMF (30 ml) was stirred at 80 °C for 48 hours. K₂CO₃ was filtered off and the solvent was evaporated. The crude product was purified by column chromatography over silica gel (CH₂Cl₂/CH₂OH 30/1). Yield: 0.40 g of **intermediate 47** (27 %).

### Example A13

### a. Preparation of intermediate 50

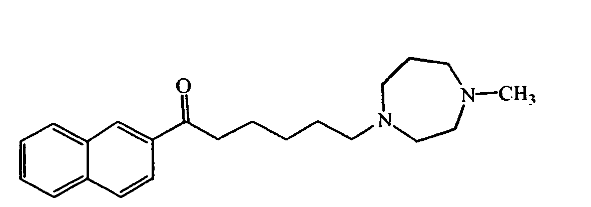

**Intermediate 50** was prepared according the procedure described for **intermediate 21** (Example A4.d-1) but starting from 6-chloro-1-(2-naphthalenyl)-1-hexanone (prepared according to the procedures of W02007/000435) instead of **intermediate 20,** and hexahydro-1 methyl-1,4-diazepine. Yield: 3.5 g of **intermediate 50** (55 %).

### B. Preparation of the final compounds

### Example B1

### Preparation of compound 1 and 2

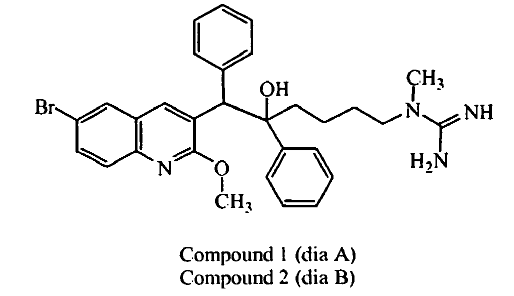

1H-Pyrazole-1-carboximidamide, monohydrochloride (0.0005 mol) (E+Z) was added to a mixture of **intermediate 4** (0.0002 mol) and DIPEA (0.0005 mol) in DMF (2 ml). The mixture was stirred at room temperature for 24 hours. H₂O was added. The mixture was extracted with EtOAc. The organic layer was washed with H₂O, then with saturated aqueous NaCl solution, dried (MgSO₄), filtered and the solvent was evaporated. The residue was crystallized from diethyl ether. The precipitate was filtered off and dried. Yield: 0.019 g of **compound 1** (12 %; dia A).

**Compound 2** was prepared according to the same protocol as compound 1, but starting from **intermediate 5**. The residue was crystallized from diethyl ether/DIPE. Yield: 0.027 g **compound 2** (17 %; dia B).

### Example B2

### Preparation of compound 3

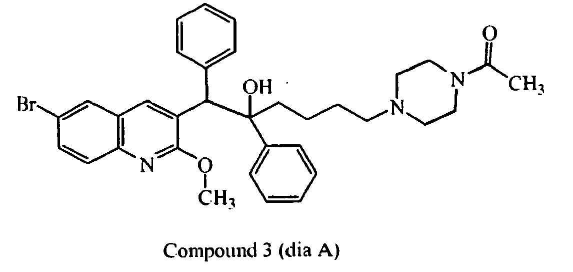

A mixture of **intermediate 8** (0.0001 mol), acetyl chloride (0.0001 mol) and Et₃N (0.0001 mol) in DCM (3 ml) was stirred at room temperature for 18 hours. H₂O was added. The mixture was extracted with CH₂Cl₂. The organic layer was washed with saturated aqueous NaCl solution, dried (MgSO₄), filtered and the solvent was evaporated. The residue (0.08 g) was purified by column chromatography over silica gel (eluent: CH₂Cl₂/CH₃OH/NH₄OH 98/2/0.2 to 93/7/0.7; 5 µm). The pure fractions were collected and the solvent was evaporated. Yield: 0.033 g of **compound 3** (41 %; dia A).

### Example B3

### a. Preparation of compound 4 and 5

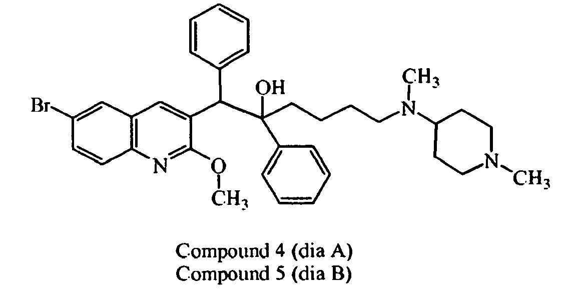

Lithium diisopropylamine ([4111-54-0]) (1.00 ml of a 2.0 M solution in THF/heptane; 0.002 mol) was dissolved in THF (6.65 ml; dry) and cooled on an ice-bath at -70 °C. 6-Bromo-2-methoxy-3-(phenylmethyl)-quinoline (intermediate compound 3 (Ex. A3) of WO2004/011436) (0.55 g, 0.00166 mol,; [654655-69-3]) was added dropwise as a solution in THF (5.00 ml; dry) and the mixture was stirred for 2 hours at -70 °C. Then **intermediate 9** (0.48 g, 0.00166 mol) was added dropwise as a solution in THF (5.00 ml; dry) and the reaction mixture was stirred for 3 hours at -70 °C. Then H₂O was added (quenching) at -70 °C, followed by EtOAc. The layers were separated and the organic layer was washed with brine, dried (MgSO₄), filtered and the solvent was evaporated. The residue was purified by flash chromatography (eluent: DCM/CH₃OH 50:1). The desired fractions were collected and the solvent was evaporated. The crude residue (mixture of diastereoisomers) was purified into the diastereoisomers by column chromatography (normal phase, Kromasil Si 10 µm, eluent: CH₂Cl₂/CH₃OH/NH₄OH : 95/5/0.5). The desired fractions were collected and the solvent was evaporated. Yield : 0.025g of **Compound 4** (dia A, 2.6%) and 0.023g of **compound 5** (dia B, 2.5%).

### b. Preparation of compound 10 and 11

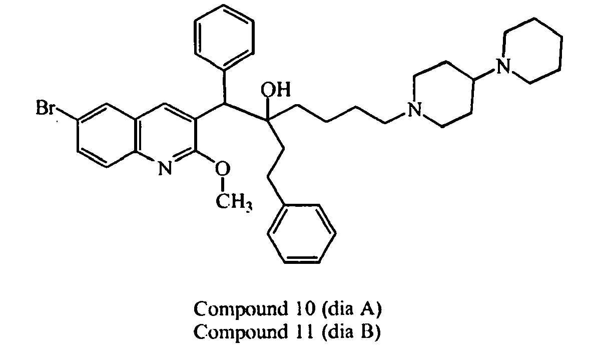

*n*BuLi (0.007 mol, 4.4 ml of a 1.6 M solution in hexane) was added dropwise at -20 °C to a solution of diisopropylamine (0.007 mol) in THF (12 ml) under N₂ flow. The mixture was stirred for 20 minutes, then cooled to -70 °C. A solution of 6-Bromo-2-methoxy-3-(phenylmethyl)-quinoline (intermediate compound 3 (Ex. A3) of W02004/011436) (0.0058 mol) in THF (20 ml) was added. The mixture was stirred at -70 °C for 90 minutes. A solution of **intermediate 12** (0.007 mol) in THF (25 ml) was added. The mixture was stirred at -70 °C for 3 hours. H₂O was added at -30 °C. The mixture was extracted with EtOAc. The organic layer was separated, dried (MgSO₄), filtered and the solvent was evaporated. The residue (1.09 g) was purified by column chromatography over silica gel (eluent: CH₂Cl₂/CH₃OH/NH₄OH 95/5/0.1; 15-40 µm) and then over Kromasil (eluent: CH₂Cl₂/CH₃OH/NH₄OH 97/3/0.3 to 88/12/1.2; 5 µm). Two fractions were collected and the solvent was evaporated. Yield: 0.42 g of fraction 1 and 0.23 g of fraction 2. Fraction 1 was crystallized from DIPE/diethyl ether. The precipitate was filtered off and dried. Yield: 0.31 g of **compound 10** (8 %; dia A). Fraction 2 was crystallized from DIPE. The precipitate was filtered off and dried. Yield: 0.195 g of **compound 11** (5 %) (m.p.: 164 °C; dia B).

### c. Preparation of compound 12 and 13

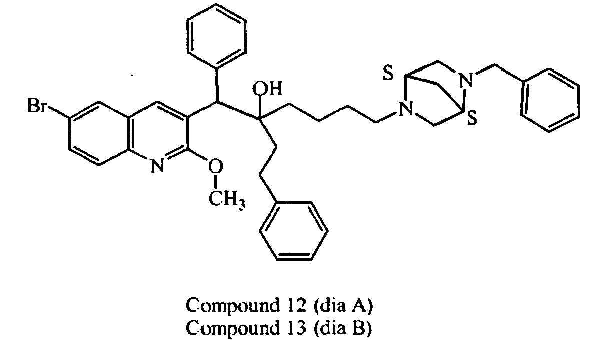

nBuLi (0.0033 mol, 2.1 ml of a 1.6 M solution in hexane) was added dropwise at -20 °C to a solution of diisopropylamine (0.0033 mol) in THF (5 ml) under N₂ flow. The mixture was stirred for 20 minutes, then cooled to -70 °C. A solution of 6-bromo-2-methoxy-3-(phenylmethyl)-quinoline (intermediate compound 3 (Ex. A3) of W02004/011436) (0.0027 mol) in THF (9 ml) was added. The mixture was stirred at -70 °C for 90 minutes. A solution of **intermediate 13** (0.0033 mol) in THF (12 ml) was added. The mixture was stirred at -70 °C for 90 minutes, then poured out on ice at -30 °C. The organic layer was separated, dried (MgSO₄), filtered and the solvent was evaporated. The residue was purified by column chromatography over silica gel (eluent: CH₂Cl₂/CH₃OH/NH₄OH 95/5/0.1; 15-40 µm). Two fractions were collected and the solvent was evaporated. Yield: 0.16 g of fraction 1 and 0.1 g fraction 2. Fraction 2 was purified by Super Critical Fluid chromatography (eluent: CO₂/CH₃OH/isopropylamine 88/12/0.5). The pure fractions were collected and the solvent was evaporated. The residue was crystallized from DIPE. The precipitate was filtered off and dried. Yield: 0.145 g of **compound 12** (7.6 %; dia A) and 0.057 g of **compound 13** (3.1 %; dia B).

### d. Preparation of compound 14 and 15

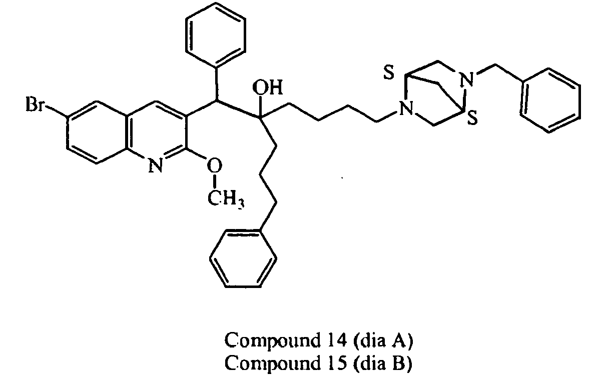

**Compound 14** and **compound 15** were prepared according to the procedure for compound 12 and 13 (B3.c), but starting from **intermediate 15.** The work-up procedure was different, namely after the column chromatography over silica gel, only 1 fraction was obtained and this fraction was further purified over a Sunfire^{™} C18 column from Waters (5 µm 19 x 150 mm) with a flow rate of 20 ml/min. Two mobile phases (mobile phase A: 100 % acetonitrile; mobile phase B: 100 % 63mM ammonium hydrogen carbonate pH=8 (in ultra pure water) were employed to run a gradient condition from 90 % A, 10 % B to 100 % A in 14 minutes, and reequilibrated with initial conditions for 6 minutes. Two fractions were collected and the solvent was evaporated. Both residues were crystallized from DIPE. The precipitates were filtered off and dried. Yield: 0.139 g of **compound 14** (6.6 %). Yield: 0.06 g of **compound 15** (2.9 %).

### e. Preparation of compound 16 and 17

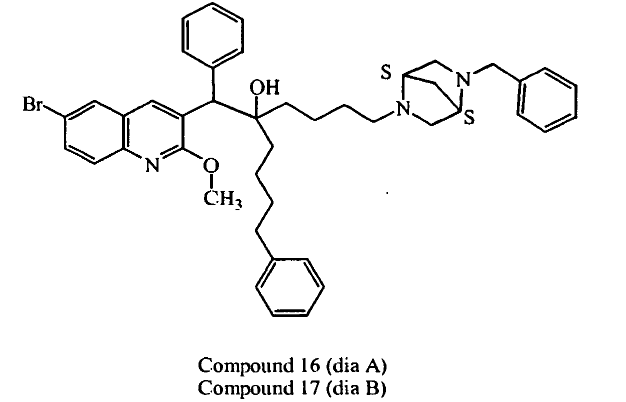

*n*BuLi (0.0024 mol, 1.54 ml of a 1.6 M solution in hexane) was added dropwise at -20 °C to a solution of diisopropylamine (0.0024 mol) in THF (4.8 ml) under N₂ flow. The mixture was stirred for 20 minutes, then cooled to -70 °C. A solution of 6-bromo-2-methoxy-3-(phenylmethyl)-quinoline (intermediate compound 3 (Ex. A3) of W02004/011436) (0.002 mol) in THF (6.8 ml) was added. The mixture was stirred at -70 °C for 90 minutes. A solution of **intermediate 18** (0.0024 mol) in THF (10 ml) was added. The mixture was stirred for 90 minutes, brought to -20 °C, poured out into H₂O and extracted with EtOAc. The organic layer was washed with saturated aqueous NaCl solution, dried (MgSO₄), filtered and the solvent was evaporated. The residue was purified by column chromatography over silica gel (eluent: CH₂Cl₂/CH₃OH/NH₄OH 95/5/0.1; 15-40 µm). The pure fractions were collected and the solvent was evaporated. The residue (0.473 g; mixture of diastereoisomers) was further purified with reversed phase chromatography on a Sunfre^{™} C18 column from Waters (5 µm19 x 150 mm) with a flow rate of 20 ml/min. Two mobile phases (mobile phase A: 100 % methanol; mobile phase B: 100 % 63mM ammonium hydrogen carbonate pH=8 (in ultra pure water)) were employed to run a gradient condition from 90 % A, 10 % B to 100 % A in 14 minutes, and reequilibrated with initial conditions for 6 minutes. Three fractions were collected and the solvent was evaporated. Yield: 0.134 g of **compound 16** (8.9 %; fraction 1; dia A) and 0.1 g of **compound 17** (6.6 %; fraction 3; dia B).

### Example B4

### Preparation of compound 6, 7, 8 and 9

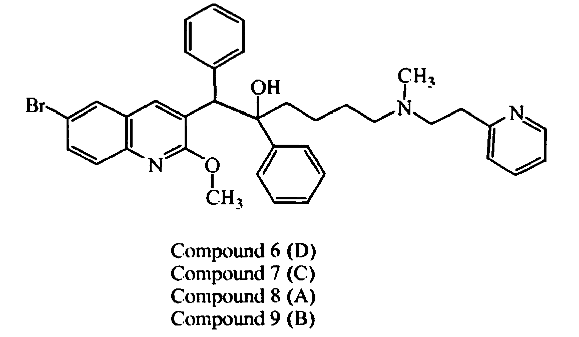

Lithium diisopropylamine ([4111-54-0]) (3.03 ml of a 2.0 M solution in THF/heptanes; 0.00607 mol) was dissolved in THF (20.24 ml; dry) and cooled to -70 °C. 6-Bromo-2-methoxy-3-(phenylmethyl)-quinoline (intermediate compound 3 (Ex. A3) of W02004/011436) (1.66 g, 0.00506 mol) was added dropwise as a solution in THF (15.18 ml; dry) and the mixture was stirred for 2 hours at -70 °C. Then **intermediate 10** (1.50 g, 0.00506 mol) was added dropwise as a solution in THF (15.18 ml; dry) and the reaction mixture was stirred for 3 hours at -70 °C. Then H₂O was added (quenching) at -70 °C, followed by EtOAc. The layers were separated and the organic layer was washed with brine, dried (MgSO₄), filtered and the solvent was evaporated to give a yellow oil. The residue was purified by flash chromatography. The desired fractions were collected and the solvent was evaporated. Yield: 0.41 g crude residue (mixture of diastereoisomers). Part of this mixture of diastereoisomers was separated into its enantiomers by supercritical fluid chromatography (SFC) over a Chiralpak AD-H column (20 x 250 mm) (eluent gradient: CO₂/(2-propanol with 0.1 % isopropylamine) from 90/10 to 60/40 in 18.75 minutes, 60/40 was hold for 4.5 minutes; flow 50 ml/min; column heater at 40 °C; nozzle pressure: 100 bar). The 4 product fractions were collected and the solvent was evaporated. Yield: 0.053 g of **compound 8** (A; 1^{st} fraction), 0.051 g of **compound 9** (B, 2^{nd} fraction), 0.077 g of **compound 7** (C, 3^{rd} fraction) and 0.082 g of **compound 6** (D, 4^{th} fraction).

### Example B5

### a. Preparation of compound 20, 21, 22 and 23

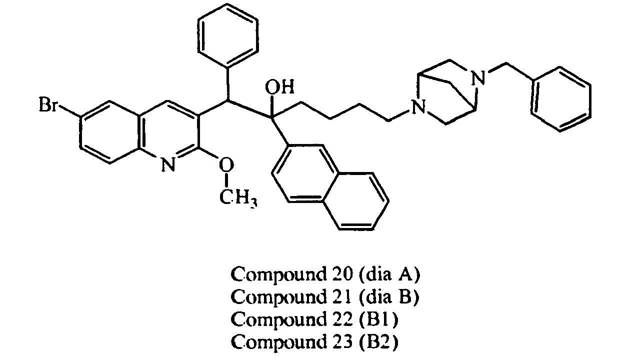

*n*BuLi (0.00465 mol, 2.9 ml of a 1.6 M solution in hexane) was added dropwise at -20 °C to a solution of diisopropylamine (0.00465 mol) in THF (10 ml) under N₂ flow. The mixture was stirred at -20 °C for 20 minutes, then cooled to -70 °C. A solution of 6-bromo-2-methoxy-3-(phenylmethyl)-quinoline (intermediate compound 3 (Ex. A3) of W02004/011436) (0.00388 mol) in THF (13 ml) was added. The mixture was stirred at -70 °C for 1 hour. A solution of **intermediate 21** (0.00427 mol) in THF (17 ml) was added. The mixture was stirred at -70 °C for 2 hours, then water was added. The mixture was extracted with EtOAc. The organic layer was separated, washed with brine, dried over MgSO₄, filtered and the solvent was evaporated. The residue (3.3 g) was purified by column chromatography over silica gel (eluent: CH₂Cl₂/CH₃OH/NH₄OH 96/4/0.1; 15-40 µm). Two fractions were collected and the solvent was evaporated. Yield: 0.45 g of **compound 20** (dia A, 16 %, fraction 1) and 0.7 g of **compound 21** (dia B, 25 %, fraction 2). To obtain the corresponding enantiomers, diastereoisomer B was purified by chiral chromatography (Super Critical Fluid chromatography) over silica gel (chiralpack AD-H, CO₂/MeOH: 65/35). Two fractions were collected and the solvent was evaporated. The residue of fraction 1 was crystallized from DIPE. The precipitate was filtered off and dried. Yield: 0.13 g of **compound 22** (5 %, enantiomer B1, m.p.: 171 °C). The residue of fraction 2 was crystallized from DIPE. The precipitate was filtered off and dried. Yield: 0.08 g of **compound 23** (3 %, enantiomer B2, m.p.: 156 °C).

### b. Preparation of compound 18 and 19

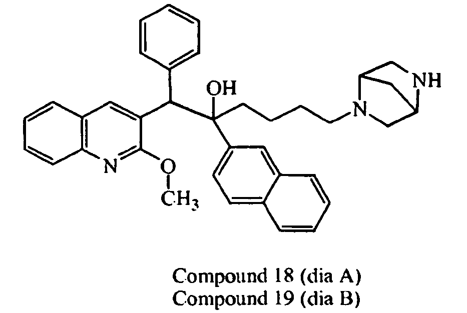

A mixture of **compound 20** (0.0006 mol), ammonium formate (0.0031 mol) and Pd/C 10 % (0.45 g) in CH₃OH (10 ml) was stirred and refluxed for 2 hours, then cooled to room temperature and filtered over Celite. The Celite was washed with CH₂Cl₂. H₂O was added. The organic layer was washed with H₂O and with saturated aqueous NaCl solution, dried (MgSO₄), filtered and the solvent was evaporated. The residue (0.16 g) was purified by column chromatography over Kromasil (eluent: CH₂Cl₂/CH₃OH/NH₄OH 90/10/1 to 78/22/2.2; 5 µm). The pure fractions were collected and the solvent was evaporated. Yield: 0.085 g of **compound 18** (25 %, dia A).

### Compound 19 was prepared according to the procedure of compound 18 (B5.b), but starting from intermediate 21. Yield: 53 % (dia B)

### c. Preparation of compound 24 and 25

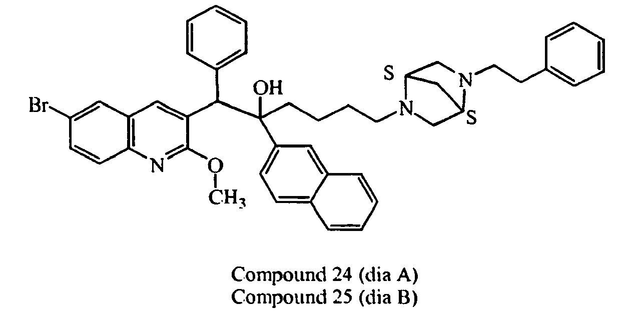

*N*-(1-Methylethyl)-2-propanamine lithium salt (1.26 ml of a 2.0 M solution in THF/heptane; 0.0025 mol) was dissolved in THF (8 ml; dry) and cooled to -70 °C. 6-Bromo-2-methoxy-3-(phenylmethyl)-quinoline (intermediate compound 3 (Ex. A3) of W02004/011436) (0.636 g, 0.0019 mol) was added dropwise as a solution in THF (6 ml; dry) and the mixture was stirred for 2 hours at -70 °C. Then **intermediate 24** (0.8 g, 0.0019 mol) was added dropwise as a solution in THF (6 ml) and the reaction mixture was stirred for 3 hours at -70 °C. Then H₂O was added (quenching) at -70 °C, followed by EtOAc. The layers were separated and the organic layer was washed with brine (2x10 ml), dried (MgSO₄), filtered and the solvent was evaporated. The residue was purified by flash chromatography (eluent: CH₂Cl₂/CH₃OH from 50/1 to 40/1 to 30/1 to 20/1 to 10/1). The desired fractions were collected and the solvent was evaporated. The residue was dried (vacuum, room temperature). Yield: 0.244 g of a pale green product (17 %). The product was crystallized from diethyl ether and dried (vacuum, room temperature). Yield: 0.142 g of the mixture of diastereoisomers (10 %). This product was separated by column chromatography (normal phase, Kromasil Si 10 µm, eluent CH₂Cl₂/MeOH/NH₄OH : 95/5/0.5). Fractions were crystallized from DIPE. Yield : 0.030g of **Compound 24** (dia A, foam, 2.1%) and 0.055g of **compound 25** (dia B, mp=137°C, 3.9%).

### d. Preparation of compound 26 and 27

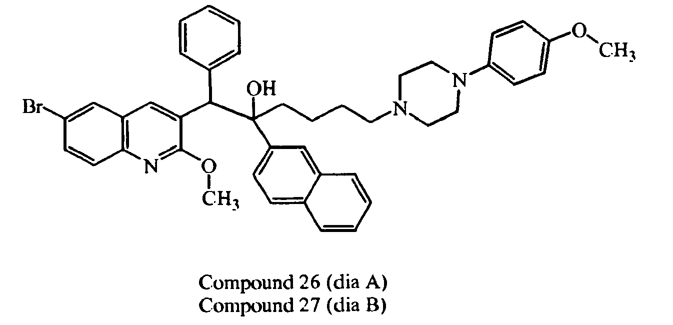

Lithium diisopropylamine (0.74 ml of a 2.0 M solution in THF/heptane; 0.00149 mol) was dissolved in THF (4.97 ml; dry) and cooled with an ice-bath at -70 °C. 6-Bromo-2-methoxy-3-(phenylmethyl)-quinoline (intermediate compound 3 (Ex. A3) of WO2004/011436) (0.41 g, 0.00124 mol) was added dropwise as a solution in THF (3.72 ml; dry) and the mixture was stirred for 2 hours at -70 °C. Then intermediate **25** (0.50 g, 0.00124 mol) was added dropwise as a solution in THF (3.72 ml; dry) and the reaction mixture was stirred for 3 hours at -70 °C. Then H₂O was added (quenching) at -70 °C, followed by EtOAc. The layers were separated and the organic layer was washed with brine, dried (MgSO₄), filtered and the solvent was evaporated to give a yellow oil. The residue was purified by flash chromatography (eluent: *n*-hexane/EtOAc). The desired fractions were collected and the solvent was evaporated. Yield: 0.081 g of **compound 26** (dia A) and 0.040 g of **compound 27** (dia B).

### e. Preparation of compound 28 and 29

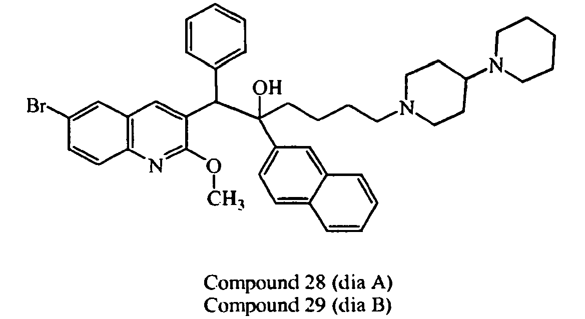

nBuLi (0.0024 mol, 1.53 ml of a 1.6 M solution in hexane) was added dropwise at -20 °C to a solution of diisopropylamine (0.0024 mol) in THF (5 ml) under N₂ flow. The mixture was stirred at -20 °C for 20 minutes, then cooled to -70 °C. A solution of 6-bromo-2-methoxy-3-(phenylmethyl)-quinoline (intermediate compound 3 (Ex. A3) of WO2004/011436) (0.002 mol) in THF (7 ml) was added. The mixture was stirred at -70 °C for 1 hour. A solution of **intermediate 26** (0.0022 mol) in THF (9 ml) was added at -70 °C. The mixture was stirred at -70 °C for 1 hour. H₂O was added. The mixture was extracted with EtOAc. The organic layer was washed with saturated aqueous NaCl solution, dried (MgSO₄), filtered and the solvent was evaporated. The residue was purified by column chromatography over Kromasil (eluent: CH₂Cl₂/CH₃OH/NH₄OH 92/8/0.5; 15-40 µm). Two fractions were collected and the solvent was evaporated. Yield: 0.25 g of **compound 28** (17 %; dia A) and 0.27 g of **compound 29** (19 %; dia B) (m.p.: 177 °C).

### f. Preparation of compound 52 and 53

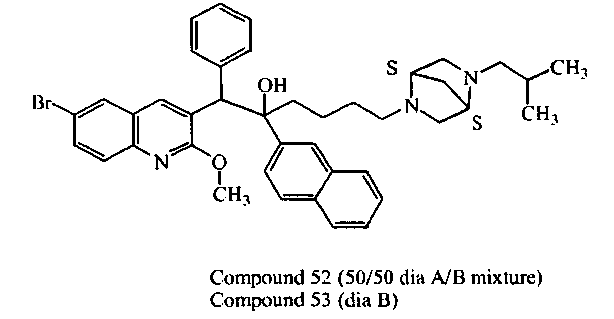

Lithium diisopropylamine (1.9 ml of a 2.0 M solution in THF/heptanes; 0.0028mol) was slowly added to a solution of 6-bromo-2-methoxy-3-(phenylmethyl)-quinoline (intermediate compound 3 (Ex. A3) of WO2004/011436) (0.94 g, 0.0028 mol) in THF (8 ml; dry) at -78 °C under Ar atmosphere. The mixture was stirred for 2 hours at -78 °C. Then **intermediate 44** (1.04 g, 0.0028 mol) was added dropwise as a solution in THF (8 ml; dry) and the reaction mixture was stirred for 3 hours at -70 °C. Then H₂O was added (quenching) at -70 °C, followed by EtOAc. The layers were separated and the organic layer was washed with brine and dried (MgSO₄), filtered and the solvent was evaporated. The residue was purified by flash chromatography over silica gel (CH₂Cl₂/McOH from 50/1 to 10/1). The desired fractions were collected and the solvent was evaporated and crystallized from DIPE. Yield: 0,24 g of a mixture of dia A and dia B. This mixture was further purified by column chromatography over Kromasil (CH₂Cl₂/MeOH/NH₄OH 96/4/0.4).The desired fractions were collected and the solvent was evaporated. Yield : 0.076 g of **compound 52** (50/50 dia A/B mixture; 3.8 %) and 0.0.023g of **compound 53** (dia B; 1.2 %).

### Example B6

### a. Preparation of compound 30

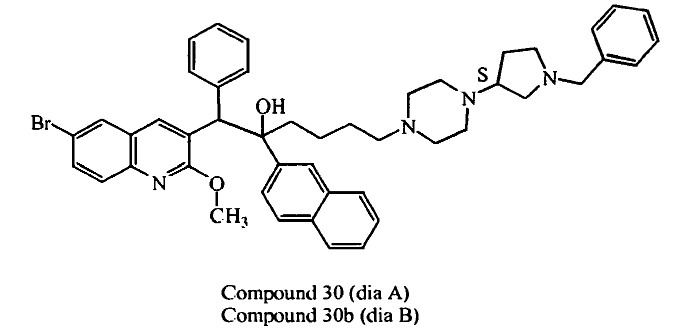

nBuLi (0.0019 mol, 1.2 ml of a 1.6 M solution in hexane) was added dropwise at -20 °C to a solution of diisopropylamine (0.0019 mol) in THF (4 ml) under N₂ flow. The mixture was stirred at -20 °C for 20 minutes, then cooled to -70 °C. A solution of 6-bromo-2-methoxy-3-(phenylmethyl)-quinoline (intermediate compound 3 (Ex. A3) of W02004/011436) (0.0016 mol) in THF (5 ml) was added. The mixture was stirred at -70 °C for 1 hour. A solution of **intermediate 32** (0.0017 mol) in THF (8 ml) was added. The mixture was stirred at -70 °C for 2 hours. H₂O was added at -20 °C. The mixture was extracted with EtOAc. The organic layer was washed with saturated aqueous NaCl solution, dried (MgSO₄), filtered and the solvent was evaporated. The residue was purified by column chromatography over silica gel (eluent: CH₂Cl₂/CH₃OH/NH₄OH 94/6/0.2 15-40 µm). Two fractions were collected and the solvent was evaporated. Yield fraction 1: 0.14 g of **compound 30** (11 %; dia A) and 0.2 g of the crude compound 30b. This crude compound 30b was crystallized from DIPE.

The precipitate was filtered off and dried. Yield: 0.12 g of **compound 30b** (10 %; m.p. 150 °C).

### b. Preparation of compound 31 and 32

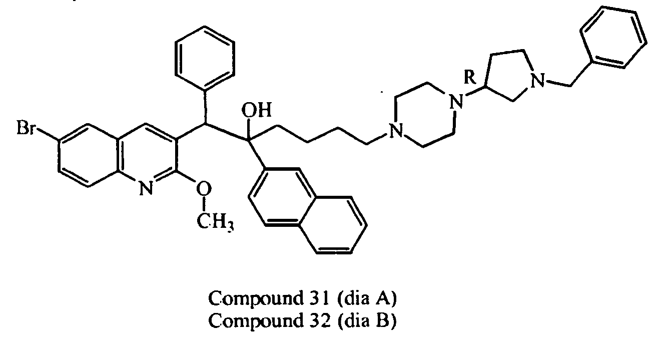

**Compounds 31** and **compound 32** were prepared according to the procedure for compound 30 (B6.a), but starting from **intermediate 31**. The work-up procedure yielded fraction 1: **Compound 31** (12 %). The second fraction was crystallized from DIPE. The precipitate was filtered off and dried. Yield: **Compound 32** (9 %; dia B; m.p.: 150°C).

### Example B7

### a. Preparation of compound 33

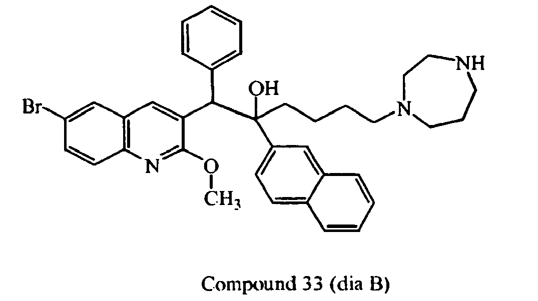

*n*BuLi (3.8 ml, 0.0061 mol) was added dropwise at -20°C to a solution of diisopropylamine (0.86 ml, 0.0061 mol) in THF (4 ml) under N₂ flow. The mixture was stirred at -20 °C for 20 minutes and then cooled to -70 °C. A solution of 6-bromo-2-methoxy-3-(phenylmethyl)-quinoline (intermediate compound 3 (Ex. A3) of WO2004/011436) (1 g, 0.00305 mol) in THF (5 ml) was added. The mixture was stirred at -70 °C for 1 hour and then a solution of **intermediate 33** (1.22 g, 0.00396 mol) in THF (5 ml) was added. The mixture was stirred at -70 °C for 90 minutes. Water was added and the mixture was extracted with EtOAc. The organic layer was separated, washed with brine, dried (MgSO₄), filtered and the solvent was evaporated. The residue (0.9 g) was purified by column chromatography over silica gel (eluent: CH₂Cl₂/CH₃OH/NH₄OH 93/7/0.1; 15-40 µm, 300 g). The pure fractions were collected and the solvent was evaporated. The residue was crystallized from DIPE. Yield: 0.242g of **compound 33** (dia B, 13 %, mp=119°C).

### b. Preparation of compounds 34 and 35

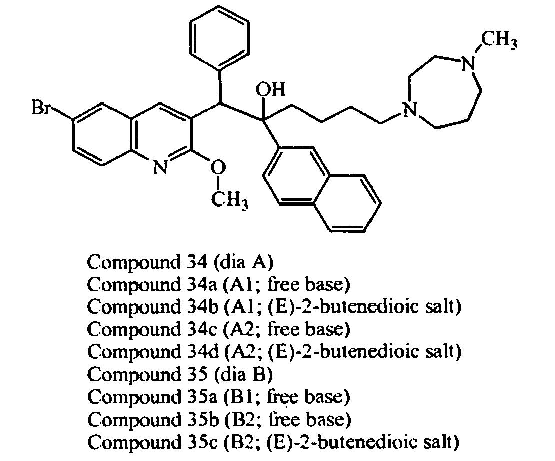

nBuLi (65.8 ml of a 1.6 M solution in hexanes, 0.105 mol) was added at -20 °C to a solution of diisopropylamine (14.7 ml, 0.105 mol) in THF (8 ml) under N₂ flow. The mixture was stirred at -20 °C for 30 minutes. A solution of 6-bromo-2-methoxy-3-(phenylmethyl)-quinoline [654655-69-3] (intermediate compound 3 of WO 2004/011436) (14 g, 0.042 mol) in THF (10 ml) was added dropwise at -70 °C. The mixture was stirred at -70 °C for 1 hour. A solution of **intermediate 34** (17.9 g, 0.0553 mol) in THF (10 ml) was added at -70 °C. The mixture was stirred at -70 °C for 1 hour, poured out into H₂O and extracted with EtOAc. The organic layer was washed with saturated aqueous NaCl solution, dried (MgSO₄), filtered and the solvent was evaporated. The residue (40 g) was purified by column chromatography over silica gel (CH₂Cl₂/CH₃OH/NH₄OH 94/5/1; 15-40 µm ). The desired fractions were collected and the solvent was evaporated. Yield: 5 g of **compound 34** (dia A; 17 %), 6.8 g of **compound 35** (dia B; 36 %). **Compound 34** was purified by SFC (Chiralpak AD-H :CO₂/EtOH/isopropylamine from 70/30/0.3 to 50/50/0.3). Two fractions were collected and the solvent was evaporated. Yielding: 1.45 g of **compound 34a** (fraction 1; free base; A1 enantiomer) and 1.5 g of **compound 34c** (fraction 2; free base; A2 enantiomer). **Compound 34a** was diluted with 2-propanone and converted into the (E)-2-butenedioic acid salt by addition of 1 eq of fumaric acid in EtOH/2-propanone. The precipitate was filtered off and dried. Yield: 0.802 g of **compound 34b** (fumaric acid salt of A1 enantiomer). **Compound 34c** was diluted with 2-propanone and converted into the (E)-2-butenedioic acid salt by addition of 1 eq of fumaric acid in EtOH/2-propanone. The precipitate was filtered off and dried. Yield: 0.822 g of **compound 34d** (fumaric acid salt of A2 enantiomer). **Compound 35** was also purified by SFC (Chiralpak AD-H :CO₂/CH₃OH/2-propanol/isopropylamine 70/15/15/0.3). Two fractions were collected and the solvent was evaporated. Yield: 1 g of **compound 35a** (free base; B1 enantiomer) and 1.3 g of **compound 35b** (free base; B2 enantiomer). **Compound 35b** was diluted in 2-propanone/ethanol and converted into the (E)-2-butenedioic acid salt by addition of 1 eq of fumaric acid in EtOH. The precipitate was filtered off and dried. Yielding: **Compound 35c** (fumaric acid salt of B2 enantiomer).

### c. Preparation of compound 45 and 50

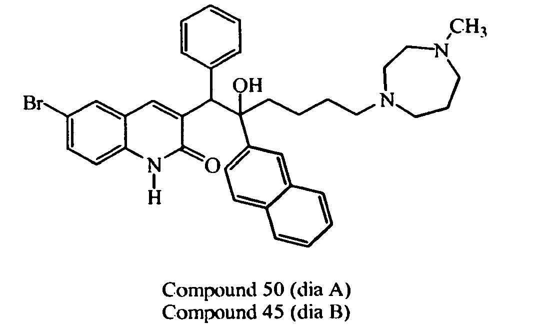

A mixture of **compound 35 and compound 34** (0.375 g; 0.0005 mol) in HCl (10 ml; 3 N solution) and dioxane (10 ml) was stirred for 2 hours at 60°C. Then the mixture was basified with K₂CO₃ (10 %) and extracted with CH₂Cl₂. The organic layer was washed with H₂O, dried (MgSO₄), filtered and the solvent was evaporated. The residue was purified by column chromatography over Kromasil (CH₂Cl₂/CH₃OH/NH₄OH 90/10/1; 10 µm). The desired fractions were collected and the solvent was evaporated. 0.074 g of the residue of the first fraction was crystallized from DIPE. The precipitate was filtered off and dried. Yield: 0.043 g of compound 50 (dia A; m.p.: 210 °C; 20 % yield). Fractions for the diastereoisomer B were collected and the solvent was evaporated. Yield: 0.035 g of **compound 45** (dia B, 10 %).

### Example B8

### a. Preparation of compounds 36 and 37

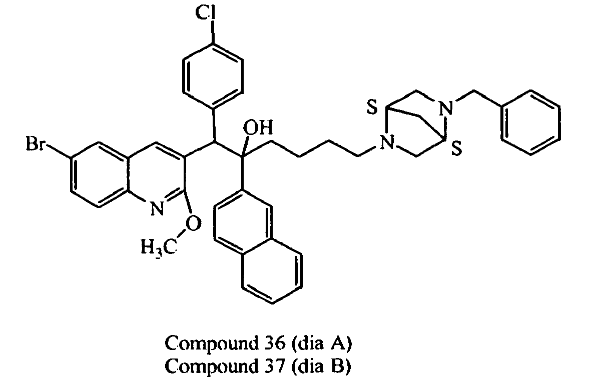

Lithium diisopropylamine (2.0 M solution in THF/heptane) (0.78 ml, 0.00156 mol) was dissolved in THF (5.22 ml; dry) and the solution was cooled down to -70 °C. **Intermediate 43** (6-bromo-3-[(4-chlorophenyl)methyl]-2-methoxyquinoline) (0.47 g, 0.00130 mol) was added dropwise as a solution in THF (3.91 ml; dry) and the mixture was stirred for 2 hours at -70 °C. Then **intermediate 35** (0.52 g, 0.00130 mol) was added dropwise as a solution in THF (3.91 ml; dry) and the reaction mixture was stirred for 3 hours at -70 °C. Then water was added to the mixture at -70 °C (quenching), followed by the addition of EtOAc. The layers were separated and the organic layer was washed with brine, dried (MgSO₄, anhydrous), filtered and the solvent was evaporated. The residue was purified by flash chromatography (eluent: DCM/MeOH 50/1). The desired fractions were collected and the solvent was evaporated, yielding 0.638 g of the mixture of dia A and dia B. This residue was further purified by supercritical fluid chromatography (SFC) (diphenyl varian; 20 x 150 mm) (eluent gradient: CO₂/(methanol with 0.5 % isopropylamine) 80/20). Finally, 0.097 g of **compound 36** (dia A) and 0.065 g of **compound 37** (dia B) was obtained.

### b. Preparation of compounds 48 and 49

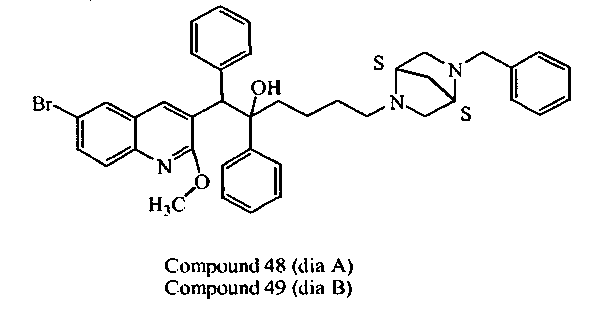

Lithium diisopropylamine (2.0 M solution in THF/heptane) (0.41 ml, 0.00082 mol) was dissolved in THF (2.75 ml; dry) and the solution was cooled down to -70 °C. Then 6-bromo-2-methoxy-3-(phenylmethyl)-quinoline [654655-69-3] (intermediate compound 3 of WO 2004/011436) (0.22 g, 0.00069 mol) was added dropwise as a solution in THF (2.07 ml; dry) and the reaction mixture was stirred for 2 hours at -70 °C. **Intermediate 36** (0.24 g, 0.00069 mol) was added dropwise as a solution in THF (2.07 ml) and the mixture was stirred for 3 hours at -70 °C. Then water was added to the mixture at -70 °C (quenching), followed by the addition of EtOAc. The layers were separated and the organic layer was washed with brine, dried (MgSO₄, anhydrous), filtered and the solvent was evaporated, yielding 0.243 g of the mixture of dia A and dia B. This residue was further purified by column chromatography over Kromasil Si 10µm (eluent: CH₂Cl₂/MeOH/NH₄OH 95/5/0.5). The desired fractions were collected and the solvent was evaporated. Yield: 0.042 g of **compound 48** (dia A) and 0.055 g of **compound 49** (dia B).

### Example B9

### a. Preparation of compounds 38, 39 and 40

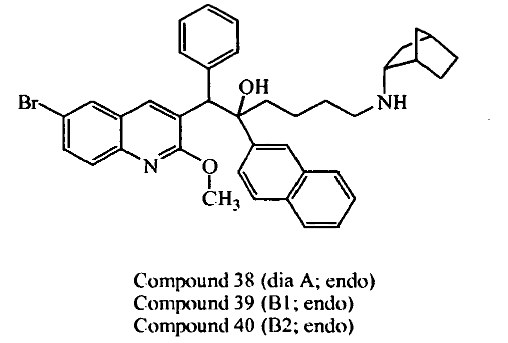

Lithium diisopropylamine (1.24 ml of a 2.0 M solution in THF/heptanes; 0.00249 mol) was slowly added to a solution of 6-bromo-2-methoxy-3-(phenylmethyl)-quinoline (intermediate compound 3 (Ex. A3) of WO2004/011436)(0.817 g, 0.00249 mol) in THF (13 ml; dry) at -78°C under Ar atmosphere. The mixture was stirred for 0.5 hours at -78 °C. Then **intermediate 38** (0.4 g, 0.00124 mol) was added dropwise as a solution in THF (5ml; dry) and the reaction mixture was stirred for 0.5 hours at -70 °C. Then H₂O was added (quenching) at -70 °C, followed by CH₂Cl₂. The layers were separated and the organic layer was dried (MgSO₄), filtered and the solvent was evaporated. The residue was purified by flash chromatography over silica gel (from petroleum ether/hexane/EtOAc 3.5/0.5/1 to petroleum ether/hexane/EtOAc/Et₃N 3.5/0.5/1/0.04). Different product fractions were collected and the solvent was evaporated. The 1^{st} fraction (0.056 g) was crystallized from petroleum ether to give 0.055 g of **compound 38** (dia A, endo; 6.9 %). The 2^{nd} fraction (0.080 g) was crystallized from Et₂O to give 0.021 g of **compound 39** (B1, endo, 2.5 %). The 3^{rd} fraction (0.120 g) was crystallized from Et₂O to give 0.028 g of **compound 40** (B2, endo, 3.4 %).

### Example B10

### a. Preparation of compounds 41 and 42

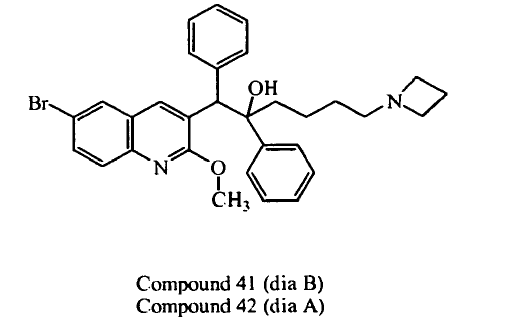

A mixture of **intermediate 39** (0.204 g; 0.00035 mol) and azetidine (0.12 ml; 0.0013 mol) was heated at 50 °C for 20 minutes. The crude product was purified by column chromatography over silica gel (Et₂O/petroleum ether/Et₃N 4/2/0.1) and was then further purified by column chromatography over Kromasil (CH₂Cl₂/MeOH/NH₄OH 95/5/0.5). The desired fractions were collected and the solvent was evaporated. Yield: 35 mg of **compound 42** (dia A; 18%) and 25 mg of **compound 41** (dia B; 13%).

### b. Preparation of compounds 65 and 66

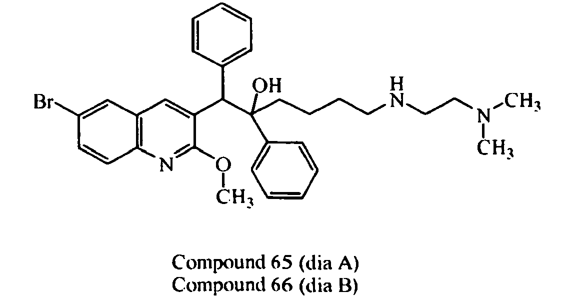

A mixture of **intermediate 39** (0.092 g, 0.00016 mol) and *N*,*N*-dimetyl-1,2-ethanediamine (0.3 ml, 0.00275 mol) was heated at 65 °C for 20 minutes. The crude product was dissolved in CH₂Cl₂, washed with H₂O and was then purified by column chromatography over silica gel (petroleum ether/EtOAc 4.5/0.5). Subsequently, the product was further purified by column chromatography over Kromasil (CH₂Cl₂/MeOH/NH₄OH 95/5/0.5). The desired fractions were collected and the solvent was evaporated. Yield: 21 mg of **compound 65** (dia A; 22 %) and 20 mg of **compound 66** (dia B; 22 %).

### c. Preparation of compound 60

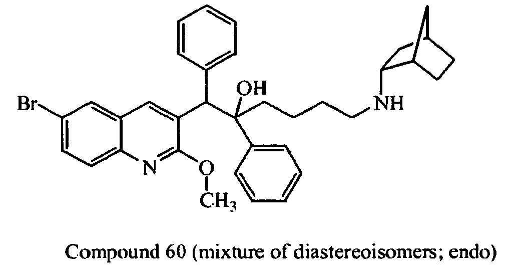

A mixture of **intermediate 39** (0.15 g, 0.00026 mol), 2-norbornanamine, hydrochloride (0.083 g, 0.00053 mol) and potassium carbonate (0.16 g, 0.00118 mol) in CH₃CN was refluxed for 4.5 hours. The reaction mixture was cooled to room temperature and the solid phase was filtered off and washed with CH₂Cl₂. The organic solution was evaporated till dryness. The crude product was purified by chromatography over silica gel (from petroleum ether/EtOAc 4.5/0.5 to petroleum ether/EtOAc/Et₃N 4.5/0.5/0.025). The desired fractions were collected and the solvent was evaporated. Yield: 22 mg of **compound 60** (mixture of diastereoisomers; endo).

### Example B11

### a. Preparation of compound 47

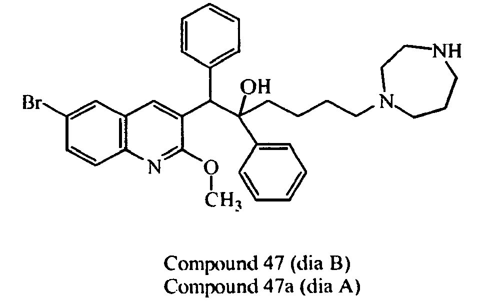

*n*BuLi (4.2 ml of a 1.6 M solution in hexanes; 0.0067 mol) was added at -20°C to a solution of diisopropylamine (0.94 ml, 0.0067 mol) in THF (6 ml) under N₂ flow. The mixture was stirred at -20°C for 30 minutes. A solution of 6-bromo-2-methoxy-3-(phenylmethyl)-quinoline (intermediate compound 3 (Ex. A3) of WO2004/011436) (1.1 g, 0.0033 mol) in THF (7 ml) was added dropwise at -70 °C. The mixture was stirred at -70 °C for 1 hour. A solution of intermediate 40 (0.0043 mol) in THF (7 ml) was added at -70 °C. The mixture was stirred at -70 °C for I hour, poured out into H₂O and extracted with EtOAc. The organic layer was washed with saturated aqueous NaCl solution, dried (MgSO₄), filtered and the solvent was evaporated. The residue was purified by column chromatograpy over Kromasil (eluent: CH₂Cl₂/CH₃OH/NH₄OH 98/2/0.1 to 95/5/0.1; 10µm) and then over Sunfire C-18 (5 µm; 19x150 mm; with a flow rate of 20 ml/min. Two mobile phases (mobile phase A: 100 % methanol; mobile phase B: 100 % 63mM ammonium hydrogen carbonate pH=8 (in ultra pure water). Two fractions were collected and the solvent was evaporated. Yielding: 0.022 g of compound 47a (fraction 1; dia A) and 0.18 g of compound 47 (fraction 2; dia B).

### Example B12

### a. Preparation of compound 58

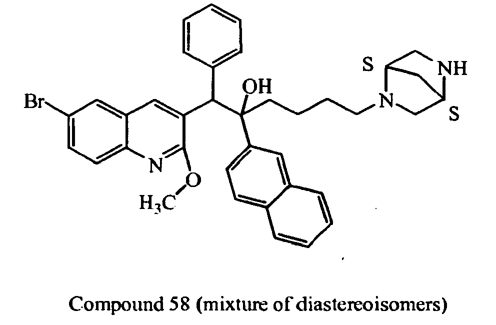

Lithium diisopropylamine (1.3 ml of a 2.0 M solution in THF/heptanes; 0.0026mol) was slowly added to a solution of 6-bromo-2-methoxy-3-(phenylmethyl)-quinoline (intermediate compound 3 (Ex. A3) of WO2004/011436) (0.67 g, 0.0020 mol) in THF (6 ml; dry) at -78°C under Ar atmosphere. The mixture was stirred for 2 hours at -78 °C. Then **intermediate 46** (0.63 g, 0.0020 mol) was added dropwise as a solution in THF (6 ml; dry) and the reaction mixture was stirred for 3 hours at -70 °C. Then H₂O was added (quenching) at -70 °C, followed by EtOAc. The layers were separated and the organic layer was wahed with brine and dried (MgSO₄), filtered and the solvent was evaporated. The residue was purified by flash chromatography over silica gel (CH₂Cl₂/MeOH from 50/1 to 4/1). The desired fractions were collected and the solvent was evaporated and crystallized in DIPE. Yield: 0.26 g of **compound 58** (a mixture of dia A and dia B, pale yellow solid).

### b. Preparation of compounds 61 and 62

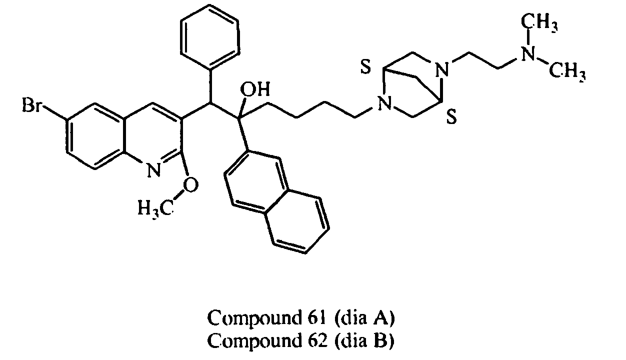

A mixture of **compound 58** (1.61 g, 0.0025 mol), 2-chloro-*N*,*N*-dimethyl-ethanamine hydrochloride (0.474 g, 0.0033 mol), Et₃N (1.05 ml, 0.0076 mol), KI (0.42 g, 0.0025 mol) and *N,N,N*-tributyl-1-butanaminium chloride (0.161 g) in CH₃CN (10 ml) was heated for 20 hours at 80 °C. Then the mixture was cooled to room temperature and the solvent was evaporated. The product was purified by flash column chromatography (eluent: CH₂Cl₂/CH₃OH from 20/1 till 4/1). Then the product was further purified over Kromasil (eluent: CH₂Cl₂/CH₃OH/NH₄OH 90/10/1) Two fractions were collected and the solvent was evaporated. Yield: 32 mg of **compound 61** (dia A; 1.8 %) and 33 mg of **compound 62** (dia B; 1.8 %).

### c. Preparation of compounds 56 and 57

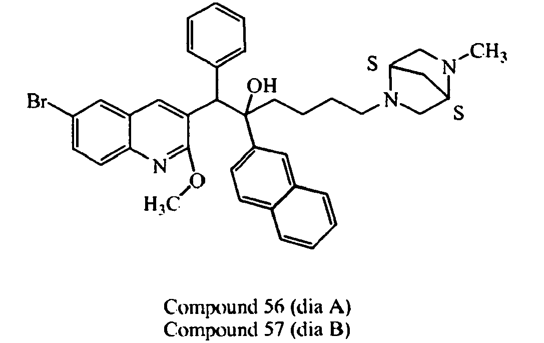

**Compound 58** (0.6 g, 0.0009 mol) and paraformaldehyde (0.057 g, 0.0019 mol) were dissolved in MeOH (10 ml). Then NaBH₃CN (0.09 g, 0.0014 mol) was added slowly portionwise. When the addition was completed, the reaction was stirred at room temperature for 48 hours. The solvent was evaporated and the product was purified by flash chromatography (CH₂Cl₂/CH₃OH from 50/1 to 4/1) and then over Kromasil (CH₂Cl₂/CH₃OH/NH₄OH 90/10/1). Two fractions were collected and the solvent was evaporated. Yield: 60 mg of **compound 56** (dia A, 9.8 %) and 53 mg of **compound 57** (dia B, 8.6 %).

### d. Preparation of compounds 63 and 64

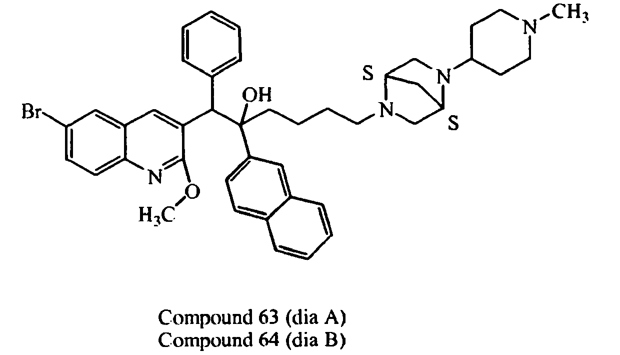

**Compound 58** (1.13 g, 0.0018 mol) and 1-methyl-4-piperidinone (0.41 ml, 0.0035 mol) were dissolved in MeOH (18 ml). Then NaBH₃CN (0.17 g, 0.0027 mol) was added slowly portionwise. When the addition was completed, the reaction was stirred overnight at room temperature. The solvent was evaporated and the product was purified by flash chromatography (CH₂Cl₂/CH₃OH from 20/1 to 4/1) and then over reversed phase (Xbridge^{™} C 18 column from Waters (5 µm; 19 x 150 mm) with a flow rate of 20 ml/min. Two mobile phases (mobile phase A: 100 % acetonitrile; mobile phase B: 100 % 63mM ammonium hydrogen carbonate pH=10.2 (in ultra pure water) were employed to run a gradient condition from 95 % A, 5 % B to 100 % A in 14 minutes, and reequilibrated with initial conditions for 6 minutes. Two fractions were collected and the solvent was evaporated. Yield: 34 mg of **compound 63** (dia A; 2.6 %) and 97 mg of **compound 64** (dia B; 7.4 %).

### Example B13

### a. Preparation of compound 51

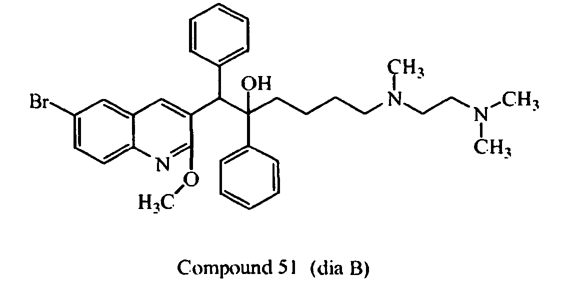

Lithium diisopropylamine (0.91 ml of a 2.0 M solution in THF/heptanes; 0.0018mol) was slowly added to a solution of 6-bromo-2-methoxy-3-(phenylmethyl)-quinoline (intermediate compound 3 (Ex. A3) of WO2004/011436) (0.5 g, 0.0015 mol) in THF (4.5 ml; dry) at -78°C under Ar atmosphere. The mixture was stirred for 2 hours at -78 °C. Then **intermediate 47** (0.40 g, 0.0015 mol) was added dropwise as a solution in THF (4.5 ml; dry) and the reaction mixture was stirred for 3 hours at -70 °C. Then H₂O was added (quenching) at -70 °C, followed by EtOAc. The layers were separated and the organic layer was washed with brine and dried (MgSO₄), filtered and the solvent was evaporated. The residue was first purified by flash chromatography over silica gel (CH₂Cl₂/MeOH: 50/1) and then over Kromasil (CH₂Cl₂/CH₃OH/NH₄OH 90/10/1). The desired fractions were collected and the solvent was evaporated. Yield of the desired fraction: 23 mg of **compound 51** (2.5 %; dia B).

### Example B14

### Preparation of compounds 54 and 55

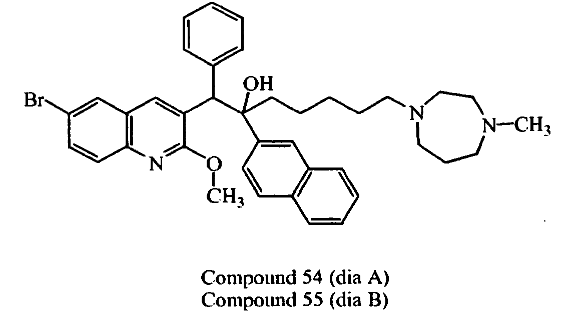

nBuLi (6.4 ml of a 1.6 M solution in hexanes; 0.01 mol) was added at -20°C to a solution of diisopropylamine (1.4 ml; 0.01 mol) in THF (10 ml) under N₂ flow. The mixture was stirred at -20 °C for 30 minutes. A solution of 6-bromo-2-methoxy-3-(phenylmethyl)-quinoline (intermediate compound 3 (Ex. A3) of WO2004/011436) (1.3 g, 0.0041 mol) in THF (10 ml) was added dropwise at -70 °C. The mixture was stirred at -70 °C for 1 hour. A solution of **intermediate 50** (1.8 g, 0.0054 mol) in THF (10 ml) was added at -70 °C. The mixture was stirred at -70 °C for 1 hour, poured out into H₂O and extracted with EtOAc. The organic layer was washed with brine, dried (MgSO₄), filtered and the solvent was evaporated. The residue was purified by column chromatography over silica gel (CH₂Cl₂/CH₃OH 94/6; 15-40µm). Then, purification was carried out on a Sunfire^{™} C18 column from Waters (5 µm19 x 150 mm) with a flow rate of 20 ml/min. Two mobile phases (mobile phase A: 100 % acetonitrile; mobile phase B: 100 % 63mM ammonium hydrogen carbonate pH=8 (in ultra pure water) were employed to run a gradient condition from 90 % A, 10 % B to 100 % A in 14 minutes, and reequilibrated with initial conditions for 6 minutes. Two fractions were collected and the solvent was evaporated till dryness. Then 0.47 g of residue from fraction 1 was crystallized from 2-propanone. The precipitate was filtered off and dried to yield 0.34 g of **compound 54** (dia A; 13 %). Also the residue from fraction 2 (0.41 g) was crystallized from DIPE. The precipitate was filtered off and dried. Yield: 0.199 g of **compound 55** (dia B; 7 %).

### Example B 15

### Preparation of compounds 46 and 59

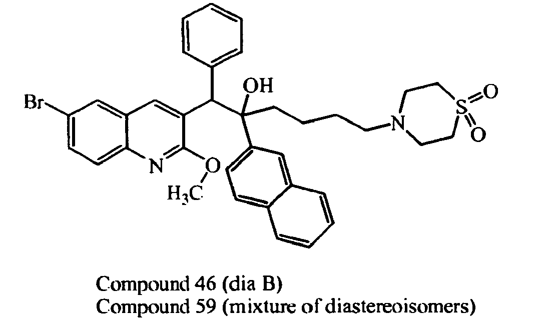

At 0 °C, 3-chloro-benzenecarboperoxoic acid (0.54 g, 3.11 mmol) was added to **intermediate 52** (0.5 g, 0.78 mmol) in CH₂Cl₂ (q.s.). The mixture was stirred at 0 °C for 30 minutes and then overnight at room temperature. The mixture was diluted with H₂O (q.s.). The separated organic layer was dried over MgSO₄, filtered and the solvent was evaporated. The crude product was purified by chromatography over silica gel (15-40 µm, 30 g; eluent: CH₂Cl₂/MeOH/NH₄OH from 95/5/0.5 to 93/7/0.7 to 88/12/1). Two fractions were collected and the solvent was evaporated. Yield: 340 mg of a first fraction (m.p.: 170 °C, dia B; the N-Oxide of the target compound) and 77 mg of a second fraction which was crystallized from Et₂O to give 51 mg of **compound 46** (10 %; m.p.: 172 °C; dia B).

### Compound 59 was prepared according to the following procedure:

A mixture of intermediate 48 (2.1mmol) and thiomorpholine-1,1-dioxyde (10.5mmol) was warmed at 85°C-90°C for 1hour followed by work-up. The crude product was purified by flash chromatography over silica gel (15-40µm) from petroleum ether/Et₂O 4/1 to petroleum ether/Et₂O/Et₃N 1.3/3.7/0.05. After evaporation **compound 59** was obtained Yield: 0.101g (77%).

### Example B16

### Preparation of compounds 43 and 44

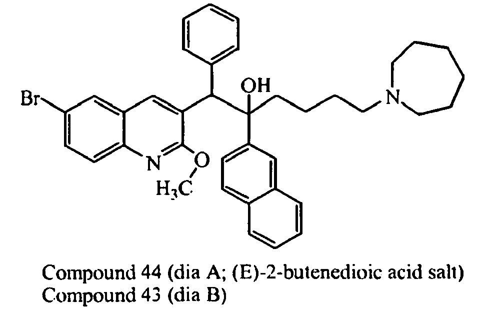

nBuLi (0.0060 mol; 3.8 ml of a 1.6 M solution in hexane) was added at -20 °C to a solution of diisopropylamine (0.15 ml, 0.0060 mol) in THF (8 ml; anhydrous) under N₂ flow. The mixture was stirred for 30 minutes at -20°C. Then a solution of 6-bromo-2-methoxy-3-(phenylmethyl)-quinoline [654655-69-3] (intermediate compound 3 of WO 2004/011436) (1 g, 0.0030 mol) in THF (10 ml, anhydrous) was added at -70 °C. The mixture was stirred at -70 °C for 1 hour. Then a solution of **intermediate 53** (1.21 g, 0.0040 mol) in THF (12 ml, anhydrous) was added at -70 °C. The mixture was stirred for 1 hour at -70 °C, poured out into H₂O and was extracted with EtOAc. The organic layer was washed with a saturated aqueous NaCl solution, dried (MgSO₄), filtered and the solvent was evaporated. The residue was purified by column chromatography over Kromasil (eluent: CH₂Cl₂/CH₃OH/NH₄OH from 98/2/0.1 to 95/5/0.1; 10 µm). Crude compound 44 (0.088 g) was eluted first from the column, and crude compound 43 (0.177 g) was eluted second from the column. The crude compound 43 was crystallized from DIPE. The precipitate was filtered off and dried. Yielding: 0.107 g of **compound 43** (m.p.: 167 °C, dia B). The crude compound 44 was dissolved in 2-propanone and was converted into the (E)-2-butenedioic acid salt (in EtOH/2-propanone and addition of fumaric acid). The precipitate was filtered off and dried. This fraction was washed with 2-propanone. The filtrate was evaporated. Yield: 0.036 g of **compound 44** (m.p.: 155 °C, dia A; (E)-2-butenedioic acid salt).

Tables 1a,1b, 1c and 1d list compounds of formula (1a) according to the present invention prepared according to one of the above Example No. (Ex.No.).

For a number of compounds, melting points were obtained with a Kofler hot bench, consisting of a heated plate with linear temperature gradient, a sliding pointer and a temperature scale in degrees Celsius.

**Table 1a :**

| | | | | | |
|---|---|---|---|---|---|
| 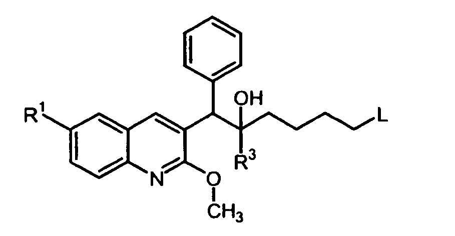 | | | | | |

| Comp. No. | Ex. No. | R¹ | R³ | L | Stereochemistry and physico-chemical data |
|---|---|---|---|---|---|
| 1 | B1 | --Br | 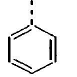 | 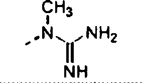 | dia A; 173 °C |
| 2 | B1 | --Br | 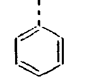 | 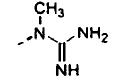 | dia B; 179 °C |
| 51 | B13.a | --Br | 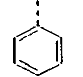 | 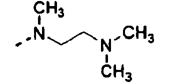 | dia B |
| 65 | B10.b | --Br | 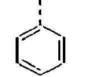 | 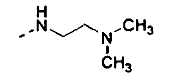 | dia A |
| 66 | B10.b | --Br | 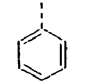 | 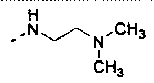 | dia B |
| 41 | B10.a | --Br | 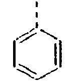 | 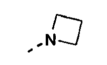 | dia B |
| 42 | B10.a | --Br | 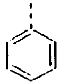 | 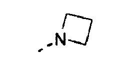 | dia A |
| 3 | B2 | --Br | 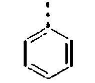 | 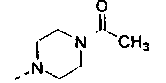 | dia A |
| 4 | B3.a | --Br | 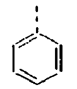 | 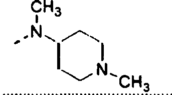 | dia A |
| 5 | B3.a | --Br | 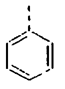 | 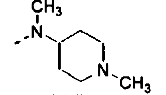 | dia B |
| 6 | B4 | --Br | 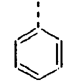 | 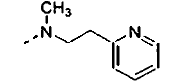 | D |
| 7 | B4 | --Br | 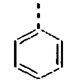 | 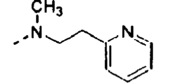 | C |
| 8 | B4 | --Br | 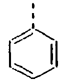 | 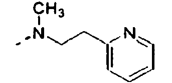 | A |
| 9 | B4 | --Br | 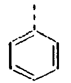 | 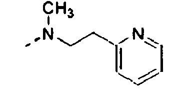 | B |
| 47 | B11.a | --Br | 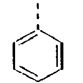 | 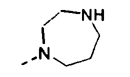 | dia B |
| 47a | B11.a | --Br | 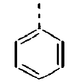 | 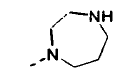 | dia A |
| 48 | B8.b | --Br | 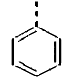 | 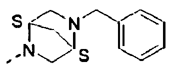 | dia A |
| 49 | B8.b | --Br | 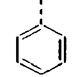 | 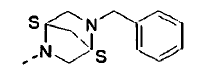 | dia B |
| 60 | B10.c | --Br | 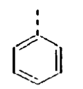 | 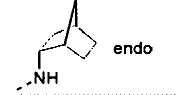 | mixture of diastereoisomers; endo |
| 10 | B3.b | --Br | 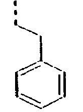 | 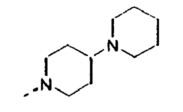 | dia A |
| 11 | B3.b | --Br | 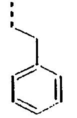 | 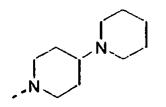 | dia B; 164 °C |
| 12 | B3.c | --Br | 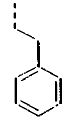 | 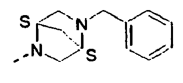 | dia A |
| 13 | B3.c | --Br | 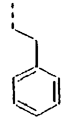 | 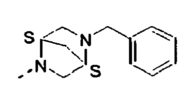 | dia B |
| 14 | B3.d | --Br | 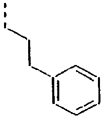 | 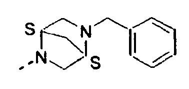 | dia A |
| 15 | B3.d | --Br | 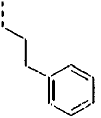 | 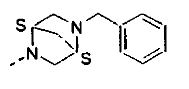 | dia B |
| 16 | B3.e | --Br | 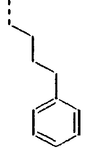 | 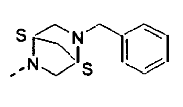 | dia A |
| 17 | B3.e | --Br | 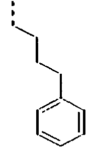 | 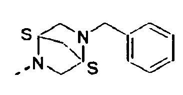 | dia B |
| 59 | B15 | --Br | 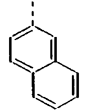 | 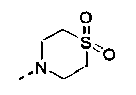 | mixture of diastereoisomers; 127 °C |
| 46 | B15 | --Br | 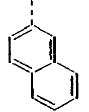 | 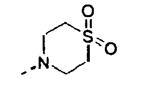 | dia B; 172 °C |
| 18 | B5.b | --H | 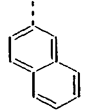 | 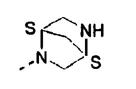 | dia A |
| 19 | B5.b | --H | 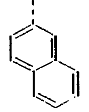 | 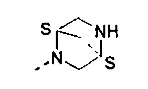 | dia B |
| 58 | B12.a | --Br | 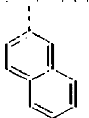 | 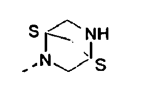 | mixture of diastereoisomers |
| 56 | B12.c | --Br | 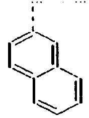 | 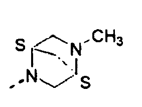 | dia A |
| 57 | B12.c | --Br | 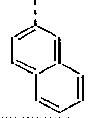 | 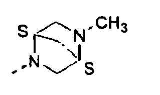 | dia B |
| 52 | B5.f | --Br | 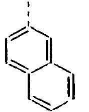 | 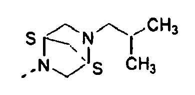 | mixture of diastereoisomers |
| 53 | B5.f | --Br | 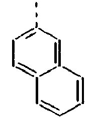 | 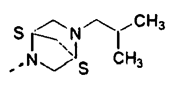 | dia B |
| 61 | B12.b | --Br | 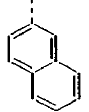 | 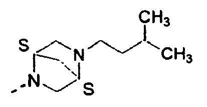 | dia A |
| 62 | B12.b | --Br | 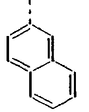 | 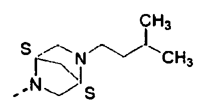 | dia B |
| 63 | B12.d | --Br | 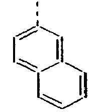 | 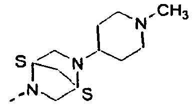 | dia A |
| 64 | B12.d | --Br | 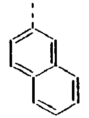 | 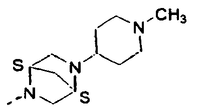 | dia B |
| 20 | B5.a | --Br | 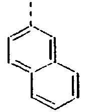 | 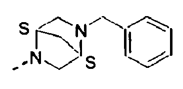 | dia A |
| 21 | B5.a | --Br | 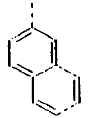 | 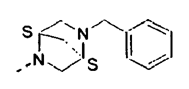 | dia B |
| 22 | B5.a | --Br | 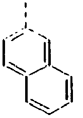 | 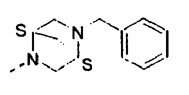 | B1; 171 °C |
| 23 | B5.a | --Br | 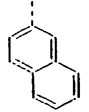 | 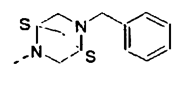 | B2; 156 °C |
| 24 | B5.c | --Br | 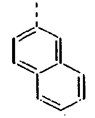 | 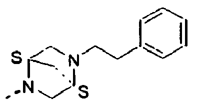 | dia A |
| 25 | B5.c | --Br | 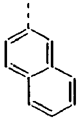 | 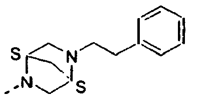 | dia B; 137 °C |
| 26 | B5.d | --Br | 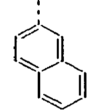 | 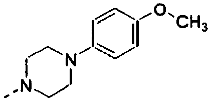 | dia A |
| 27 | B5.d | --Br | 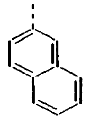 | 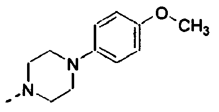 | dia B |
| 28 | B5.e | --Br | 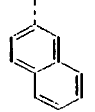 | 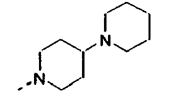 | dia A |
| 29 | B5.e | --Br | 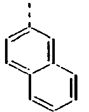 | 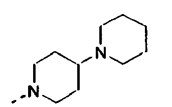 | dia B; 177 °C |
| 30 | B6.a | --Br | 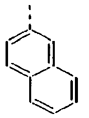 | 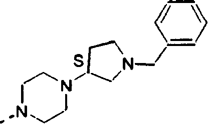 | dia A |
| 30b | B6.a | --Br | 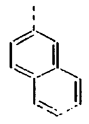 | 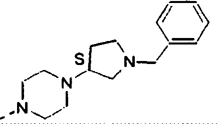 | dia B; 150 °C |
| 31 | B6.b | --Br | 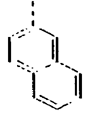 | 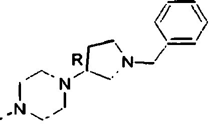 | dia A |
| 32 | B6.b | --Br | 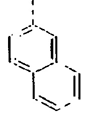 | 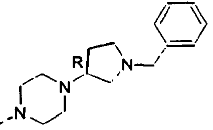 | dia B; 150 °C |
| 33 | B7.a | --Br | 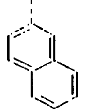 | 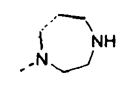 | dia B; 189 °C |
| 43 | B16 | --Br | 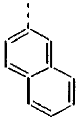 | 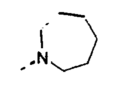 | dia B; 167 °C |
| 44 | B16 | --Br | 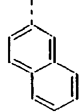 | 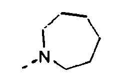 | dia A; fumarate salt; 155 °C |
| 38 | B9.a | --Br | 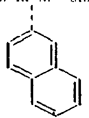 | 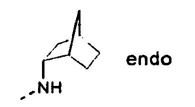 | dia A; endo; 153 °C |
| 39 | B9.a | --Br | 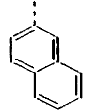 | 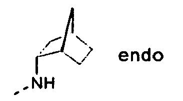 | B1; endo |
| 40 | B9.a | --Br | 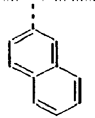 | 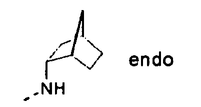 | B2; endo |
| 34 | B7.b | --Br | 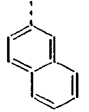 | 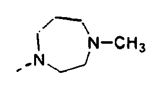 | dia A; 141 °C |
| 34a | B7.b | --Br | 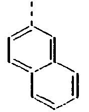 | 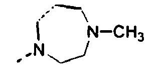 | A1 |
| 34b | B7.b | --Br | 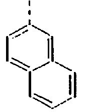 | 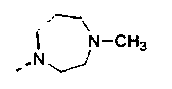 | A1; fumarate salt; 132 °C |
| 34c | B7.b | --Br | 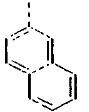 | 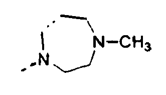 | A2 |
| 34d | B7.b | --Br | 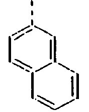 | 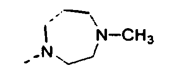 | A2; fumarate salt; 111 °C |
| 35 | B7.b | --Br | 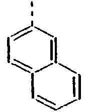 | 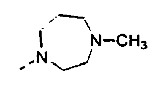 | dia B |
| 35a | B7.b | --Br | 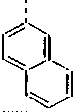 | 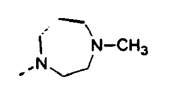 | B1 |
| 35b | B7.b | --Br | 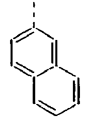 | 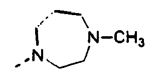 | B2 |
| 35c | B7.b | --Br | 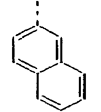 | 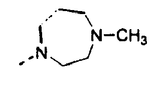 | B2; fumarate salt; 113 °C |

**Table 1b :**

| | | | | | | |
|---|---|---|---|---|---|---|
| 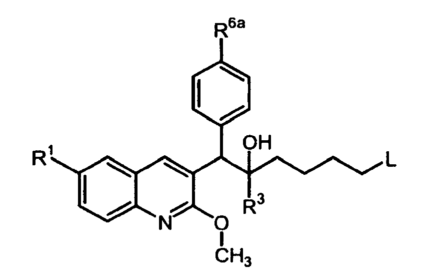 | | | | | | |

| Comp. Ex. No. | No. | R¹ | R³ | R^{6a} | L | Stereochemistry and physico-chemical data |
|---|---|---|---|---|---|---|
| 36 | B8.a | --Br | 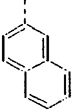 | --Cl | 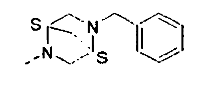 | dia A |
| 37 | B8.a | --Br | 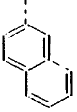 | --Cl | 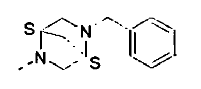 | dia B |

**Table 1c :**

| | | | | |
|---|---|---|---|---|
| 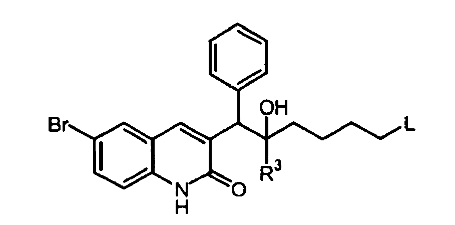 | | | | |

| Comp. No. | Ex. No. | R³ | L | Stereochemistry and physico-chemical data |
|---|---|---|---|---|
| 50 | B7.c | 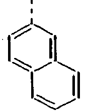 | 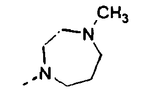 | dia A; 210 °C |
| 45 | B7.c | 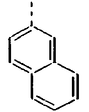 | 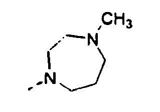 | dia B |

**Table 1d :**

| | | | |
|---|---|---|---|
| 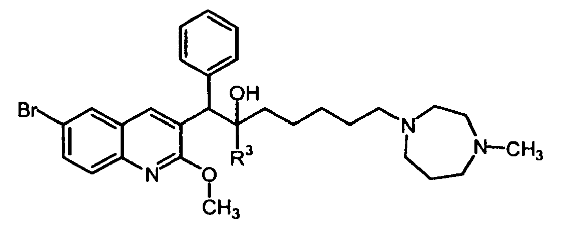 | | | |

| Comp. No. | Ex. No. | R³ | Stereochemistry and physico-chemical data |
|---|---|---|---|
| 54 | B14 | 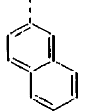 | dia A; 111 °C |
| 55 | B14 | 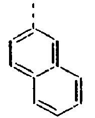 | dia B; 148 °C |

### C. Analytical methods

### LCMS

The mass of some compounds was recorded with LCMS (liquid chromatography mass spectrometry). The methods used are described below.

### General procedure A

The HPLC measurement was performed using an Alliance HT 2795 (Waters) system comprising a quaternary pump with degasser, an autosampler, a diode-array detector (DAD) and a column as specified in the respective methods below, the column is hold at a temperature of 30°C. Flow from the column was split to a MS spectrometer. The MS detector was configured with an electrospray ionization source. The capillary needle voltage was 3 kV and the source temperature was maintained at 100 °C on the LCT (Time of Flight Zspray™ mass spectrometer from Waters - for methods 1,3 and 8), and 3.15 kV at 110 °C on the ZQ™ (simple quadrupole Zspray™ mass spectrometer from Waters - for methods 2, 4 and 5). Nitrogen was used as the nebulizer gas. Data acquisition was performed with a Waters-Micromass MassLynx-Openlynx data system.

### General procedure B

The HPLC measurement was performed using an Agilent 1100 series liquid chromatography system comprising a binary pump with degasser, an autosampler, a column oven, a UV detector and a column as specified in the respective methods below. Flow from the column was split to a MS spectrometer. The MS detector was configured with an electrospray ionization source. The capillary voltage was 3 kV, the quadrupole temperature was maintained at 100 °C and the desolvation temperature was 300 °C. Nitrogen was used as the nebulizer gas. Data acquisition was performed with an Agilent Chemstation data system.

### General procedure C

The LC measurement was performed using a UPLC (Ultra Performance Liquid Chromatography) Acquity (Waters) system comprising a binary pump with degasser, an autosampler, a diode-array detector (DAD) and a column as specified in the respective methods below, the column is hold at a temperature of 40°C. Flow from the column was brought to a MS detector. The MS detector was configured with an electrospray ionization source. The capillary needle voltage was 3 kV and the source temperature was maintained at 130 °C on the Quattro (triple quadrupole mass spectrometer from Waters). Nitrogen was used as the nebulizer gas. Data acquisition was performed with a Waters-Micromass MassLynx-Openlynx data system.

### Method 1

In addition to general procedure A: Reversed phase HPLC was carried out on a Kromasil C18 column (5 µm, 4.6 x 150 mm) with a flow rate of 1.0 ml/min. Three mobile phases (mobile phase A: 100 % 7 mM ammonium acetate; mobile phase B: 100 % acetonitrile; mobile phase C: 0.2 % formic acid + 99.8 % ultra-pure Water) were employed to run a gradient condition from 30 % A , 40 % B and 30 % C (hold for 1 minute) to 100 % B in 4 minutes, 100 % B for 5 minutes and reequilibrated with initial conditions for 3 minutes. An injection volume of 5 µl was used. Cone voltage was 20 V for positive ionization mode. Mass spectra were acquired by scanning from 100 to 900 in 0.8 seconds using an interscan delay of 0.08 seconds.

### Method 2

In addition to general procedure A: Reversed phase HPLC was carried out on a Sunfire C18 column (3.5 µm, 4.6 x 100 mm) with an intial flow rate of 0.8 ml/min. Two mobile phases (mobile phase A: 25 % 7mM ammonium acetate + 50 % acetonitrile +25 % formic acid (2ml/l); mobile phase B: 100 % acetonitrile) were employed to run a gradient condition from 100 % A (hold for 1 minute) to 100 % B in 4 minutes, hold at 100 % B at a flow rate of 1.2 ml/min for 4 minutes and reequilibrated with initial conditions for 3 minutes). An injection volume of 10 µl was used. Cone voltage was 20 V for positive and negative ionization mode. Mass spectra were acquired by scanning from 100 to 1000 in 0.4 seconds using an interscan delay of 0.3 seconds.

### Method 3

In addition to general procedure A: Reversed phase HPLC was carried out on a Xterra-MS C18 column (5 µm, 4.6 x 150 mm) with a flow rate of 1.0 ml/min. Two mobile phases (mobile phase A: 100 % 7 mM ammonium acetate; mobile phase B: 100 % acetonitrile; were employed to run a gradient condition from 85 % A , 15 % B (hold for 3 minutes) to 20 % A, 80 % B in 5 minutes, hold at 20 % A and 80 % B for 6 minutes and reequilibrated with initial conditions for 3 minutes. An injection volume of 20 µl was used. Cone voltage was 20 V for positive ionization mode and 20 V for negative ionization mode. Mass spectra were acquired by scanning from 100 to 900 in 0.8 seconds using an interscan delay of 0.08 seconds.

### Method 4

In addition to general procedure A: Reversed phase HPLC was carried out on a Sunfire C18 column (3.5 µm, 4.6 x 100 mm) with an initial flow rate of 0.8 ml/min. Two mobile phases (mobile phase A: 35 % 6.5mM ammonium acetate + 30 % acetonitrile + 35 % formic acid (2 ml/l); mobile phase B: 100 % acetonitrile) were employed to run a gradient condition from 100 % A (hold for 1 minute) to 100% B in 4 minutes, hold at 100 % B at a flow rate of 1.2 ml/min for 4 minutes and reequilibrated with initial conditions for 3 minutes. An injection volume of 10 µl was used. Cone voltage was 20 V for positive and negative ionization mode. Mass spectra were acquired by scanning from 100 to 1000 in 0.4 seconds using an interscan delay of 0.3 seconds.

### Method 5

In addition to general procedure A: Reversed phase HPLC was carried out on a Sunfire C18 column (3.5 µm, 4.6 x 100 mm) with an initial flow rate of 0.8 ml/min. Two mobile phases (mobile phase A: 35 % 6.5mM ammonium acetate + 30 % acetonitrile + 35 % formic acid (2 ml/l); mobile phase B: 100 % acetonitrile) were employed to run a gradient condition from 100 % A (hold for 1 minute) to 100% B in 4 minutes, hold at 100 % B at a flow rate of 1.2 ml/min for 4 minutes and reequilibrated with initial conditions for 3 minutes. An injection volume of 10 µl was used. Positive ionization mode was used with four different cone voltages (20,40,50,55 V). Mass spectra were acquired by scanning from 100 to 1000 in 0.4 seconds using an interscan delay of 0.1 seconds.

### Method 6

In addition to general procedure A: Reversed phase HPLC was carried out on a Kromasil C18 column (5 µm, 4.6 x 150 mm) with a flow rate of 1.0 ml/min. Three mobile phases (mobile phase A: 100 % 7 mM ammonium acetate; mobile phase B: 100 % acetonitrile; mobile phase C: 0.2 % formic acid + 99.8 % ultra-pure Water) were employed to run a gradient condition from 30 % A , 40 % B and 30 % C (hold for 1 minute) to 100 % B in 4 minutes, 100 % B for 5 minutes and reequilibrated with initial conditions for 3 minutes. An injection volume of 5 µl was used. Cone voltages were 20, 40, 50, 55 V for positive ionization mode. Mass spectra were acquired by scanning from 100 to 1000 in 0.3 seconds using an interscan delay of 0.05 seconds.

### Method 7

In addition to general procedure B: Reversed phase HPLC was carried out on a YMC-Pack ODS-AQ C18 column (4.6 x 50 mm) with a flow rate of 2.6 ml/min. A gradient run was used from 95 % water and 5 % acetonitrile to 95 % acetonitrile in 7.30 minutes and was hold for 1.20 minutes. Mass spectra were acquired by scanning from 100 to 1000. Injection volume was 10 µl. Column temperature was 35 °C.

### Method 8

For compounds (6), (7), (8) and (9) only the mass spectra were recorded (no R(t)). The MS detector was configured with an electrospray ionization source. Mass spectra were acquired by scanning from 100 to 1000 in 1 second using a dwell time of 0.1 second. The capillary needle voltage was 3 kV and the source temperature was maintained at 140 °C. Nitrogen was used as the nebulizer gas. Data acquisition was performed with a Waters-Micromass MassLynx-Openlynx data system. Cone voltage was 10 V for positive ionization mode.

### Method 9

In addition to general procedure C: Reversed phase UPLC was carried out on a Thermo Hypersil Gold C18 column (1.9 µm, 2.1 x 100 mm) with a flow rate of 0.40 ml/min. Two mobile phases (mobile phase A: 95 % 7 mM ammonium acetate / 5 % acetonitrile; mobile phase B: 100 % acetonitrile) were employed to run a gradient condition from 72 % A and 28 % B (hold for 0.5 minutes) to 8 % A and 92 % B in 3.5 minutes, hold for 2 min and back to the initial conditions in 0.5 min, hold for 1.5 minutes. An injection volume of 2 µl was used. Cone voltages were 20, 30, 45, 60 V for positive ionization mode. Mass spectra were acquired by scanning from 100 to 1000 in 0.2 seconds using an interscan delay of 0.1 seconds.

### Method 10

In addition to general procedure C: Reversed phase UPLC was carried out on a Waters Acquity BEH (bridged ethylsiloxane/silica hybrid) C18 column (1.7 µm, 2.1 x 100 mm) with a flow rate of 0.35 ml/min. Two mobile phases (mobile phase A: 100 % 7 mM ammonium acetate; mobile phase B: 100 % acetonitrile) were employed to run a gradient condition from 75 % A and 25 % B (hold for 0.5 minutes) to 8% A and 92 % B in 3.5 minutes, hold for 2 minutes and reequilibrated with initial conditions for 2 minutes. An injection volume of 2 µl was used. Cone voltages were 20, 30, 45, 60 V for positive ionization mode. Mass spectra were acquired by scanning from 100 to 1000 in 0.2 seconds using an interscan delay of 0.1 seconds.

### Method 11

In addition to general procedure C: Reversed phase UPLC was carried out on a Waters Acquity bridged ethylsiloxane/silica hybrid (BEH) C18 column (1.7 µm, 2.1 x 100 mm) with a flow rate of 0.33 ml/min. Two mobile phases (mobile phase A: 100 % 7 mM ammonium acetate; mobile phase B: 100 % acetonitrile) were employed to run a gradient condition from 80 % A and 20 % B (hold for 0.75 minutes) to 10% A and 90 % B in 2.75 minutes, hold for 3 minutes and reequilibrated with initial conditions for 2 minutes. An injection volume of 2 µl was used. Cone voltage was 20 V for positive and negative ionization mode. Mass spectra were acquired by scanning from 100 to 1000 in 0.2 seconds using an interscan delay of 0.1 seconds.

### Method 12

In addition to general procedure C: Reversed phase UPLC was carried out on a Waters Acquity bridged ethylsiloxane/silica hybrid (BEH) C18 column (1.7 µm, 2.1 x 100 mm) with a flow rate of 0.4 ml/min. Two mobile phases (mobile phase A: 100 % 7 mM ammonium acetate; mobile phase B: 100 % acetonitrile) were employed to run a gradient condition from 80 % A and 20 % B (hold for 0.5 minutes) to 10% A and 90 % B in 3.5 minutes, hold for 2 minutes and reequilibrated with initial conditions for 2 minutes. An injection volume of 2 µl was used. Cone voltages were 20, 30, 45, 60 V for positive ionization mode. Mass spectra were acquired by scanning from 100 to 1000 in 0.2 seconds using an interscan delay of 0.1 seconds.

### Method 13

In addition to general procedure C: Reversed phase UPLC was carried out on a Thermo Hypersil Gold C18 column (1.9 µm, 2.1 x 100 mm) with a flow rate of 0.50 ml/min. Two mobile phases (mobile phase A: 95 % 7 mM ammonium acetate / 5 % acetonitrile; mobile phase B: 100 % acetonitrile) were employed to run a gradient condition from 40 % A and 60 % B (hold for 0.5 minutes) to 5 % A and 95 % B in 3.5 minutes, hold for 2 min and back to the initial conditions in 0.5 min, hold for 1.5 minutes. An injection volume of 2 µl was used. Cone voltage was 20 V for positive and negative ionization mode. Mass spectra were acquired by scanning from 100 to 1000 in 0.2 seconds using an interscan delay of 0.1 seconds.

### Method 14

In addition to general procedure C: Reversed phase UPLC was carried out on a Thermo Hypersil Gold C18 column (1.9 µm, 2.1 x 100 mm) with a flow rate of 0.50 ml/min. Two mobile phases (mobile phase A: 95 % 7 mM ammonium acetate / 5 % acetonitrile; mobile phase B: 100 % acetonitrile) were employed to run a gradient condition from 40 % A and 60 % B (hold for 0.5 minutes) to 5 % A and 95 % B in 3.5 minutes, hold for 2 min and back to the initial conditions in 0.5 min, hold for 1.5 minutes. An injection volume of 2 µl was used. Cone voltages were 20, 30, 45, 60 V for positive ionization mode. Mass spectra were acquired by scanning from 100 to 1000 in 0.2 seconds using an interscan delay of 0.1 seconds.

### Method 15

In addition to general procedure A: Reversed phase HPLC was carried out on a Xterra-MS C18 column (5 µm, 4.6 x 150 mm) with a flow rate of 1.0 ml/min. Two mobile phases (mobile phase A: 100 % 7 mM ammonium acetate; mobile phase B: 100 % acetonitrile; were employed to run a gradient condition from 85 % A , 15 % B (hold for 3 minutes) to 20 % A, 80 % B in 5 minutes, hold at 20 % A and 80 % B for 6 minutes and reequilibrated with initial conditions for 3 minutes. An injection volume of 20 µl was used. Cone voltages were 20, 40, 50, 55 V for positive ionization mode. Mass spectra were acquired by scanning from 100 to 1000 in 0.3 seconds using an interscan delay of 0.05 seconds.

### Method 16

In addition to general procedure C: Reversed phase UPLC was carried out on a Thermo Hypersil Gold C18 column (1.9 µm, 2.1 x 100 mm) with a flow rate of 0.35 ml/min. Two mobile phases (mobile phase A: 95 % 7 mM ammonium acetate / 5 % acetonitrile; mobile phase B: 100 % acetonitrile) were employed to run a gradient condition from 90 % A and 10 % B (hold for 0.5 minutes) to 8 % A and 92 % B in 3.5 minutes, hold for 2 min and back to the initial conditions in 0.5 min, hold for 1.5 minutes. An injection volume of 2 µl was used. Cone voltages were 20, 30, 45, 60 V for positive ionization mode. Mass spectra were acquired by scanning from 100 to 1000 in 0.2 seconds using an interscan delay of 0.1 seconds.

### Method 17

In addition to general procedure C: Reversed phase UPLC was carried out on a Thermo Hypersil Gold C18 column (1.9 µm, 2.1 x 100 mm) with a flow rate of 0.40 ml/min. Two mobile phases (mobile phase A: 95 % 7 mM ammonium acetate / 5 % acetonitrile; mobile phase B: 100 % acetonitrile) were employed to run a gradient condition from 72 % A and 28 % B (hold for 0.5 minutes) to 8 % A and 92 % B in 3.5 minutes, hold for 2 min and back to the initial conditions in 0.5 min, hold for 1.5 minutes. An injection volume of 2 µl was used. Cone voltage was 20 V for positive and negative ionization mode. Mass spectra were acquired by scanning from 100 to 1000 in 0.2 seconds using an interscan delay of 0.1 seconds.

### Method 18

In addition to general procedure A: Reversed phase HPLC was carried out on a Kromasil C18 column (3.5 µm, 4.6 x 100 mm) with a flow rate of 0.85 ml/min. Three mobile phases (mobile phase A: 100 % 7 mM ammonium acetate; mobile phase B: 100 % acetonitrile; mobile phase C: 0.2 % formic acid + 99.8 % ultra-pure Water) were employed to run a gradient condition from 35 % A , 30 % B and 35 % C (hold for 1 minute) to 100 % B in 3 minutes, 100 % B for 4.5 minutes and reequilibrated with initial conditions for 3 minutes. An injection volume of 5 µl was used. Cone voltage was 20 V for positive and negative ionization mode. Mass spectra were acquired by scanning from 100 to 1000 in 0.4 seconds using an interscan delay of 0.3 seconds.

When a compound is a mixture of isomers which give different peaks in the LCMS method , only the retention time of the main component is given in the LCMS table.

**Table 2 : LCMS data: (MH⁺), protonated molecular ion (of the free base), and retention time (Rₜ, in minutes)**

| **Compound No** | **LCMS method** | **(MH⁺)** | **Rₜ (min)** |
|---|---|---|---|
| 20 | 2 | 726 | 4.70 |
| 21 | 2 | 726 | 4.64 |
| 26 | 7 | 730 | 4.94 |
| 27 | 7 | 730 | 4.86 |
| 10 | 1 | 684 | 4.74 |
| 11 | 1 | 684 | 4.80 |
| 3 | 1 | 616 | 5.81 |
| 8 | 8 | 624 | - |
| 9 | 8 | 624 | - |
| 7 | 8 | 624 | - |
| 6 | 8 | 624 | - |
| 28 | 3 | 706 | 11.67 |
| 29 | 3 | 706 | 11.34 |
| 2 | 4 | 561 | 5.05 |
| 1 | 4 | 561 | 5.18 |
| 22 | 4 | 726 | 5.55 |
| 23 | 4 | 726 | 5.40 |
| 30 | 4 | 783 | 4.35 |
| 30b | 4 | 783 | 4.33 |
| 31 | 4 | 783 | 4.36 |
| 32 | 4 | 783 | 4.35 |
| 14 | 4 | 718 | 5.55 |
| 15 | 4 | 718 | 5.57 |
| 12 | 4 | 704 | 5.42 |
| 18 | 3 | 558 | 9.28 |
| 19 | 3 | 558 | 9.16 |
| 13 | 4 | 704 | 5.44 |
| 16 | 5 | 732 | 5.58 |
| 17 | 5 | 732 | 5.32 |
| 33 | 6 | 638 | 4.50 |
| 24 | 5 | 740 | 5.53 |
| 25 | 5 | 740 | 5.43 |
| 4 | 6 | 616 | 4.01 |
| 5 | 6 | 616 | 3.94 |
| 34 | 9 | 652 | 4.68 |
| 34b | 6 | 652 | 3.47 |
| 34d | 6 | 652 | 3.55 |
| 35 | 9 | 652 | 4.61 |
| 35a | 10 | 652 | 4.68 |
| 35c | 6 | 652 | 3.48 |
| 36 | 11 | 760 | 5.48 |
| 37 | 11 | 760 | 5.67 |
| 38 | 12 | 649 | 4.84 |
| 41 | 12 | 545 | 4.07 |
| 42 | 12 | 545 | 4.09 |
| 43 | 12 | 637 | 4.71 |
| 44 | 12 | 637 | 4.82 |
| 45 | 13 | 638 | 1.59 |
| 46 | 14 | 673 | 1.57 |
| 47 | 6 | 588 | 2.39 |
| 48 | 6 | 676 | 5.11 |
| 49 | 6 | 676 | 4.99 |
| 50 | 3 | 638 | 9.45 |
| 51 | 10 | 590 | 4.40 |
| 52 | 10 | 692 | 4.87 |
| 53 | 10 | 692 | 4.91 |
| 54 | 15 | 666 | 12.22 |
| 55 | 10 | 666 | 4.88 |
| 56 | 10 | 650 | 4.41 |
| 57 | 16 | 650 | 4.49 |
| 58 | 7 | 636 | 3.19 |
| 59 | 17 | 673 | 4.48 |
| 60 | 9 | 599 | 4.19 |
| 61 | 18 | 707 | 4.24 |
| 62 | 18 | 707 | 4.21 |
| 63 | 15 | 733 | 10.64 |
| 64 | 15 | 733 | 10.52 |
| 65 | 9 | 576 | 4.34 |
| 66 | 9 | 576 | 4.38 |

### Optical rotation

The optical rotation was measured using a polarimeter. [α]_{D}²⁰ indicates the optical rotation measured with light at the wavelength of the D-line of sodium (589 nm) at a temperature of 20°C. The cell pathlength is 1 dm. Behind the actual value the concentration and solvent of the solution which was used to measure the optical rotation are mentioned.

**Table 3 : Optical rotation data**

| **Comp. No.** | **[α]_{D}²⁰** | **concentration** | **solvent** |
|---|---|---|---|
| 34b | + 63.94 ° | 0.5005 w/v % | DMF |
| 34d | -63.48° | 0.5120 w/v % | DMF |
| 35a | +46.81 ° | 0.4700 w/v % | DMF |
| 35c | -43.95 ° | 0.5120 w/v % | DMF |

### D. Pharmacological examples

### D.1. In-vitro method for testing compounds against M. tuberculosis.

Flat-bottom, sterile 96-well plastic microtiter plates were filled with 100 µl of Middlebrook (1x) broth medium. Subsequently, stock solutions (10 x final test concentration) of compounds were added in 25 µl volumes to a series of duplicate wells in column 2 so as to allow evaluation of their effects on bacterial growth. Serial five-fold dilutions were made directly in the microtiter plates from column 2 to 11 using a customised robot system (Zymark Corp., Hopkinton, MA). Pipette tips were changed after every 3 dilutions to minimize pipetting errors with high hydrophobic compounds. Untreated control samples with (column 1) and without (column 12) inoculum were included in each microtiter plate. Approximately 5000 CFU per well of Mycobacterium tuberculosis (strain H37RV), in a volume of 100 µl in Middlebrook (1x) broth medium, was added to the rows A to H, except column 12. The same volume of broth medium without inoculum was added to column 12 in row A to H. The cultures were incubated at 37°C for 7 days in a humidified atmosphere (incubator with open air valve and continuous ventilation). One day before the end of incubation, 6 days after inoculation, Resazurin (1:5) was added to all wells in a volume of 20 µl and plates were incubated for another 24 hours at 37°C. On day 7 the bacterial growth was quantitated fluorometrically.

The fluorescence was read in a computer-controlled fluorometer (Spectramax Gemini EM, Molecular Devices) at an excitation wavelength of 530 nm and an emission wavelength of 590 nm. The percentage growth inhibition achieved by the compounds was calculated according to standard methods and expressed as IC₉₀ (µg/ml) which defines the 90 % inhibitory concentration for bacterial growth. The results are shown in Table 4.

### D.2. In-vitro method for testing compounds for anti-bacterial activity against strain M. Smegmatis ATCC607.

Flat-bottom, sterile 96-well plastic microtiter plates were filled with 180 µl of sterile deionized water, supplemented with 0.25 % BSA. Subsequently, stock solutions (7.8 x final test concentration) of compounds were added in 45 µl volumes to a series of duplicate wells in column 2 so as to allow evaluation of their effects on bacterial growth. Serial five-fold dilutions (45 µl in 180 µl) were made directly in the microtiter plates from column 2 to 11 using a customised robot system (Zymark Corp., Hopkinton, MA). Pipette tips were changed after every 3 dilutions to minimize pipetting errors with high hydrophobic compounds. Untreated control samples with (column 1) and without (column 12) inoculum were included in each microtiter plate. Approximately 250 CFU per well of bacteria inoculum, in a volume of 100 µl in 2.8x Mueller-Hinton broth medium, was added to the rows A to H, except column 12. The same volume of broth medium without inoculum was added to column 12 in row A to H. The cultures were incubated at 37°C for 48 hours in a humidified 5% CO₂ atmosphere (incubator with open air valve and continuous ventilation). At the end of incubation, two days after inoculation, the bacterial growth was quantitated fluorometrically. Therefore Alamar Blue (10x) was added to all wells in a volume of 20 µl and plates were incubated for another 2 hours at 50°C.

The fluorescence was read in a computer-controlled fluorometer (Cytofluor, Biosearch) at an excitation wavelength of 530 nm and an emission wavelength of 590 nm (gain 30). The percentage growth inhibition achieved by the compounds was calculated according to standard methods and expressed as IC₉₀ (µg/ml) which defines the 90 % inhibitory concentration for bacterial growth. The results are shown in Table 4.

### D.3. In-vitro method for testing compounds for anti-bacterial activity against various non-mycobacterial strains

### Preparation of bacterial suspensions for susceptibility testing:

The bacteria used in this study were grown overnight in flasks containing 100 ml Mueller-Hinton Broth (Becton Dickinson - cat. no. 275730) in sterile de-ionized water, with shaking, at 37 °C. Stocks (0.5 ml/tube) were stored at -70 °C until use. Bacteria titrations were performed in microtiter plates to detect the TCID₅₀, in which the TCID50 represents the dilution that gives rise to bacterial growth in 50 % of inoculated cultures.

In general, an inoculum level of approximately 100 TCID₅₀ was used for susceptibility testing.

### Anti bacterial Susceptibility testing: IC₉₀ determination

### Microtitre plate assay

Flat-bottom, sterile 96-well plastic microtiter plates were filled with 180 µl of sterile deionized water, supplemented with 0.25 % BSA. Subsequently, stock solutions (7.8 x final test concentration) of compounds were added in 45 µl volumes in column 2. Serial five-fold dilutions (45 µl in 180 µl) were made directly in the microtiter plates from column 2 to reach column 11. Untreated control samples with (column 1) and without (column 12) inoculum were included in each microtiter plate. Depending on the bacteria type, approximately 10 to 60 CFU per well of bacteria inoculum (100 TCID50), in a volume of 100 µl in 2.8x Mueller-Hinton broth medium, was added to the rows A to H, except column 12. The same volume of broth medium without inoculum was added to column 12 in row A to H. The cultures were incubated at 37°C for 24 hours under a normal atmosphere (incubator with open air valve and continuous ventilation). At the end of incubation, one day after inoculation, the bacterial growth was quantitated fluorometrically. Therefore resazurin (0.6 mg/ml) was added in a volume of 20 µl to all wells 3 hours after inoculation, and the plates were re-incubated overnight. A change in colour from blue to pink indicated the growth of bacteria.
The fluorescence was read in a computer-controlled fluorometer (Cytofluor Biosearch) at an excitation wavelength of 530 nm and an emission wavelength of 590 nm. The % growth inhibition achieved by the compounds was calculated according to standard methods. The IC₉₀ (expressed in µg/ml) was defined as the 90 % inhibitory concentration for bacterial growth. The results are shown in Table 4.

### Agar dilution method.

MIC₉₉ values (the minimal concentration for obtaining 99 % inhibition of bacterial growth) can be determined by performing the standard Agar dilution method according to NCCLS standards* wherein the media used includes Mueller-Hinton agar.
* Clinical laboratory standard institute. 2005. Methods for dilution Antimicrobial susceptibility tests for bacteria that grows Aerobically: approved standard -sixth edition

### Time kill assays

Bactericidal or bacteriostatic activity of the compounds may be determined in a time kill assay using the broth microdilution method *. In a time kill assay on *Staphylococcus aureus* and methicillin resistant *S. aureus* (MRSA), the starting inoculum of *S. aurues* and MRSA is 10⁶ CFU / ml in Muller Hinton broth. The antibacterial compounds are used at the concentration of 0.1 to 10 times the MIC (i.e. IC₉₀ as determined in microtitre plate assay). Wells receiving no antibacterial agent constitute the culture growth control. The plates containing the microorganism and the test compounds are incubated at 37 °C. After 0, 4, 24, and 48 hrs of incubation samples are removed for determination of viable counts by serial dilution (10⁻¹ to 10⁻⁶) in sterile PBS and plating (200 µl) on Mueller Hinton agar. The plates are incubated at 37 °C for 24 hrs and the number of colonies are determined. Killing curves can be constructed by plotting the log₁₀CFU per ml versus time. A bactericidal effect is commonly defined as 3-log₁₀ decrease in number of CFU per ml as compared to untreated inoculum. The potential carryover effect of the drugs is removed by serial dilutions and counting the colonies at highest dilution used for plating.
* Zurenko,G.E. et al. In vitro activities of U-100592 and U-100766, novel oxazolidinone antibacterial agents. Antimicrob. Agents Chemother. 40, 839-845 (1996).

### Determination of cellular A TP levels

In order to analyse the change in the total cellular ATP concentration ( using ATP bioluminescence Kit, Roche), assays are carried out by growing a culture of *S. aureus* (ATCC29213) stock in 100 ml Mueller Hinton flasks and incubate in a shaker-incubator for 24 hrs at 37 °C (300 rpm). Measure OD₄₀₅ nm and calculate the CFU/ml. Dilute the cultures to 1 x 10⁶ CFU/ml (final concentration for ATP measurement: 1 x 10⁵ CFU/100 µl per well) and add test compound at 0.1 to 10 times the MIC (i.e. IC₉₀ as determined in microtitre plate assay). Incubate these tubes for 0, 30 and 60 minutes at 300 rpm and 37°C. Use 0.6 ml bacterial suspension from the snap-cap tubes and add to a new 2 ml eppendorf tubes. Add 0.6 ml cell lysis reagent ( Roche kit), vortex at max speed and incubate for 5 minutes at room temperature. Cool on ice. Let the luminometer warm up to 30°C (Luminoskan Ascent Labsystems with injector). Fill one column (= 6 wells) with 100 µl of the same sample. Add 100 µl Luciferase reagent to each well by using the injector system. Measure the luminescence for 1 sec.

**Table 4: IC₉₀ values (µg/ml).**

| | **IC90 (µg/ml)** | | | |
|---|---|---|---|---|
| **Comp. No.** | **STA 1** | **SPN 1** | **MTB 1** | **MSM 1** |
| | **B29213** | **6305** | **H37RV** | **ATCC607** |
| 1 | 2.0 | 2.2 | 4.0 | 1.8 |
| 2 | 1.8 | 2.2 | 2.5 | 1.8 |
| 3 | 61.7 | 9.8 | | 4.9 |
| 4 | 9.8 | 2.0 | | 2.0 |
| 5 | 7.8 | 2.0 | | 2.0 |
| 6 | 2.0 | 2.2 | | 2.0 |
| 7 | 2.0 | 2.0 | | 2.0 |
| 8 | 2.0 | 2.2 | | 2.0 |
| 9 | 2.0 | 2.2 | | 2.0 |
| 10 | 2.2 | 2.2 | | 2.4 |
| 11 | 10.9 | 10.9 | | 12.2 |
| 12 | 11.2 | 2.2 | | 2.2 |
| 13 | 2.2 | 2.5 | | 5.6 |
| 14 | 11.4 | 2.6 | | 3.6 |
| 15 | 11.4. | 2.6 | | 4.0 |
| 16 | 7.3 | 1.5 | | 0.6 |
| 17 | 2.3 | 0.8 | | 0.7 |
| 18 | 1.8 | 1.8 | | 1.8 |
| 19 | 1.8 | 2.2 | | 1.6 |
| 20 | 11.5 | 0.6 | | 2.3 |
| 21 | 2.3 | 0.6 | | 5.8 |
| 22 | 9.2 | 2.9 | | 0.6 |
| 23 | 2.3 | 0.2 | 57.7 | 0.5 |
| 24 | 11.7 | 2.1 | | 2.3 |
| 25 | 2.3 | 0.5 | | 1.7 |
| 26 | 18.4 | 18.4 | | 18.4 |
| 27 | 18.4 | 18.4 | | 18.4 |
| 28 | 2.2 | 2.8 | | 2.5 |
| 29 | 5.0 | 0.8 | | 2.2 |
| 30 | 2.5 | 2.5 | | 2.5 |
| 30b | 62 | 31 | | 31 |
| 31 | 12.4 | 15.6 | | 4.4 |
| 32 | 2.5 | 2.5 | | 5.6 |
| 33 | 2.0 | 0.5 | | 0.4 |
| 34 | 2.1 | | | 2.1 |
| 34b | 2.1 | 2.1 | | 2.1 |
| 34d | 2.1 | 0.4 | | 2.1 |
| 35 | 2.1 | | | 2.1 |
| 35a | 2.1 | 0.4 | | 0.9 |
| 35c | 2.1 | 0.4 | | 1.5 |
| 36 | 53.9 | 0.9 | | 2.7 |
| 37 | 2.7 | 0.5 | | 2.1 |
| 38 | 10.3 | 2.6 | | 2.1 |
| 39 | 1.8 | 0.4 | | 1.8 |
| 40 | 1.6 | 0.5 | | 0.4 |
| 41 | 1.7 | 1.7 | | 1.7 |
| 42 | 8.6 | 4.3 | | 2.2 |
| 43 | 10.1 | 9.0 | | 4.0 |
| 44 | 2.0 | 0.9 | | 2.0 |
| 45 | 2.3 | | | 2.0 |
| 47 | 1.9 | 0.9 | | 1.9 |
| 48 | 2.1 | 0.5 | | 2.4 |
| 49 | 2.1 | 0.3 | | 2.1 |
| 50 | 10.1 | 4.5 | | 2.3 |
| 51 | 9.4 | 9.4 | | 9.4 |
| 52 | 2.2 | 0.5 | | 2.2 |
| 53 | 2.2 | 0.1 | | 1.6 |
| 54 | 2.1 | 2.1 | | 2.1 |
| 55 | 2.1 | 0.4 | | 2.1 |
| 56 | 2.3 | | | 2.1 |
| 57 | 2.3 | | | 2.1 |
| 59 | 67.4 | | | 67.4 |
| 60 | 1.9 | | | 1.9 |
| 61 | 2.2 | | | 2.2 |
| 62 | 2.2 | | | 2.2 |
| 63 | 2.3 | | | 2.3 |
| 64 | 2.3 | | | 1.3 |
| 65 | 1.8 | | | 1.8 |
| 66 | 1.8 | | | 1.8 |

STA B29213 means *Staphylococcus aureus* (ATCC29213); SPN 6305 means *Streptococcus pneumoniae* (ATCC6305); MSM 607 means *M. Smegmatis* (ATCC607); MTB H37RV means *Mycobacterium tuberculosis* (strain H37RV); ATCC means American type tissue culture;

## Claims

1. A compound of formula (Ia) or (Ib) 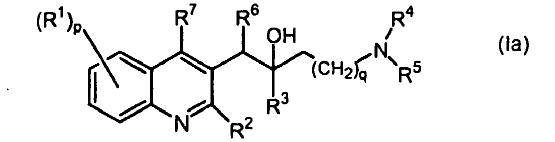 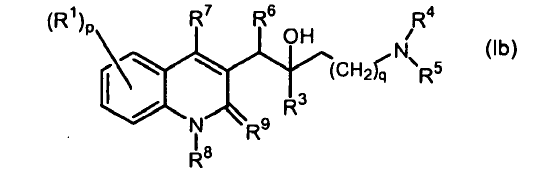 including any stereochemically isomeric form thereof, wherein
p is an integer equal to 1, 2, 3 or 4;
q is an integer equal to zero, 1, 2, 3 or 4 ;
R¹ is hydrogen, cyano, formyl, carboxyl, halo, alkyl, C₂₋₆alkenyl, C₂₋₆alkynyl, haloalkyl, hydroxy, alkyloxy, alkylthio, alkylthioalkyl, -C=N-OR¹¹, amino, mono or di(alkyl)amino, aminoalkyl, mono or di(alkyl)aminoalkyl, alkylcarbonylaminoalkyl, aminocarbonyl, mono or di(alkyl)aminocarbonyl, arylalkyl, arylcarbonyl, R^{5a}R^{4a}Nalkyl, di(aryl)alkyl, aryl, R^{5a}R^{4a}N-, R^{5a}R^{4a}N-C(=O)-, or Het;
R² is hydrogen, alkyloxy, aryl, aryloxy, hydroxy, mercapto, alkyloxyalkyloxy, alkylthio, mono or di(alkyl)amino, pyrrolidino or a radical of formula 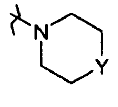 wherein Y is CH₂, O, S, NH or *N*-alkyl ;
R³ is alkyl, arylalkyl, aryl-O-alkyl, aryl-alkyl-O-alkyl, aryl, aryl-aryl, Het, Het-alkyl, Het-O-alkyl, Het-alkyl-O-alkyl or 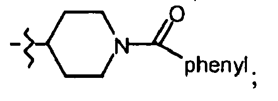
R⁴ is hydrogen or alkyl;
R⁵ is -C(=NH)-NH₂; arylalkyl; Het-alkyl; mono- or dialkylaminoalkyl; bicyclo[2.2.1]heptyl; Het; or aryl; or
R⁴ and R⁵ together with the nitrogen atom to which they are attached form a radical selected from the group consisting of azetidinyl; 2,3-dihydroisoindol-1-yl; thiazolidin-3-yl; 1,2,3,6-tetrahydropyridyl; hexahydro-1*H-*azepinyl; hexahydro-1*H*-1,4-diazepinyl; hexahydro-1,4- oxazepinyl; 1,2,3,4-tetrahydroisoquinolin-2-yl; 2,5-diazabicyclo[2.2.1]heptyl; 1,1-dioxide-thiomorpholinyl; each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, mono- or dialkylaminoalkyl, alkylthio, alkyloxyalkyl, alkylthioalkyl, aryl, piperidinyl optionally substituted with alkyl, pyrrolidinyl optionally substituted with arylalkyl, pyridyl or pyrimidinyl; or
R⁴ and R⁵ together with the nitrogen atom to which they are attached form a radical selected from the group consisting piperidinyl or piperazinyl, each substituted with aryl, alkylcarbonyl, piperidinyl or pyrrolidinyl optionally substituted with arylalkyl;
R^{4a} and R^{5a} together with the nitrogen atom to which they are attached form a radical selected from the group consisting of pyrrolidino, piperidino, piperazino, morpholino, 4-thiomorpholino, 2,3-dihydroisoindol-1-yl, thiazolidin-3-yl, 1,2,3,6-tetrahydropyridyl, hexahydro-1*H*-azepinyl, hexahydro-1*H*-1,4-diazepinyl, hexahydro-1,4-oxazepinyl, 1,2,3,4- tetrahydroisoquinolin-2-yl, pyrrolinyl, pyrrolyl, imidazolidinyl, pyrazolidinyl, 2-imidazolinyl, 2-pyrazolinyl, imidazolyl, pyrazolyl, triazolyl, pyridinyl, pyridazinyl, pyrimidinyl, pyrazinyl and triazinyl, each radical optionally substituted with 1, 2, 3 or 4 substituents, each substituent independently selected from alkyl, haloalkyl, halo, arylalkyl, hydroxy, alkyloxy, amino, mono- or dialkylamino, alkylthio, alkylthioalkyl, aryl, pyridyl or pyrimidinyl;
R⁶ is aryl¹ or Het;
R⁷ is hydrogen, halo, alkyl, aryl or Het;
R⁸ is hydrogen or alkyl;
R⁹ is oxo; or
R⁸ and R⁹ together form the radical -CH=CH-N=;
R¹¹ is hydrogen or alkyl;
aryl is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, C₂₋₆alkenyl optionally substituted with phenyl, haloalkyl, alkyloxy, haloalkyloxy, carboxyl, alkyloxycarbonyl, aminocarbonyl, morpholinyl or mono- or dialkylaminocarbonyl;
aryl¹ is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, alkylthio, haloalkyloxy, carboxyl, alkyloxycarbonyl, aminocarbonyl, morpholinyl, Het or mono- or dialkylaminocarbonyl;
Het is a monocyclic heterocycle selected from *N*-phenoxypiperidinyl, piperidinyl, piperazine, pyrrolyl, pyrazolyl, imidazolyl, furanyl, thienyl, oxazolyl, isoxazolyl, thiazolyl, isothiazolyl, pyridinyl, pyrimidinyl, pyrazinyl or pyridazinyl; or a bicyclic heterocycle selected from quinolinyl, quinoxalinyl, indolyl, benzimidazolyl, benzoxazolyl, benzisoxazolyl, benzothiazolyl, benzisothiazolyl, benzofuranyl, benzothienyl, 2,3-dihydrobenzo[1,4]dioxinyl or benzo[1,3]dioxolyl; each monocyclic and bicyclic heterocycle being optionally substituted with 1, 2 or 3 substituents, each substituent independently selected from halo, hydroxy, alkyl or alkyloxy;
provided R⁵ is other than benzyl;
a *N*-oxide thereof, a pharmaceutically acceptable salt thereof or a solvate thereof.

2. A compound according to claim 1 wherein
R³ is alkyl, arylalkyl, aryl-O-alkyl, aryl-alkyl-O-alkyl, aryl, Het, Het- alkyl, Het-O-alkyl, Het-alkyl-O-alkyl or 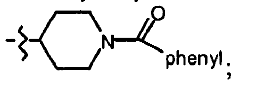
R⁴ is hydrogen or alkyl;
R⁵ is -C(=NH)-NH₂; arylalkyl; Het-alkyl; mono- or dialkylaminoalkyl; Het; or aryl; or
R⁴ and R⁵ together with the nitrogen atom to which they are attached form a radical selected from the group consisting of 2,3-dihydroisoindol-1- yl; thiazolidin-3-yl; 1,2,3,6-tetrahydropyridyl; hexahydro-1*H*- azepinyl; hexahydro-1*H*-1,4-diazepinyl; hexahydro-1,4-oxazepinyl; 1,2,3,4-tetrahydroisoquinolin-2-yl or 2,5-diazabicyclo[2.2.1]heptyl; each radical optionally substituted with 1, 2, 3 or 4 substituents, each substituent independently selected from alkyl, haloalkyl, alkylcarbonyl, halo, arylalkyl, hydroxy, alkyloxy, amino, mono- or dialkylamino, alkylthio, alkyloxyalkyl, alkylthioalkyl, aryl, pyridyl or pyrimidinyl; or
R⁴ and R⁵ together with the nitrogen atom to which they are attached form a radical selected from the group consisting piperidinyl or piperazinyl, each substituted with aryl, alkylcarbonyl, piperidinyl or pyrrolidinyl optionally substituted with arylalkyl;
aryl is a homocycle selected from phenyl, naphthyl, acenaphthyl or tetrahydronaphthyl, each being optionally substituted with 1, 2 or 3 substituents, each substituent being independently selected from hydroxy, halo, cyano, nitro, amino, mono- or dialkylamino, alkyl, haloalkyl, alkyloxy, haloalkyloxy, carboxyl, alkyloxycarbonyl, aminocarbonyl, morpholinyl or mono- or dialkylaminocarbonyl;

3. A compound according to claim 1 or 2 wherein alkyl represents C₁₋₆alkyl.

4. A compound according to any one of the preceding claims wherein R¹ is hydrogen or halo.

5. A compound according to any one of the preceding claims wherein p is equal to 1.

6. A compound according to any one of the preceding claims wherein R² is C₁₋₆alkyloxy.

7. A compound according to claim 6 wherein R² is methoxy.

8. A compound according to any one of the preceding claims wherein R³ is arylC₁₋₆alkyl or aryl.

9. A compound according to any one of the preceding claims wherein q is equal to 3 or 4.

10. A compound according to any one of the preceding claims wherein R⁴ is hydrogen or C₁₋₆alkyl.

11. A compound according to claim 10 wherein R⁴ is C₁₋₆alkyl.

12. A compound according to any one of the preceding claims wherein R⁵ is -C(=NH)-NH₂; Het-C₁₋₆alkyl; mono- or di(C₁₋₆alkyl)aminoC₁₋₆alkyl; bicyclo[2.2.1]heptyl; or Het.

13. A compound according to claim 12 wherein R⁵ is -C(=NH)-NH₂; Het-C₁₋₆alkyl; bicyclo[2.2.1]heptyl; or Het.

14. A compound according to any one of claims 1 to 9 wherein R⁴ and R⁵ together with the nitrogen atom to which they are attached form a radical selected from the group consisting of azetidinyl; 2,3-dihydroisoindol-1-yl; thiazolidin-3-yl; 1,2,3,6-tetrahydropyridyl; hexahydro-1*H*-azepinyl; hexahydro-1*H*-1,4-diazepinyl ; hexahydro-1,4-oxazepinyl; 1,2,3,4-tetrahydroisoquinolin-2-yl; 2,5-diazabicyclo[2.2.1]heptyl; 1,1-dioxide-thiomorpholinyl; each radical optionally substituted with 1, 2, 3 or 4 substituents, each substituent independently selected from C₁₋₆alkyl, haloC₁₋₆alkyl, C₁₋₆alkylcarbonyl, halo, arylC₁₋₆alkyl, hydroxy, C₁₋₆alkyloxy, amino, mono- or diC₁₋₆alkylamino, mono- or diC₁₋₆alkylaminoC₁₋₆alkyl, C₁₋₆alkylthio, C₁₋₆alkyloxyC₁₋₆alkyl, C₁₋₆alkylthioC₁₋₆alkyl, aryl, piperidinyl optionally substituted with C₁₋₆alkyl, pyrrolidinyl optionally substituted with arylC₁₋₆alkyl, pyridyl or pyrimidinyl.

15. A compound according to any one of claims 1 to 9 wherein R⁴ and R⁵ together with the nitrogen atom to which they are attached form a radical selected from the group consisting of azetidinyl, hexahydro-1*H*-1,4-diazepinyl, 2,5-diazabicyclo[2.2.1]heptyl or hexahydro-1*H*-azepinyl; each radical optionally substituted with 1, 2, 3 or 4 substituents, each substituent independently selected from C₁₋₆alkyl or arylC₁₋₆alkyl; or R⁴ and R⁵ together with the nitrogen atom to which they are attached form a radical selected from the group consisting of piperidinyl or piperazinyl, each substituted with aryl, C₁₋₆alkylcarbonyl, piperidinyl or pyrrolidinyl optionally substituted with arylC₁₋₆alkyl.

16. A compound according to claim 15 wherein R⁴ and R⁵ together with the nitrogen atom to which they are attached form a radical selected from the group consisting of azetidinyl, hexahydro-1*H-*1,4-diazepinyl, 2,5-diazabicyclo[2.2.1]heptyl or hexahydro-1*H*-azepinyl; each radical optionally substituted with 1, 2, 3 or 4 substituents, each substituent independently selected from C₁₋₆alkyl or arylC₁₋₆alkyl.

17. A compound according to any one of the preceding claims wherein R⁶ is phenyl optionally substituted with halo.

18. A compound according to any one of the preceding claims wherein R⁷ is hydrogen.

19. A compound according to any one of the preceding claims wherein the compound is a compound of formula (Ia).

20. A compound according to any one of claims 1 to 18 wherein the compound is a compound of formula (Ib) and wherein R⁸ is hydrogen and R⁹ is oxo.

21. A compound according to claim 1 wherein the compound is a compound of formula (Ia) and wherein R¹ is hydrogen or halo; R² is C₁₋₆alkyloxy; R³ is arylC₁₋₆alkyl or aryl; R⁴ is hydrogen or C₁₋₆alkyl; R⁵ is -C(=NH)-NH₂; Het-C₁₋₆alkyl; mono- or di(C₁₋₆alkyl)aminoC₁₋₆alkyl; bicyclo[2.2.1]heptyl;or Het; or R⁴ and R⁵ together with the nitrogen atom to which they are attached form a radical selected from the group consisting of azetidinyl; hexahydro-1*H*-azepinyl; hexahydro-1*H*-1,4-diazepinyl; 2,5-diazabicyclo[2.2.1]heptyl; or 1,1-dioxide-thiomorpholinyl; each radical optionally substituted with 1, 2, 3 or 4 substituents, each substituent independently selected from C₁₋₆alkyl, arylC₁₋₆alkyl, piperidinyl optionally substituted with C₁₋₆alky; or R⁴ and R⁵ together with the nitrogen atom to which they are attached form a radical selected from the group consisting of piperidinyl or piperazinyl, each substituted with aryl, C₁₋₆alkylcarbonyl, piperidinyl or pyrrolidinyl optionally substituted with arylC₁₋₆alkyl; R⁶ is phenyl optionally substituted with halo; R⁷ is hydrogen; q is 3 or 4; p is 1.

22. A compound according to claim 1 wherein the compound is selected from 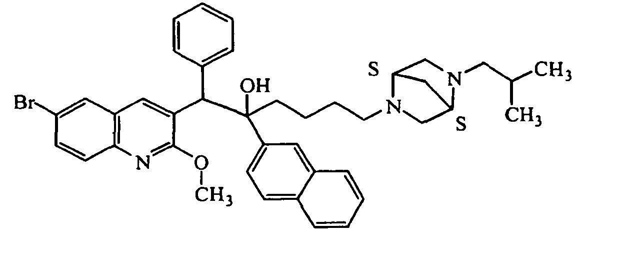 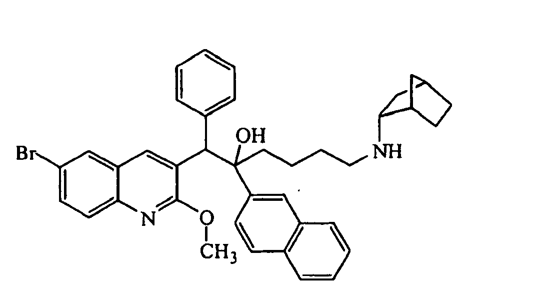 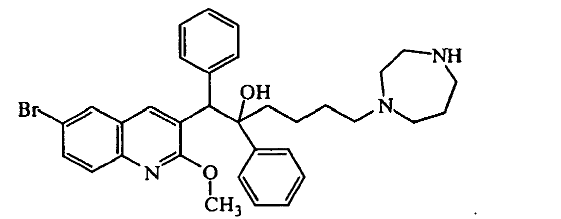 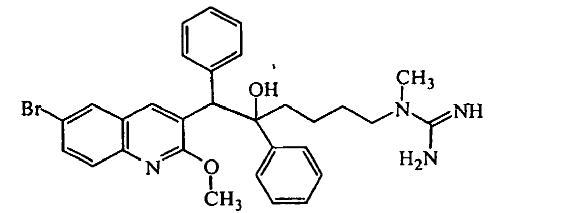 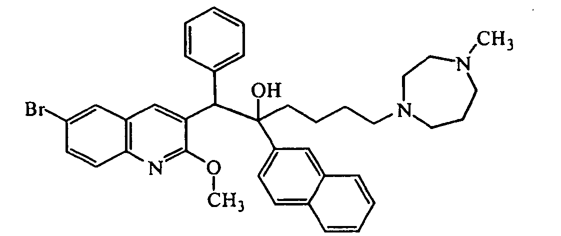 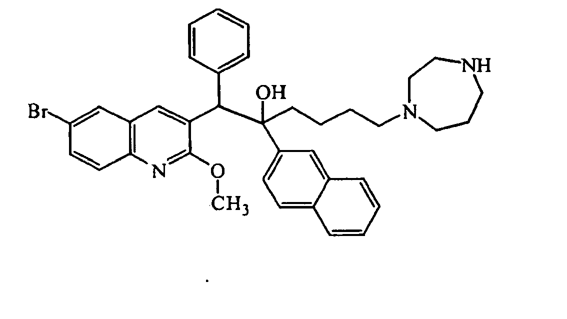 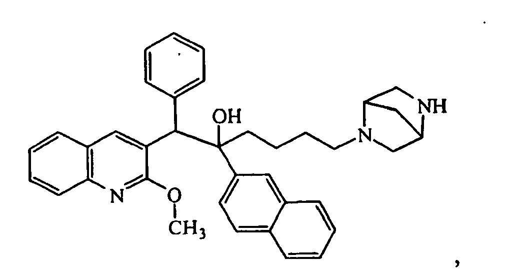 including any stereochemically isomeric form thereof;
a *N*-oxide thereof, a pharmaceutically acceptable salt thereof or a solvate thereof.

23. A compound according to any one of the preceding claims for use as a medicine.

24. A compound according to any one of claims 1 to 22 for use as a medicine for the treatment of a bacterial infection.

25. A pharmaceutical composition comprising a pharmaceutically acceptable carrier and, as active ingredient, a therapeutically effective amount of a compound as defined in any one of claims 1 to 22.

26. A compound according to claim 24 wherein the bacterial infection is an infection with a gram-positive bacterium.

27. A compound according to claim 26 wherein the gram-positive bacterium is Streptococcus pneumonia or Staphylococcus aureus.

28. A process to prepare a compound according to claim 1 **characterized by**
a) reacting an intermediate of formula (II-a) or (II-b) with 1*H*-pyrazole-1-carboximidamide in the presence of a suitable base and a suitable solvent, 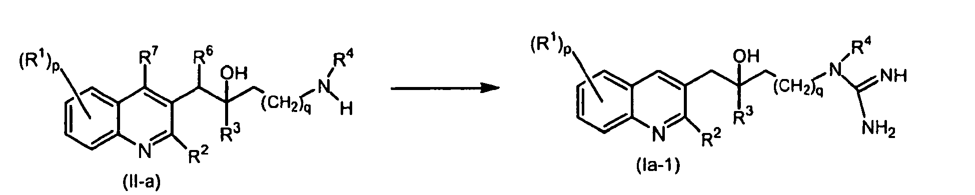 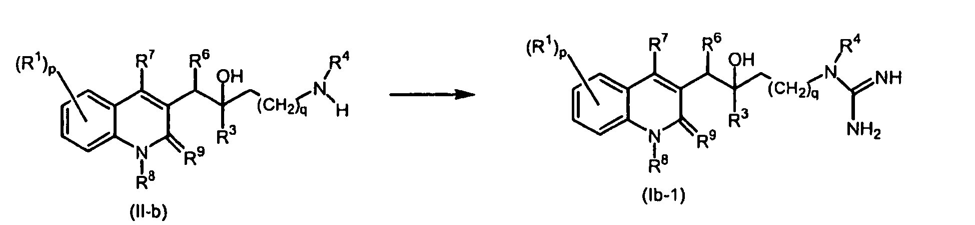 wherein all variables are as defined in claim 1;
b) reacting an intermediate of formula (III-a) or (III-b) with an intermediate of formula (IV) according to the following reaction scheme : 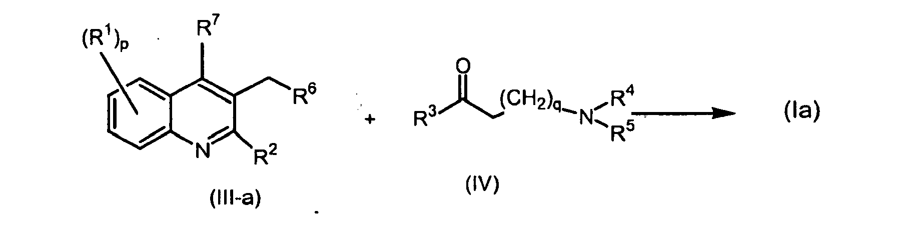 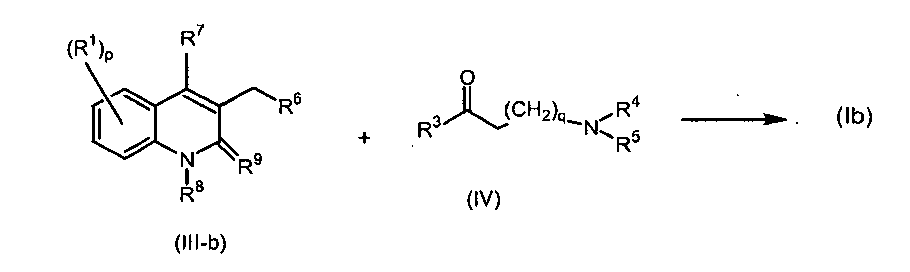
using nBuLi in a mixture of a suitable base and a suitable solvent, wherein all variables are defined as in claim 1;
c) reacting an intermediate of formula (V-a) or (V-b) wherein q' is 0, 1 or 2, with a primary or secondary amine HNR⁴R⁵ in the presence of a suitable catalyst, optionally in the presence of a second catalyst (for the reduction), in the presence of a suitable ligand, in a suitable solvent, in the presence of CO and H₂ (under pressure), 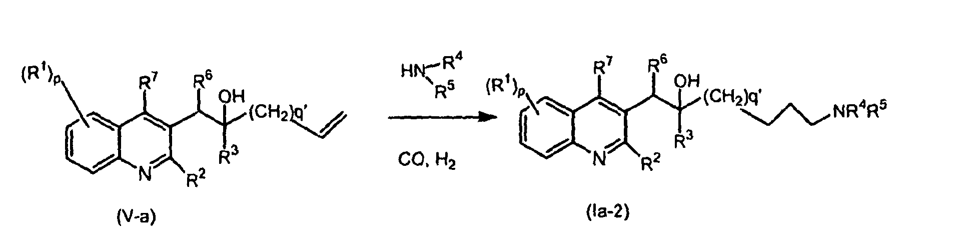 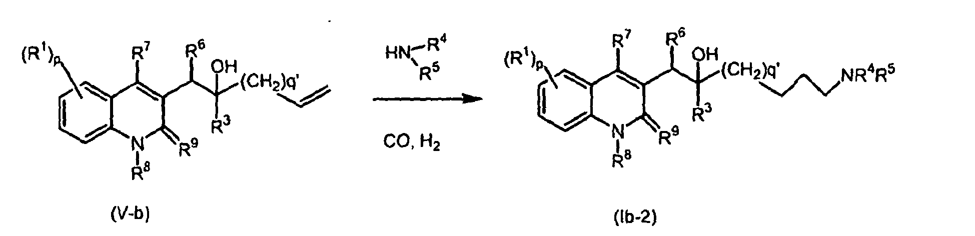 wherein all variables are defined as in claim 1;
d) reacting an intermediate of formula (VI-a) or (VI-b) wherein W₂ represents a suitable leaving group, with a suitable primary or secondary amine HNR⁴R⁵, optionally in the presence of a suitable solvent 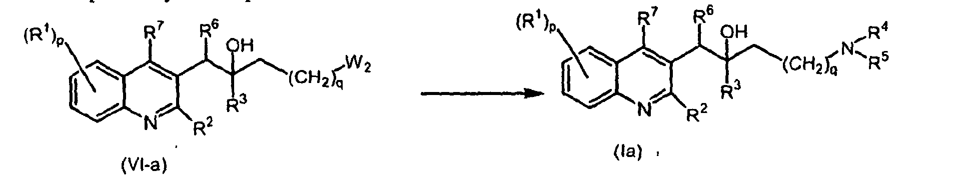 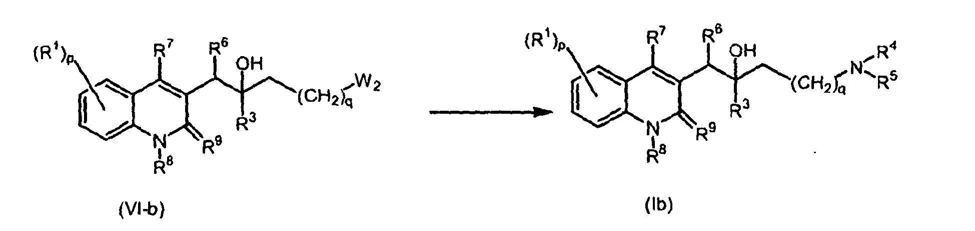 wherein all variables are defined as in claim 1;
or, if desired, converting compounds of formula (Ia) or (Ib) into each other following art-known transformations, and further, if desired, converting the compounds of formula (Ia) or (Ib), into a therapeutically active non-toxic acid addition salt by treatment with an acid, or into a therapeutically active non-toxic base addition salt by treatment with a base, or conversely, converting the acid addition salt form into the free base by treatment with alkali, or converting the base addition salt into the free acid by treatment with acid; and, if desired, preparing stereochemically isomeric forms, quaternary amines or *N*-oxide forms thereof.

29. A combination of (a) a compound according to any one of claims 1 to 22, and (b) one or more other antibacterial agents.

30. A product containing (a) a compound according to any one of claims 1 to 22, and (b) one or more other antibacterial agents, as a combined preparation for simultaneous, separate or sequential use in the treatment of a bacterial infection.

31. A compound according to claim 27 wherein the gram-positive bacterium is methicillin resistant *Staphylococcus aureus.*

32. A compound according to claim 24 wherein the bacterial infection is an infection with *Mycobacterium tuberculosis.*

## Patentansprüche

1. Verbindung der Formel (Ia) oder (Ib) 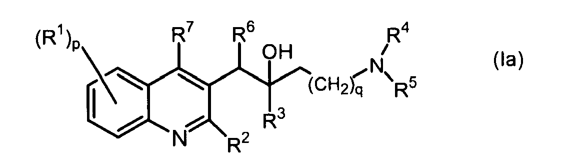 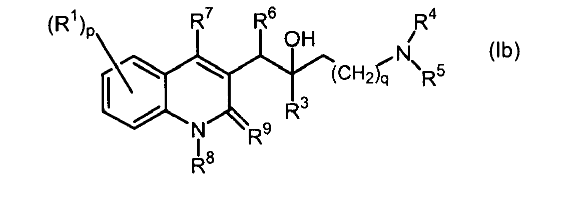 einschließlich beliebiger stereochemisch isomerer Formen davon, worin
p für eine ganze Zahl mit einem Wert von 1, 2, 3 oder 4 steht;
q für eine ganze Zahl mit einem Wert von null, 1, 2, 3 oder 4 steht;
R¹ für Wasserstoff, Cyano, Formyl, Carboxyl, Halogen, Alkyl, C₂₋₆-Alkenyl, C₂₋₆-Alkinyl, Halogenalkyl, Hydroxy, Alkyloxy, Alkylthio, Alkylthioalkyl, -C=N-OR¹¹, Amino, Mono- oder Di- (alkyl)amino, Aminoalkyl, Mono- oder Di(alkyl)- aminoalkyl, Alkylcarbonylaminoalkyl, Amino- carbonyl, Mono- oder Di(alkyl)aminocarbonyl, Arylalkyl, Arylcarbonyl, R^{5a}R^{4a}N-Alkyl, Di(aryl)- alkyl, Aryl, R^{5a}R^{4a}N-, R^{5a}R^{4a}-N-C(=O)- oder Het steht;
R² für Wasserstoff, Alkyloxy, Aryl, Aryloxy, Hydroxy, Mercapto, Alkyloxyalkyloxy, Alkylthio, Mono- oder Di(alkyl)amino, Pyrrolidino oder einen Rest der Formel 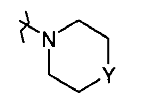 worin Y CH₂, O, S, NH oder *N*-Alkyl bedeutet, steht;
R³ für Alkyl, Arylalkyl, Aryl-O-alkyl, Arylalkyl- O-alkyl, Aryl, Arylaryl, Het, Het-Alkyl, Het-O- Alkyl, Het-Alkyl-O-alkyl oder 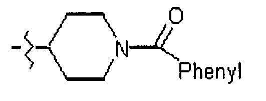 steht;
R⁵ R⁴ für Wasserstoff oder Alkyl steht; für -C(=NH)-NH₂; Arylalkyl; Het-Alkyl; Mono- oder Dialkylaminoalkyl; Bicyclo[2.2.1]heptyl; Het oder Aryl steht oder R⁴ und R⁵ gemeinsam mit dem Stickstoffatom, an das sie gebunden sind, einen Rest aus der Gruppe bestehend aus Azetidinyl; 2,3-Dihydroisoindol- 1-yl; Thiazolidin-3-yl; 1,2,3,6-Tetrahydro- pyridyl; Hexahydro-1*H*-azepinyl; Hexahydro-1*H*- 1,4-diazepinyl; Hexahydro-1,4-oxazepinyl; 1,2,3,4-Tetrahydroisochinolin-2-yl; 2,5-Diaza- bicyclo[2.2.1]heptyl; Thiomorpholinyl-1,1- dioxid bilden; wobei jeder Rest gegebenenfalls durch 1, 2, 3 oder 4 Substituenten substituiert ist, wobei jeder Substituent unabhängig unter Alkyl, Halogenalkyl, Alkylcarbonyl, Halogen, Arylalkyl, Hydroxy, Alkyloxy, Amino, Mono- oder Dialkylamino, Mono- oder Dialkylaminoalkyl, Alkylthio, Alkyloxyalkyl, Alkylthioalkyl, Aryl, Piperidinyl, das gegebenenfalls durch Alkyl substituiert ist, Pyrrolidinyl, das gegebenen- falls durch Arylalkyl substituiert ist, Pyridyl oder Pyrimidinyl ausgewählt ist; oder
R⁴ und R⁵ gemeinsam mit dem Stickstoffatom, an das sie gebunden sind, einen Rest aus der Gruppe bestehend aus Piperidinyl oder Piperazinyl bilden, wobei jeder Rest durch Aryl, Alkyl- carbonyl, Piperidinyl oder Pyrrolidinyl, das gegebenenfalls durch Arylalkyl substituiert ist, substituiert ist;
R^{4a} und R^{5a} gemeinsam mit dem Stickstoffatom, an das sie gebunden sind, einen Rest aus der Gruppe bestehend aus Pyrrolidino, Piperidino, Piperazino, Morpholino, 4-Thiomorpholino, 2,3- Dihydroisoindol-1-yl, Thiazolidin-3-yl, 1,2,3,6-Tetrahydropyridyl, Hexahydro-1*H*- azepinyl, Hexahydro-1*H*-1,4-diazepinyl, Hexa- hydro-1,4-oxazepinyl, 1,2,3,4-Tetrahydroiso- chinolin-2-yl, Pyrrolinyl, Pyrrolyl, Imidazolidinyl, Pyrazolidinyl, 2-Imidazolinyl, 2-Pyrazolinyl, Imidazolyl, Pyrazolyl, Triazolyl, Pyridinyl, Pyridazinyl, Pyrimidinyl, Pyrazinyl und Triazinyl bilden, wobei jeder Rest gegebenenfalls durch 1, 2, 3 oder 4 Substituenten substituiert ist, wobei jeder Substituent unabhängig unter Alkyl, Halogen- alkyl, Halogen, Arylalkyl, Hydroxy, Alkyloxy, Amino, Mono- oder Dialkylamino, Alkylthio, Alkylthioalkyl, Aryl, Pyridyl oder Pyrimidinyl ausgewählt ist;
R⁶ für Aryl¹ oder Het steht;
R⁷ für Wasserstoff, Halogen, Alkyl, Aryl oder Het steht;
R⁸ für Wasserstoff oder Alkyl steht;
R⁹ für Oxo steht; oder
R⁸ und R⁹ gemeinsam den Rest -CH=CH-N= bilden;
R¹¹ für Wasserstoff oder Alkyl steht; Aryl für einen Homocyclus steht, der unter Phenyl, Naphthyl, Acenaphthyl oder Tetrahydronaphthyl ausgewählt ist, wobei jeder dieser Cyclen gegebenenfalls durch 1, 2 oder 3 Substituenten substituiert ist, wobei jeder Substituent unabhängig unter Hydroxy, Halogen, Cyano, Nitro, Amino, Mono- oder Dialkylamino, Alkyl, C₂₋₆-Alkenyl, das gegebenenfalls durch Phenyl substituiert ist, Halogenalkyl, Alkyloxy, Halogenalkyloxy, Carboxyl, Alkyloxycarbonyl, Aminocarbonyl, Morpholinyl oder Mono- oder Dialkylaminocarbonyl ausgewählt ist;
Aryl¹ für einen Homocyclus steht, der unter Phenyl, Naphthyl, Acenaphthyl oder Tetrahydronaphthyl ausgewählt ist, wobei jeder dieser Reste gegebenenfalls durch 1, 2 oder 3 Substituenten substituiert ist, wobei jeder Substituent unabhängig unter Hydroxy, Halogen, Cyano, Nitro, Amino, Mono- oder Dialkylamino, Alkyl, Halogenalkyl, Alkyloxy, Alkylthio, Halogen- alkyloxy, Carboxyl, Alkyloxycarbonyl, Amino- carbonyl, Morpholinyl, Het oder Mono- oder Dialkylaminocarbonyl ausgewählt ist;
Het für einen monocyclischen Heterocyclus, der unter N-Phenoxypiperidinyl, Piperidinyl, Piperazin, Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl oder Pyridazinyl ausgewählt ist, oder einen bicyclischen Heterocyclus, der unter Chinolinyl, Chinoxalinyl, Indolyl, Benzimidazolyl, Benzoxazolyl, Benzisoxazolyl, Benzothiazolyl, Benzisothiazolyl, Benzofuranyl, Benzothienyl, 2,3-Dihydrobenzo[1,4]dioxinyl oder Benzo[1,3]dioxolyl ausgewählt ist, steht; wobei jeder monocyclische und bicyclische Hetero- cyclus gegebenenfalls durch 1, 2 oder 3 Substituenten substituiert ist, wobei jeder Substituent unabhängig unter Halogen, Hydroxy, Alkyl oder Alkyloxy ausgewählt ist;
unter der Vorraussetzung, daß R⁵ nicht für Benzyl steht;
ein N-Oxid davon, ein pharmazeutisch unbedenkliches Salz davon oder ein Solvat davon.

2. Verbindung nach Anspruch 1, worin
R³ für Alkyl, Arylalkyl, Aryl-O-alkyl, Arylalkyl- O-alkyl, Aryl, Het, Het-Alkyl, Het-O-Alkyl, Het-Alkyl-O-alkyl oder 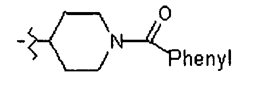 steht;
R⁴ für Wasserstoff oder Alkyl steht;
R⁵ für -C(=NH)-NH_{2;} Arylalkyl; Het-Alkyl; Mono- oder Dialkylaminoalkyl; Het oder Aryl steht oder
R⁴ und R⁵ gemeinsam mit dem Stickstoffatom, an das sie gebunden sind, einen Rest aus der Gruppe bestehend aus 2,3-Dihydroisoindol-1-yl, Thiazolidin-3-yl, 1,2,3,6-Tetrahydropyridyl, Hexahydro-1*H*-azepinyl, Hexahydro-1*H*-1,4- diazepinyl, Hexahydro-1,4-oxazepinyl, 1,2,3,4- Tetrahydroisochinolin-2-yl oder 2,5- Diazabicyclo[2.2.1]heptyl bilden, wobei jeder Rest gegebenenfalls durch 1, 2, 3 oder 4 Substituenten substituiert ist, wobei jeder Substituent unabhängig unter Alkyl, Halogenalkyl, Alkylcarbonyl, Halogen, Arylalkyl, Hydroxy, Alkyloxy, Amino, Mono- oder Dialkylamino, Alkylthio, Alkyloxyalkyl, Alkyl- thioalkyl, Aryl, Pyridyl oder Pyrimidinyl aus- gewählt ist; oder
R⁴ und R⁵ gemeinsam mit dem Stickstoffatom, an das sie gebunden sind, einen Rest aus der Gruppe bestehend aus Piperidinyl oder Piperazinyl bilden, wobei jeder Rest durch Aryl, Alkyl- carbonyl, Piperidinyl oder Pyrrolidinyl, das gegebenenfalls durch Arylalkyl substituiert ist, substituiert ist; Aryl für einen Homocyclus steht, der unter Phenyl, Naphthyl, Acenaphthyl oder Tetrahydronaphthyl ausgewählt ist, wobei jeder dieser Cyclen gegebenenfalls durch 1, 2 oder 3 Substituenten substituiert ist, wobei jeder Substituent unabhängig unter Hydroxy, Halogen, Cyano, Nitro, Amino, Mono- oder Dialkylamino, Alkyl, Halogenalkyl, Alkyloxy, Halogenalkyloxy, Carboxyl, Alkyloxycarbonyl, Aminocarbonyl, Morpholinyl oder Mono- oder Dialkylamino- carbonyl ausgewählt ist.

3. Verbindung nach Anspruch 1 oder 2, worin Alkyl für C₁₋₆-Alkyl steht.

4. Verbindung nach einem der vorhergehenden Ansprüche, worin R¹ für Wasserstoff oder Halogen steht.

5. Verbindung nach einem der vorhergehenden Ansprüche, worin p gleich 1 ist.

6. Verbindung nach einem der vorhergehenden Ansprüche, worin R² für C₁₋₆-Alkyloxy steht.

7. Verbindung nach Anspruch 6, worin R² für Methoxy steht.

8. Verbindung nach einem der vorhergehenden Ansprüche, worin R³ für Aryl-C₁₋₆-alkyl oder Aryl steht.

9. Verbindung nach einem der vorhergehenden Ansprüche, worin q für 3 oder 4 steht.

10. Verbindung nach einem der vorhergehenden Ansprüche, worin R⁴ für Wasserstoff oder C₁₋₆-Alkyl steht.

11. Verbindung nach Anspruch 10, worin R⁴ für C₁₋₆-Alkyl steht.

12. Verbindung nach einem der vorhergehenden Ansprüche, worin R⁵ für -C(=NH)-NH₂; Het-C₁₋₆-Alkyl; Mono- oder Di(C₁₋₆-alkyl)amino-C₁₋₆-alkyl; Bicyclo-[2.2.1]heptyl oder Het steht.

13. Verbindung nach Anspruch 12, worin R⁵ für -C(=NH)-NH₂; Het-C₁₋₆-Alkyl; Bicyclo[2.2.1]heptyl oder Het steht.

14. Verbindung nach einem der Ansprüche 1 bis 9, worin R⁴ und R⁵ gemeinsam mit dem Stickstoffatom, an das sie gebunden sind, einen Rest aus der Gruppe bestehend aus Azetidinyl; 2,3-Dihydroisoindol-1-yl; Thiazolidin-3-yl; 1,2,3,6-Tetrahydropyridyl; Hexahydro-1*H*-azepinyl; Hexahydro-1*H*-1,4-diazepinyl; Hexahydro-1,4-oxazepinyl; 1,2,3,4-Tetrahydroisochinolin-2-yl; 2,5-Diazabicyclo-[2.2.1]heptyl; Thiomorpholinyl-1,1-dioxid bilden; wobei jeder Rest gegebenenfalls durch 1, 2, 3 oder 4 Substituenten substituiert ist, wobei jeder Substituent unabhängig unter C₁₋₆-Alkyl, Halogen-C₁₋₆-alkyl, C₁₋₆-Alkylcarbonyl, Halogen, Aryl-C₁₋₆-alkyl, Hydroxy, C₁₋₆-Alkyloxy, Amino, Mono- oder Di-C₁₋₆-alkylamino, Mono- oder Di-C₁₋₆-alkylamino-C₁₋₆-alkyl, C₁₋₆-Alkylthio, C₁₋₆-Alkyloxy-C₁₋₆-alkyl, C₁₋₆-Alkylthio-C₁₋₆-alkyl, Aryl, Piperidinyl, das gegebenenfalls durch C₁₋₆-Alkyl substituiert ist, Pyrrolidinyl, das gegebenenfalls durch Aryl-C₁₋₆-alkyl substituiert ist, Pyridyl oder Pyrimidinyl ausgewählt ist.

15. Verbindung nach einem der Ansprüche 1 bis 9, worin R⁴ und R⁵ gemeinsam mit dem Stickstoffatom, an das sie gebunden sind, einen Rest aus der Gruppe bestehend aus Azetidinyl; Hexahydro-1*H*-1,4-diazepinyl; 2,5-Diazabicyclo[2.2.1]heptyl oder Hexahydro-1*H*-azepinyl bilden; wobei jeder Rest gegebenenfalls durch 1, 2, 3 oder 4 Substituenten substituiert ist, wobei jeder Substituent unabhängig unter C₁₋₆-Alkyl oder Aryl-C₁₋₆-alkyl ausgewählt ist; oder R⁴ und R⁵ gemeinsam mit dem Stickstoffatom, an das sie gebunden sind, einen Rest aus der Gruppe bestehend aus Piperidinyl oder Piperazinyl bilden, wobei jeder Rest durch Aryl, C₁₋₆-Alkylcarbonyl, Piperidinyl oder Pyrrolidinyl, das gegebenenfalls durch Aryl-C₁₋₆-alkyl substituiert ist, substituiert ist.

16. Verbindung nach Anspruch 15, worin R⁴ und R⁵ gemeinsam mit dem Stickstoffatom, an das sie gebunden sind, einen Rest aus der Gruppe bestehend aus Azetidinyl; Hexahydro-1*H*-1,4-diazepinyl; 2,5-Diazabicyclo[2.2.1]heptyl oder Hexahydro-1*H-*azepinyl bilden; wobei jeder Rest gegebenenfalls durch 1, 2, 3 oder 4 Substituenten substituiert ist, wobei jeder Substituent unabhängig unter C₁₋₆-Alkyl oder Aryl-C₁₋₆-alkyl ausgewählt ist.

17. Verbindung nach einem der vorhergehenden Ansprüche, worin R⁶ für Phenyl, das gegebenenfalls durch Halogen substituiert ist, steht.

18. Verbindung nach einem der vorhergehenden Ansprüche, worin R⁷ für Wasserstoff steht.

19. Verbindung nach einem der vorhergehenden Ansprüche, bei der es sich um eine Verbindung der Formel (Ia) handelt.

20. Verbindung nach einem der Ansprüche 1 bis 18, bei der es sich um eine Verbindung der Formel (Ib) handelt und worin R⁸ für Wasserstoff steht und R⁹ für Oxo steht.

21. Verbindung nach Anspruch 1, bei der es sich um eine Verbindung der Formel (Ia) handelt und worin R¹ für Wasserstoff oder Halogen steht; R² für C₁₋₆-Alkyloxy steht; R³ für Aryl-C₁₋₆-alkyl oder Aryl steht; R⁴ für Wasserstoff oder C₁₋₆-Alkyl steht; R⁵ für -C(=NH)-NH₂; Het-C₁₋₆-Alkyl; Mono- oder Di(C₁₋₆-alkyl)amino-C₁₋₆-alkyl; Bicyclo[2.2.1]heptyl oder Het steht oder R⁴ und R⁵ gemeinsam mit dem Stickstoffatom, an das sie gebunden sind, einen Rest aus der Gruppe bestehend aus Azetidinyl; Hexahydro-1*H*-azepinyl; Hexahydro-1*H*-1,4-diazepinyl; 2,5-Diazabicyclo[2.2.1]heptyl oder Thiomorpholinyl-1,1-dioxid bilden; wobei jeder Rest gegebenenfalls durch 1, 2, 3 oder 4 Substituenten substituiert ist, wobei jeder Substituent unabhängig unter C₁₋₆-Alkyl , Aryl-C₁₋₆-alkyl oder Piperidinyl, das gegebenenfalls durch C₁₋₆-Alkyl substituiert ist, ausgewählt ist; oder R⁴ und R⁵ gemeinsam mit dem Stickstoffatom, an das sie gebunden sind, einen Rest aus der Gruppe bestehend aus Piperidinyl oder Piperazinyl bilden, wobei jeder Rest durch Aryl, C₁₋₆-Alkylcarbonyl, Piperidinyl oder Pyrrolidinyl, das gegebenenfalls durch Aryl-C₁₋₆-alkyl substituiert ist, substituiert ist; R⁶ für Phenyl, das gegebenenfalls durch Halogen substituiert ist, steht; R⁷ für Wasserstoff steht; q für 3 oder 4 steht; p für 1 steht.

22. Verbindung nach Anspruch 1, ausgewählt unter 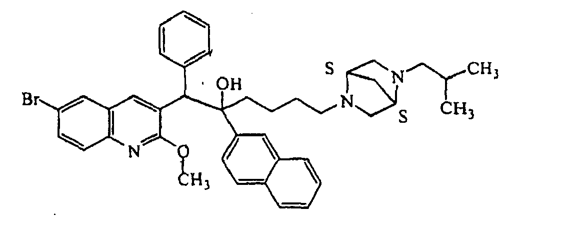 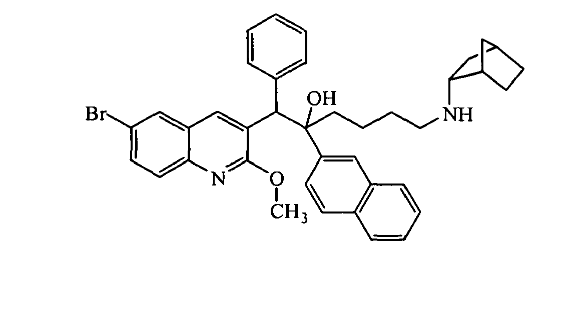 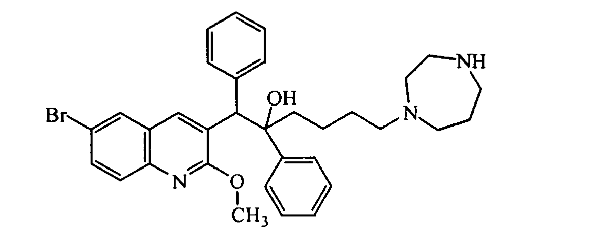 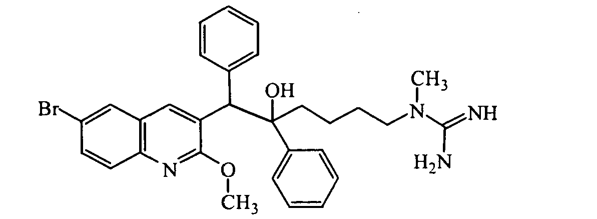 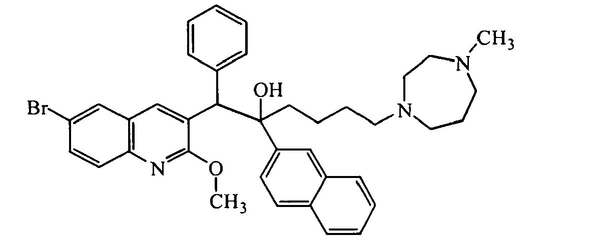 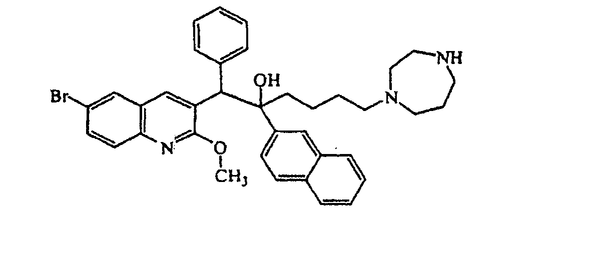 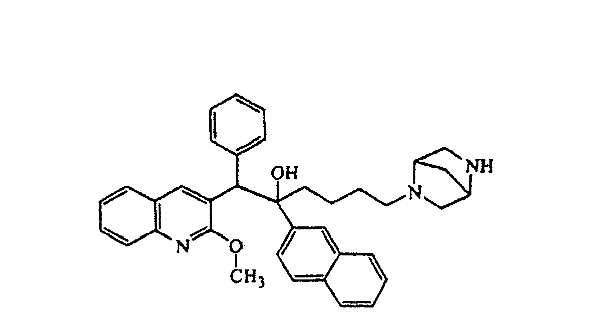 einschließlich beliebiger stereochemisch isomerer Formen davon,
ein N-Oxid davon, ein pharmazeutisch unbedenkliches Salz davon oder ein Solvat davon.

23. Verbindung nach einem der vorhergehenden Ansprüche zur Verwendung als Arzneimittel.

24. Verbindung nach einem der Ansprüche 1 bis 22 zur Verwendung als Arzneimittel für die Behandlung einer bakteriellen Infektion.

25. Pharmazeutische Zusammensetzung, enthaltend einen pharmazeutisch unbedenklichen Träger und als Wirkstoff eine therapeutisch wirksame Menge einer Verbindung gemäß einem der Ansprüche 1 bis 22.

26. Verbindung nach Anspruch 24, wobei es sich bei der bakteriellen Infektion um eine Infektion mit einem grampositiven Bakterium handelt.

27. Verbindung nach Anspruch 26, wobei es sich bei dem grampositiven Bakterium um Streptococcus pneumoniae oder Staphylococcus aureus handelt.

28. Verfahren zur Herstellung einer Verbindung nach Anspruch 1, **dadurch gekennzeichnet, daß** man
a) ein Zwischenprodukt der Formel (II-a) oder (IIb) in Gegenwart einer geeigneten Base und eines geeigneten Lösungsmittels mit 1*H*-Pyrazol-1-carboximidamid umsetzt, 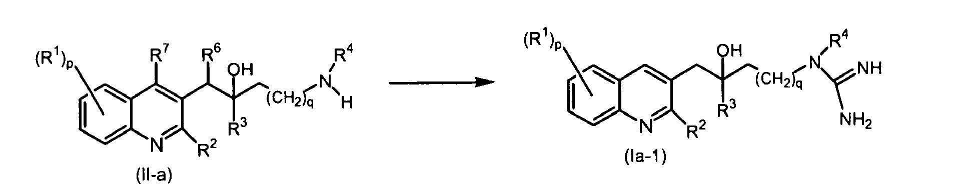 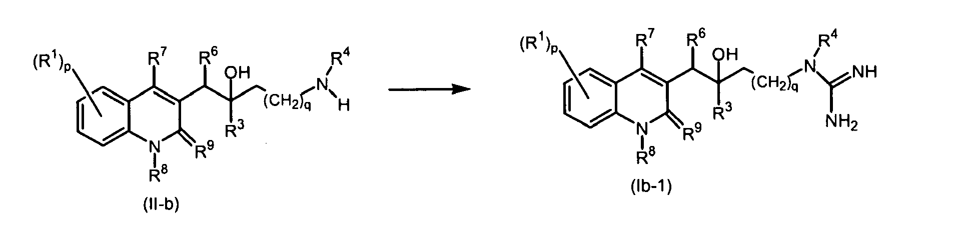 wobei alle Variablen die in Anspruch 1 angegebene Bedeutung besitzen;
b) ein Zwischenprodukt der Formel (III-a) oder (III-b) gemäß dem folgenden Reaktionsschema unter Verwendung von nBuLi in einer Mischung einer geeigneten Base und eines geeigneten Lösungsmittels mit einem Zwischenprodukt der Formel (IV) umsetzt: 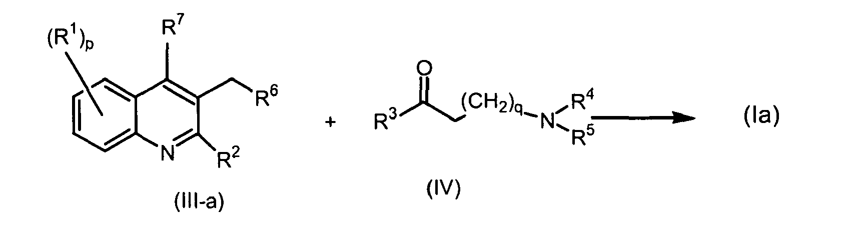 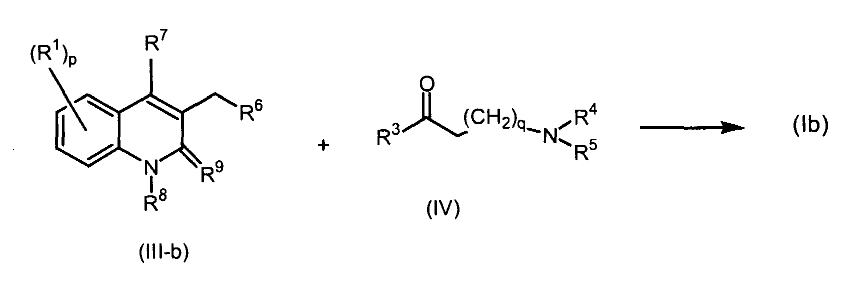 wobei alle Variablen die in Anspruch 1 angegebene Bedeutung besitzen;
c) ein Zwischenprodukt der Formel (V-a) oder (V-b), worin q' für 0, 1 oder 2 steht, in Gegenwart eines geeigneten Katalysators, gegebenenfalls in Gegenwart eines zweiten Katalysators (für die Reduktion), in Gegenwart eines geeigneten Liganden in einem geeigneten Lösungsmittel in Gegenwart von CO und H₂ (unter Druck) mit einem primären oder sekundären Amin HNR⁴R⁵ umsetzt, 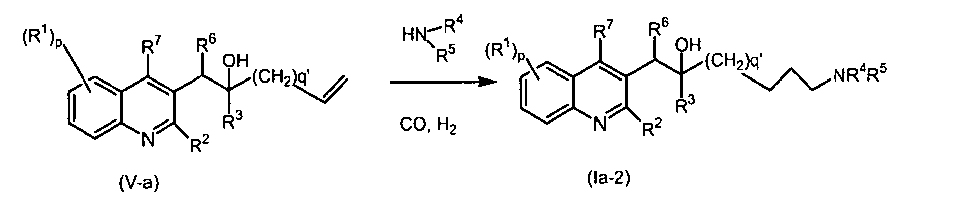 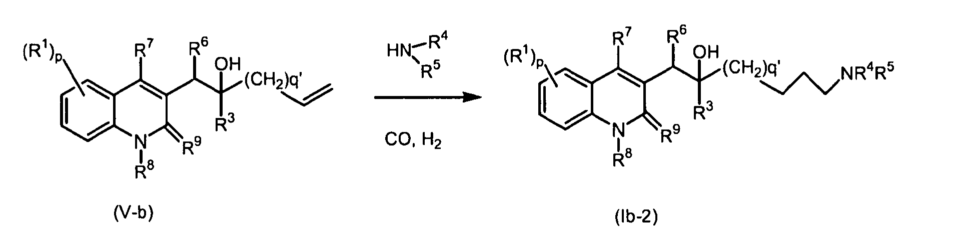 wobei alle Variablen die in Anspruch 1 angegebene Bedeutung besitzen;
d) ein Zwischenprodukt der Formel (VI-a) oder (VIb), worin W₂ für eine geeignete Abgangsgruppe steht, mit einem geeigneten primären oder sekundären Amin HNR⁴R⁵ umsetzt, gegebenenfalls in Gegenwart eines geeigneten Lösungsmittels, 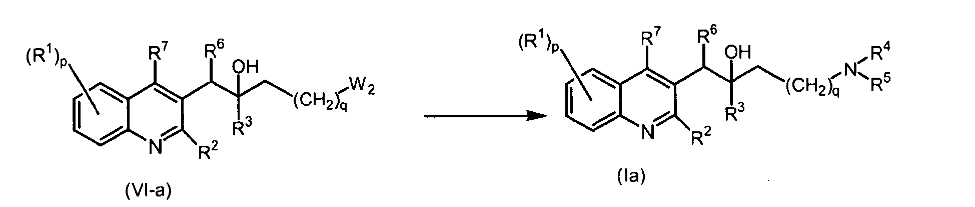 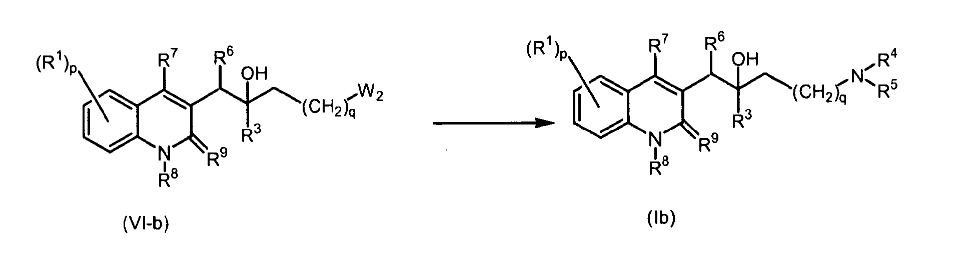 wobei alle Variablen die in Anspruch 1 angegebene Bedeutung besitzen;
oder gegebenenfalls Verbindungen der Formel (Ia) oder (Ib) durch an sich bekannte Transformationen ineinander umwandelt und weiterhin gegebenenfalls die Verbindungen der Formel (Ia) oder (Ib) durch Behandlung mit einer Säure in ein therapeutisch wirksames nichttoxisches Säureadditionssalz oder durch Behandlung mit einer Base in ein therapeutisch wirksames nichttoxisches Basenadditionssalz umwandelt oder umgekehrt die Säureadditionssalzform durch Behandlung mit Alkali in die freie Base oder das Basenadditionssalz durch Behandlung mit Säure in die freie Säure umwandelt und gegebenenfalls stereochemisch isomere Formen, quaternäre Amine oder N-Oxidformen davon herstellt.

29. Kombination aus (a) einer Verbindung nach einem der Ansprüche 1 bis 22, und (b) einem oder mehreren anderen antibakteriellen Mitteln.

30. Produkt, enthaltend (a) eine Verbindung nach einem der Ansprüche 1 bis 22, und (b) ein oder mehrere andere antibakterielle Mittel, als Kombinationspräparat für die gleichzeitige, getrennte oder aufeinanderfolgende Verwendung bei der Behandlung einer bakteriellen Infektion.

31. Verbindung nach Anspruch 27, wobei es sich bei dem grampositiven Bakterium um methicillinresistenten *Staphylococcus aureus* handelt.

32. Verbindung nach Anspruch 24, wobei es sich bei der bakteriellen Infektion um eine Infektion mit *Mycobacterium tuberculosis* handelt.

## Revendications

1. Composé de formule (Ia) ou (Ib) 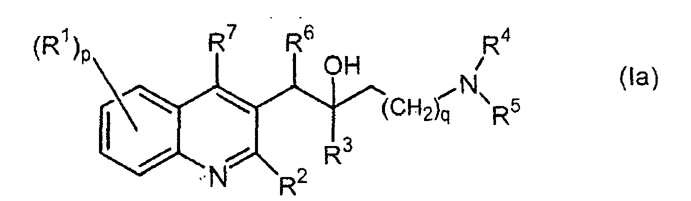 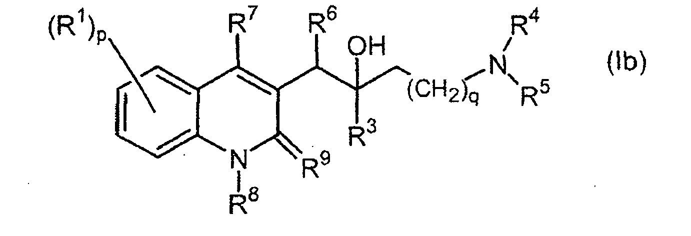 y compris toute forme stéréochimiquement isomère de celui-ci, dans lesquelles
p est un entier égal à 1, 2, 3 ou 4 ;
q est un entier égal à zéro, 1, 2, 3 ou 4 ;
R¹ est hydrogène, cyano, formyle, carboxyle, halogéno, alkyle, C₂₋ ₆alcényle, C₂₋₆alcynyle, halogénoalkyle, hydroxy, alkyloxy, alkylthio, alkylthioalkyle, -C=N-OR¹¹, amino, mono ou di(alkyl)amino, aminoalkyle, mono ou di(alkyl)aminoalkyle, alkylcarbonylaminoalkyle, aminocarbonyle, mono ou di(alkyl)aminocarbonyle, arylalkyle, arylcarbonyle, R^{5a}R^{4a}Nalkyle, di(aryl)alkyle, aryle, R^{5a}R^{4a}N-, R^{5a}R^{4a}N- C(=O)-, ou Het ;
R² est hydrogène, alkyloxy, aryle, aryloxy, hydroxy, mercapto, alkyloxyalkyloxy, alkylthio, mono ou di(alkyl)amino, pyrrolidino ou un radical de formule 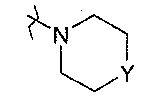 dans laquelle Y est CH₂, O, S, NH ou *N*-alkyle ;
R³ est alkyle, arylalkyle, aryl-O-alkyle, aryl-alkyl-O-alkyle, aryle, aryl-aryle, Het, Het-alkyle, Het-O-alkyle, Het- alkyl-O-alkyle ou 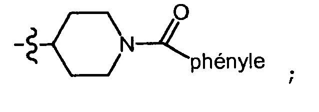
R⁴ est hydrogène ou alkyle ;
R⁵ est -C(=NH)-NH² ; arylalkyle ; Het- alkyle ; mono- ou dialkylaminoalkyle ; bicyclo[2,2,1]heptyle ; Het ; ou aryle ; ou
R⁴ et R⁵ ensemble avec l'atome d'azote auquel ils sont liés forment un radical choisi dans le groupe constitué d'azétidinyle ; 2,3- dihydroisoindol-1-yle ; thiazolidin-3- yle ; 1,2,3,6-tétrahydropyridyle ; hexahydro-1*H*-azépinyle ; hexahydro-1H- 1,4-diazépinyle ; hexahydro-1,4- oxazépinyle ; 1,2,3,4- tétrahydroisoquinoléin-2-yle ;
2,5-diazabicyclo[2,2,1]heptyle ; 1,1- dioxyde-thiomorpholinyle ; chaque radical éventuellement substitué par 1, 2, 3 ou 4 substituants, chaque substituant choisi indépendamment parmi alkyle, halogénoalkyle, alkylcarbonyle, halogéno, arylalkyle, hydroxy, alkyloxy, amino, mono- ou dialkylamino, mono- ou dialkylaminoalkyle, alkylthio, alkyloxyalkyle, alkylthioalkyle, aryle, pipéridinyle éventuellement substitué par alkyle, pyrrolidinyle éventuellement substitué par arylalkyle, pyridyle ou pyrimidinyle ; ou
R⁴ et R⁵ ensemble avec l'atome d'azote auquel ils sont liés forment un radical choisi dans le groupe constitué de pipéridinyle ou pipérazinyle, chacun substitué par aryle, alkylcarbonyle, pipéridinyle ou pyrrolidinyle éventuellement substitué par arylalkyle ;
R^{4a} et R^{5a} ensemble avec l'atome d'azote auquel ils sont liés forment un radical choisi dans le groupe constitué de pyrrolidino, pipéridino, pipérazino, morpholino, 4- thiomorpholino, 2,3-dihydroisoindol-1- yle, thiazolidin-3-yle, 1,2,3,6- tétrahydropyridyle, hexahydro-1*H*- azépinyle, hexahydro-1*H*-1,4-diazépinyle, hexahydro-1,4-oxazépinyle, 1,2,3,4- tétrahydroisoquinoléin-2-yle, pyrrolinyle, pyrrolyle, imidazolidinyle, pyrazolidinyle, 2-imidazolinyle, 2- pyrazolinyle, imidazolyle, pyrazolyle, triazolyle, pyridinyle, pyridazinyle, pyrimidinyle, pyrazinyle et triazinyle, chaque radical éventuellement substitué par 1, 2, 3 ou 4 substituants, chaque substituant choisi indépendamment parmi alkyle, halogénoalkyle, halogéno, arylalkyle, hydroxy, alkyloxy, amino, mono- ou dialkylamino, alkylthio, alkylthioalkyle, aryle, pyridyle ou pyrimidinyle ;
R⁶ est aryle¹ ou Het ;
R⁷ est hydrogène, halogéno, alkyle, aryle ou Het ;
R⁸ est hydrogène ou alkyle ;
R⁹ est oxo ; ou
R⁸ et R⁹ ensemble forment le radical -CH=CH-N= ;
R¹¹ est hydrogène ou alkyle ;
aryle est un homocycle choisi parmi phényle, naphtyle, acénaphtyle ou tétrahydronaphtyle, chacun étant éventuellement substitué par 1, 2 ou 3 substituants, chaque substituant étant choisi indépendamment parmi hydroxy, halogéno, cyano, nitro, amino, mono- ou dialkylamino, alkyle, C₂₋₆alcényle éventuellement substitué par phényle, halogénoalkyle, alkyloxy, halogénoalkyloxy, carboxyle, alkyloxycarbonyle, aminocarbonyle, morpholinyle ou mono- ou dialkylaminocarbonyle ;
aryle¹ est un homocycle choisi parmi phényle, naphtyle, acénaphtyle ou tétrahydronaphtyle, chacun étant éventuellement substitué par 1, 2 ou 3 substituants, chaque substituant étant choisi indépendamment parmi hydroxy, halogéno, cyano, nitro, amino, mono- ou dialkylamino, alkyle, halogénoalkyle, alkyloxy, alkylthio, halogénoalkyloxy, carboxyle, alkyloxycarbonyle, aminocarbonyle, morpholinyle, Het ou mono- ou dialkylaminocarbonyle ;
Het est un hétérocycle monocyclique choisi parmi *N*-phénoxypipéridinyle, pipéridinyle, pipérazine, pyrrolyle, pyrazolyle, imidazolyle, furanyle, thiényle, oxazolyle, isoxazolyle, thiazolyle, isothiazolyle, pyridinyle, pyrimidinyle, pyrazinyle ou pyridazinyle ; ou un hétérocycle bicyclique choisi parmi quinoléinyle, quinoxalinyle, indolyle, benzimidazolyle, benzoxazolyle, benzisoxazolyle, benzothiazolyle, benzisothiazolyle, benzofuranyle, benzothiényle, 2,3- dihydrobenzo[1,4]dioxinyle ou benzo[1,3]dioxolyle, chaque hétérocycle monocyclique et bicyclique étant éventuellement substitué par 1, 2 ou 3 substituants, chaque substituant étant choisi indépendamment parmi halogéno, hydroxy, alkyle ou alkyloxy ;
à condition que R⁵ soit autre que benzyle ;
un N-oxyde de celui-ci, un sel pharmaceutiquement acceptable de celui-ci ou un solvate de celui-ci.

2. Composé selon la revendication 1, **caractérisé en ce que**
R³ est alkyle, arylalkyle, aryl-O-alkyle, aryl-alkyl-O-alkyle, aryle, Het, Het- alkyle, Het-O-alkyle, Het-alkyl-O-alkyle ou 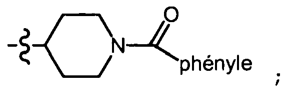
R⁴ est hydrogène ou alkyle ;
R⁵ est -C(=NH)-NH₂ ; arylalkyle ; Het- alkyle ; mono- ou dialkylaminoalkyle ; Het ; ou aryle ; ou
R⁴ et R⁵ ensemble avec l'atome d'azote auquel ils sont liés forment un radical choisi dans le groupe constitué de 2,3- dihydroisoindol-1-yle ; thiazolidin-3- yle ; 1,2,3,6-tétrahydropyridyle ; hexahydro-1*H*-azépinyle ; hexahydro-1*H*- 1,4-diazépinyle ; hexahydro-1,4- oxazépinyle ; 1,2,3,4- tétrahydroisoquinoléin-2-yle ou 2,5- diazabicyclo[2,2,1]heptyle ; chaque radical éventuellement substitué par 1, 2, 3 ou 4 substituants, chaque substituant choisi indépendamment parmi alkyle, halogénoalkyle, alkylcarbonyle, halogéno, arylalkyle, hydroxy, alkyloxy, amino, mono- ou dialkylamino, alkylthio, alkyloxyalkyle, alkylthioalkyle, aryle, pyridyle ou pyrimidinyle ; ou
R⁴ et R⁵ ensemble avec l'atome d'azote auquel ils sont liés forment un radical choisi dans le groupe constitué de pipéridinyle ou pipérazinyle, chacun substitué par aryle, alkylcarbonyle, pipéridinyle ou pyrrolidinyle éventuellement substitué par arylalkyle ;
aryle est un homocycle choisi parmi phényle, naphtyle, acénaphtyle ou tétrahydronaphtyle, chacun étant éventuellement substitué par 1, 2 ou 3 substituants, chaque substituant étant choisi indépendamment parmi hydroxy, halogéno, cyano, nitro, amino, mono- ou dialkylamino, alkyle, halogénoalkyle, alkyloxy, halogénoalkyloxy, carboxyle, alkyloxycarbonyle, aminocarbonyle, morpholinyle ou mono- ou dialkylaminocarbonyle.

3. Composé selon la revendication 1 ou 2, **caractérisé en ce que** alkyle représente C₁₋₆alkyle.

4. Composé selon l'une quelconque des revendications précédentes, **caractérisé en ce que** R¹ est hydrogène ou halogéno.

5. Composé selon l'une quelconque des revendications précédentes, **caractérisé en ce que** p est égal à 1.

6. Composé selon l'une quelconque des revendications précédentes, **caractérisé en ce que** R² est C₁₋₆alkyloxy.

7. Composé selon la revendication 6, **caractérisé en ce que** R² est méthoxy.

8. Composé selon l'une quelconque des revendications précédentes, **caractérisé en ce que** R³ est arylC₁₋₆alkyle ou aryle.

9. Composé selon l'une quelconque des revendications précédentes, **caractérisé en ce que** q est égal à 3 ou 4.

10. Composé selon l'une quelconque des revendications précédentes, **caractérisé en ce que** R⁴ est hydrogène ou C₁₋₆alkyle.

11. Composé selon la revendication 10, **caractérisé en ce que** R⁴ est C₁₋₆alkyle.

12. Composé selon l'une quelconque des revendications précédentes, **caractérisé en ce que** R⁵ est -C(=NH)-NH₂ ; Het-C₁₋₆alkyle ; mono- ou di(C₁₋₆alkyl)aminoC₁₋₆alkyle ; bicyclo[2,2,1]heptyle ; ou Het.

13. Composé selon la revendication 12, **caractérisé en ce que** R⁵ est -C(=NH)-NH₂ ; Het-C₁₋₆alkyle ; bicyclo[2,2,1]heptyle ; ou Het.

14. Composé selon l'une quelconque des revendications 1 à 9, **caractérisé en ce que** R⁴ et R⁵ ensemble avec l'atome d'azote auquel ils sont liés forment un radical choisi parmi le groupe constitué d'azétidinyle ; 2,3-dihydroisoindol-1-yle ; thiazolidin-3-yle ; 1,2,3,6-tétrahydropyridyle ; hexahydro-1*H*-azépinyle ; hexahydro-1*H*-1,4-diazépinyle ; hexahydro-1,4-oxazépinyle ; 1,2,3,4-tétrahydroisoquinoléin-2-yle ; 2,5-diazabicyclo[2,2,1]heptyle ; 1,1-dioxyde-thiomorpholinyle ; chaque radical éventuellement substitué par 1, 2, 3 ou 4 substituants, chaque substituant choisi indépendamment parmi C₁₋₆alkyle, halogénoC₁₋₆alkyle, C₁₋₆alkylcarbonyle, halogéno, arylC₁₋₆alkyle, hydroxy, C₁₋₆alkyloxy, amino, mono- ou diC₁₋₆alkylamino, mono- ou diC₁₋₆alkylaminoC₁₋₆alkyle, C₁₋₆alkylthio, C₁₋₆alkyloxyC₁₋₆alkyle, C₁₋₆alkyl thioC₁₋₆alkyle, aryle, pipéridinyle éventuellement substitué par C₁₋₆alkyle, pyrrolidinyle éventuellement substitué par arylC₁₋₆alkyle, pyridyle ou pyrimidinyle.

15. Composé selon l'une quelconque des revendications 1 à 9, **caractérisé en ce que** R⁹ et R⁵ ensemble avec l'atome d'azote auquel ils sont liés forment un radical choisi dans le groupe constitué d'azétidinyle, hexahydro-1*H*-1,4-diazépinyle, 2,5-diazabicyclo[2,2,1]heptyle ou hexahydro-1*H-*azépinyle ; chaque radical éventuellement substitué par 1, 2, 3 ou 4 substituants, chaque substituant choisi indépendamment parmi C₁₋₆alkyle ou arylC₁₋₆alkyle ; ou R⁴ et R⁵ ensemble avec l'atome d'azote auquel ils sont liés forment un radical choisi dans le groupe constitué de pipéridinyle ou pipérazinyle, chacun substitué par aryle, C₁₋₆alkylcarbonyle, pipéridinyle ou pyrrolidinyle éventuellement substitué par arylC₁₋₆alkyle .

16. Composé selon la revendication 15, **caractérisé en ce que** R⁴ et R⁵ ensemble avec l'atome d'azote auquel ils sont liés forment un radical choisi dans le groupe constitué d'azétidinyle, hexahydro-1*H*-1,4-diazépinyle, 2,5-diazabicyclo[2,2,1]heptyle ou hexahydro-1*H*-azépinyle ; chaque radical éventuellement substitué par 1, 2, 3 ou 4 substituants, chaque substituant choisi indépendamment parmi C₁₋₆alkyle ou arylC₁₋₆alkyle.

17. Composé selon l'une quelconque des revendications précédentes, **caractérisé en ce que** R⁶ est phényle éventuellement substitué par halogéno.

18. Composé selon l'une quelconque des revendications précédentes, **caractérisé en ce que** R⁷ est hydrogène.

19. Composé selon l'une quelconque des revendications précédentes, **caractérisé en ce que** le composé est un composé de formule (Ia).

20. Composé selon l'une quelconque des revendications 1 à 18, **caractérisé en ce que** le composé est un composé de formule (Ib) et **en ce que** R⁸ est hydrogène et R⁹ est oxo.

21. Composé selon la revendication 1, **caractérisé en ce que** le composé est un composé de formule (Ia) et **en ce que** R¹ est hydrogène ou halogéno ; R² est C₁₋₆alkyloxy ; R³ est arylC₁₋₆alkyle ou aryle ; R⁴ est hydrogène ou C₁₋₆alkyle ; R⁵ est -C(=NH)-NH₂ ; Het-C₁₋₆alkyle ; mono- ou di(C₁₋₆alkyl)aminoC₁₋₆alkyle ; bicyclo[2,2,1]heptyle ; ou Het ; ou R⁴ et R⁵ ensemble avec l'atome d'azote auquel ils sont liés forment un radical choisi dans le groupe constitué d'azétidinyle ; hexahydro-1*H*-azépinyle ; hexahydro-1*H*-1,4-diazépinyle ; 2,5-diazabicyclo[2,2,1]heptyle ; ou 1,1-dioxyde-thiomorpholinyle ; chaque radical éventuellement substitué par 1, 2, 3 ou 4 substituants, chaque substituant choisi indépendamment parmi C₁₋₆alkyle, arylC₁₋₆alkyle, pipéridinyle éventuellement substitué par C₁₋₆alkyle ; ou R⁴ et R⁵ ensemble avec l'atome d'azote auquel ils sont liés forment un radical choisi dans le groupe constitué de pipéridinyle ou pipérazinyle, chacun substitué par aryle, C₁₋₆alkylcarbonyle, pipéridinyle ou pyrrolidinyle éventuellement substitué par arylC₁₋₆alkyle ; R⁶ est phényle éventuellement substitué par halogéno ; R⁷ est hydrogène ; q est 3 ou 4 ; p est 1.

22. Composé selon la revendication 1, **caractérisé en ce que** le composé est choisi parmi 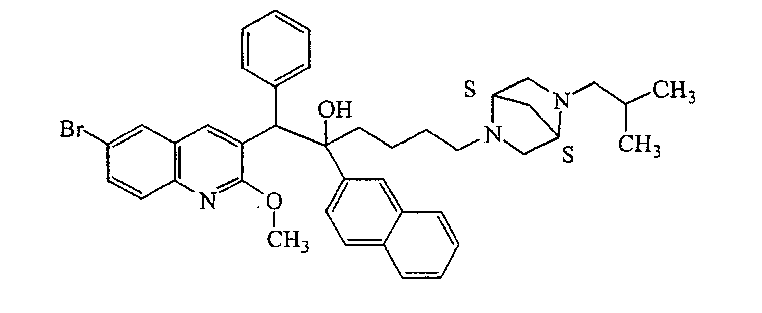 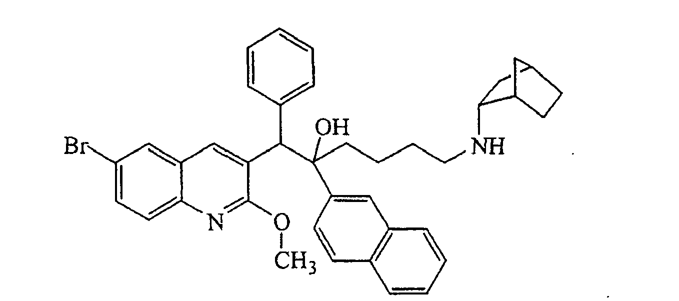 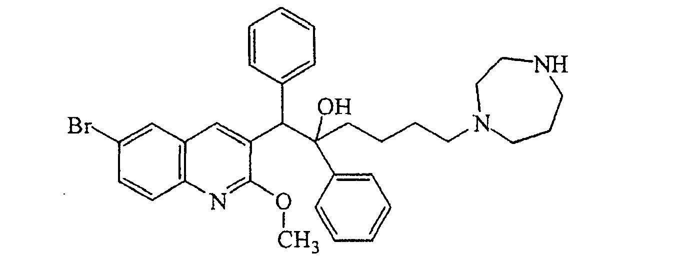 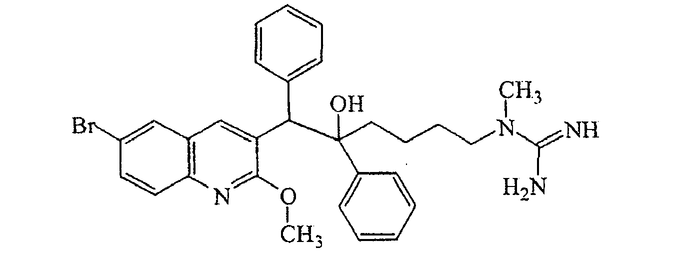 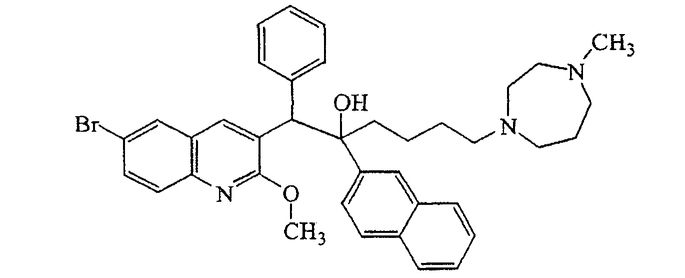 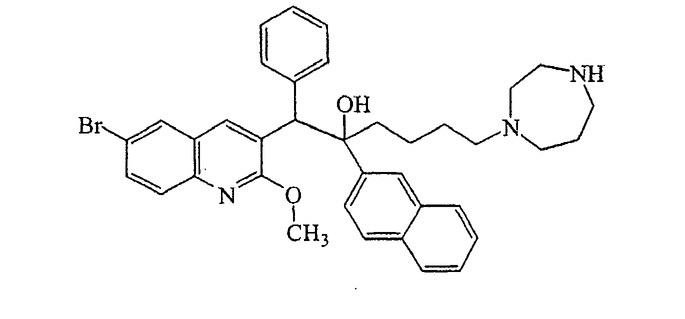 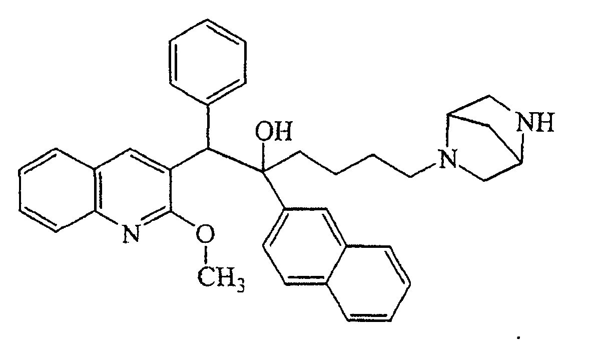 y compris toute forme stéréochimiquement isomère de celui-ci ;
un *N*-oxyde de celui-ci, un sel pharmaceutiquement acceptable de celui-ci ou un solvate de celui-ci.

23. Composé selon l'une quelconque des revendications précédentes, destiné à être utilisé comme médicament.

24. Composé selon l'une quelconque des revendications 1 à 22, destiné à être utilisé comme médicament pour le traitement d'une infection bactérienne.

25. Composition pharmaceutique comprenant un support pharmaceutiquement acceptable et, en tant que principe actif, une quantité thérapeutiquement efficace d'un composé tel que défini dans l'une quelconque des revendications 1 à 22.

26. Composé selon la revendication 24, **caractérisé en ce que** l'infection bactérienne est une infection par une bactérie gram-positif.

27. Composé selon la revendication 26, **caractérisé en ce que** la bactérie gram-positif est Streptococcus pneumonia ou Staphylococcus aureus.

28. Procédé de préparation d'un composé selon la revendication 1, **caractérisé par**
a) la réaction d'un intermédiaire de formule (II-a) ou (II-b) avec le 1*H*-pyrazole-1-carboximidamide en présence d'une base convenable et d'un solvant convenable, 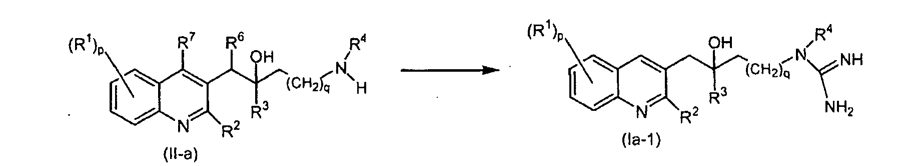 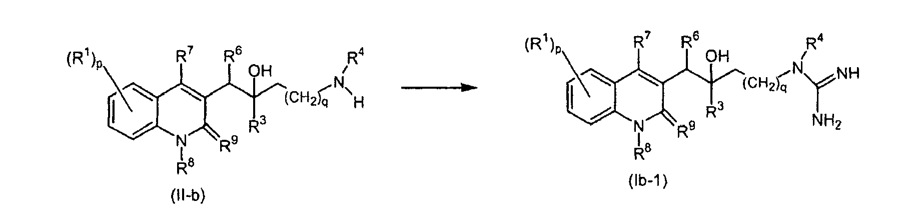 toutes les variables étant telles que définies dans la revendication 1 ;
b) la réaction d'un intermédiaire de formule (III-a) ou (III-b) avec un intermédiaire de formule (IV) selon le schéma réactionnel suivant : 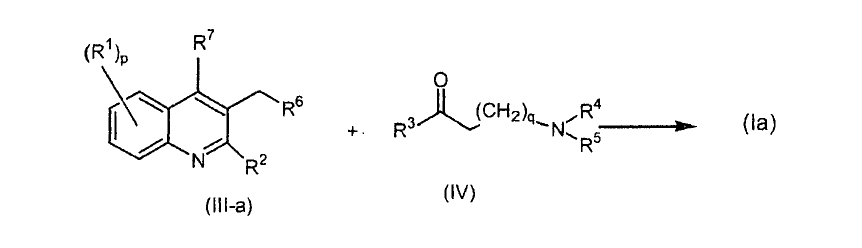 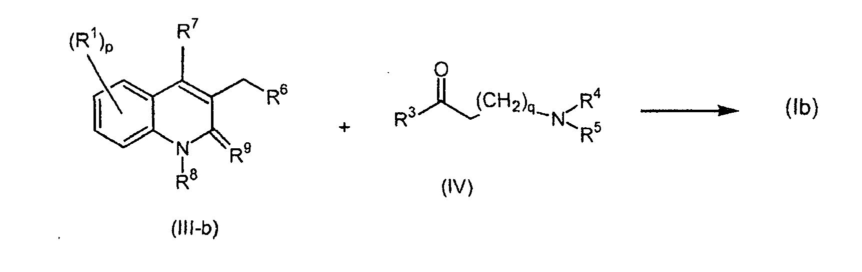 en utilisant du nBuLi dans un mélange d'une base convenable et d'un solvant convenable, toutes les variables étant telles que définies dans la revendication 1 ;
c) la réaction d'un intermédiaire de formule (Va) ou (V-b) dans lesquelles q' est 0, 1 ou 2, avec une amine primaire ou secondaire HNR⁴R⁵ en présence d'un catalyseur convenable, éventuellement en présence d'un deuxième catalyseur (pour la réduction), en présence d'un ligand convenable, dans un solvant convenable, en présence de CO et d'H₂ (sous pression), 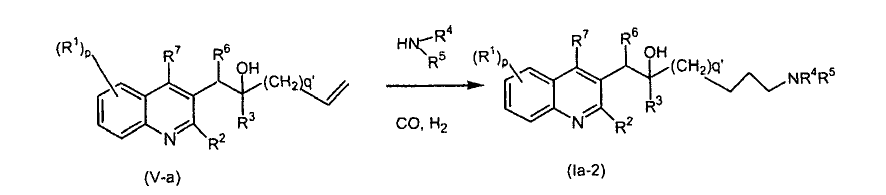 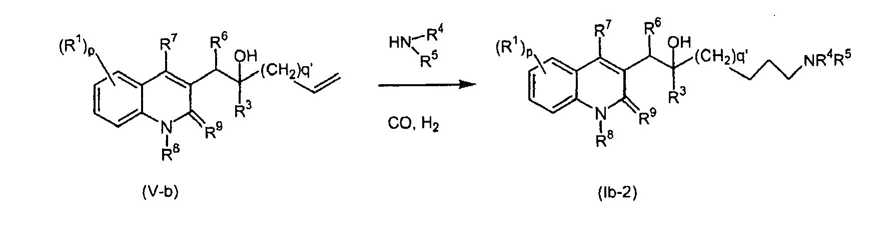 toutes les variables étant telles que définies dans la revendication 1 ;
d) la réaction d'un intermédiaire de formule (VI-a) ou (VI-b) dans lesquelles W₂ représente un groupement partant convenable, avec une amine primaire ou secondaire convenable HNR⁴R⁵, éventuellement en présence d'un solvant convenable 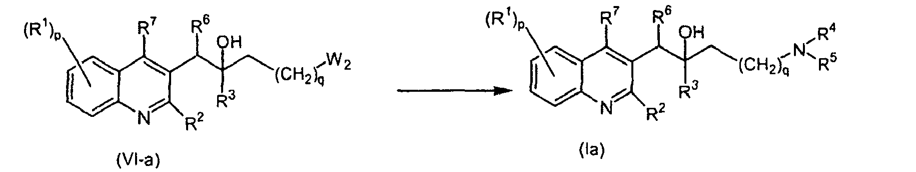 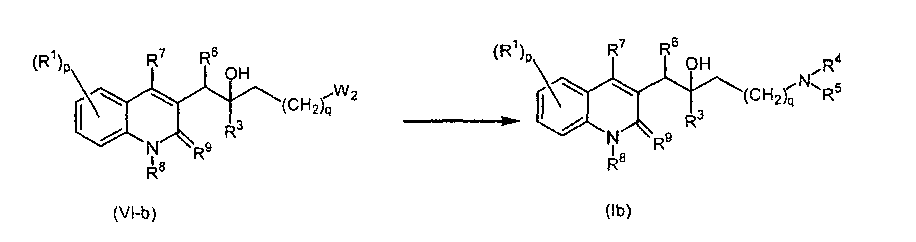 toutes les variables étant telles que définies dans la revendication 1 ;
ou, si on le souhaite, la transformation des composés de formule (Ia) ou (Ib) les uns en les autres en suivant des transformations connues dans l'art, et en outre, si on le souhaite, la transformation des composés de formule (Ia) ou (Ib), en un sel d'addition d'acide non toxique thérapeutiquement actif par traitement avec un acide, ou en un sel d'addition de base non toxique thérapeutiquement active par traitement avec une base, ou inversement, la transformation de la forme sel d'addition d'acide en la base libre par traitement avec un alcali, ou la transformation du sel d'addition de base en l'acide libre par traitement avec un acide ; et, si on le souhaite, la préparation de formes stéréochimiquement isomères, d'amines quaternaires ou de formes *N-*oxydes de ceux-ci.

29. Combinaison (a) d'un composé selon l'une quelconque des revendications 1 à 22, et (b) d'un ou plusieurs autres agents antibactériens.

30. Produit contenant (a) un composé selon l'une quelconque des revendications 1 à 22, et (b) un ou plusieurs autres agents antibactériens, en tant que préparation combinée destinée à une utilisation simultanée, séparée ou séquentielle dans le traitement d'une infection bactérienne.

31. Composé selon la revendication 27, **caractérisé en ce que** la bactérie gram-positif est *Staphylococcus aureus* résistant à la méthicilline.

32. Composé selon la revendication 24, **caractérisé en ce que** l'infection bactérienne est une infection à *Mycobacterium tuberculosis.*
